# EUROPEAN PATENT APPLICATION

(11) **EP 2 679 683 A2**
(43) Date of publication of application: **01.01.2014**
(21) Application number: 12749337.7
(22) Date of filing: 24.02.2012
(51) Int. Cl.: C12N 15/33, C12N 7/00, C07K 14/04, C12N 15/63, A61K 39/25

(54) **NOVEL VARICELLA-ZOSTER VIRUS STRAINS, AND CHICKEN POX AND HERPES ZOSTER VIRUS VACCINE USING SAME**

(30) Priority: 24.02.2011 US 201161446284 P
(71) Applicant: Mogam Biotechnology Research Institute, Kyunggi-Do 446-799 (KR)
(72) Inventor: KIM, Geun Hee, Yongin-Si Kyunggi-Do 446-799 (KR); KWON, Shi Nae, Yongin-Si Kyunggi-Do 446-799 (KR); LEE, Chan Hee, Cheongju-si Chungbuk 361-804 (KR); YOON, Yeop, Yongin-Si Kyunggi-Do 446-799 (KR)
(74) Representative: Herrmann, Uwe
(86) International application number: PCT/KR2012/001408
(87) International publication number: WO 2012/115474

(57) **Abstract**

The present invention relates to novel Varicella-zoster virus strain and to a chicken pox and herpes zoster virus vaccine using the same. More particularly, the present invention relates to genome DNA of VZV MAV/06 strain isolated from a Korean patient, to an open reading frame (ORF) thereof, to a protein coded by the genome DNA of VZV MAV/06 strain and the ORF thereof, and to a vaccine composition which contains the protein as an active ingredient.

## Description

### [Technical Field]

The present invention relates to novel varicella-zoster virus (VZV) strains and a chicken pox and herpes zoster virus vaccine using the same. More particularly, the present invention relates to genome DNA of VZV MAV/06 strain isolated from a Korean patient and attenuated, an opening reading frame (hereinafter, referred to as ORF) thereof, a protein coded by the genome DNA of VZV MAV/06 strain and the ORF thereof, and a vaccine composition containing the protein as an active ingredient.

### [Background Art]

Varicella-zoster virus (hereinafter, referred to as VZV) belongs to the *Herpes viridae* family and is a cause of a4 disease with two different symptoms (chicken pox and herpes zoster).

As a VZV strain isolated from clinical specimens, a perfect nucleotide sequence of a Dumas strain has been initially reported (Davison, A.J. and Scott, J. E., 1986). In addition, the completed entire nucleotide sequences of 23 VZV strains including a parental Oka strain and three vaccine virus strains derived from the parental Oka strain (vOka strain, Varivax strain, and Varilrix strain) have been reported to NCBI GenBank database up to now.

Currently, research into a specific region for attenuating vaccine strain may be conducted through comparative analysis of the entire nucleotide sequences in the vaccine virus strain and clinical virus strain (Argaw et al., 2000; Gomi et al., 2002; Tillieux et al., 2008; Yamanish, 2008).

Meanwhile, "Suduvax" has been mainly produced by Green Cross Corp. as an attenuated vaccine against chicken pox since 1994. A MAV/06 virus strain used to produce Suduvax was obtained by isolating Varicella-zoster virus from blisters of a 33 month old Korean patient infected by chicken pox in 1989 and primarily culturing the isolated VZV in human embryonic lung cells, and it was confirmed by testing various characteristics of varicella-zoster virus that the cultured VZV was a novel VZV (Park et al., Propagation of Varicella-Zoster Virus isolated in Korea, J. Kor. Soc. Virol. 21, 1-9, 1991).

The Suduvax has been commercialized in the Korean market since 1994 and used in the global market since 1998. However, even though efficacy and safety of the Suduvax have been proven in the market, molecular biological characteristics capable of describing a mechanism of efficacy and attenuation of the vaccine have not yet been reported.

The molecular biological characteristics are significantly important to increase accuracy of quality control and quality assurance for assuring efficacy, safety, and homogeneity of the attenuated vaccine and should be urgently investigated.

### [Disclosure]

### [Technical Problem]

In order to solve this problem, the present inventors found out a genome sequence of a novel VZV MAV/06 strain isolated from a Korean patient and performed molecular biological analysis including ORF analysis and phylogenetic analysis the isolated VZV MAV/06 strain, thereby completing the present invention.

An object of the present invention is to provide a genome DNA of VZV MAV/06 strain.

Another object of the present invention is to provide an open reading frame (ORF) of the genome DNA of VZV MAV/06 strain.

Still another object of the present invention is to provide a protein coded by the ORF of the genome DNA of VZV MAV/06 strain.

Still another object of the present invention is to provide a recombinant expression vector including the genome DNA of VZV MAV/06 strain or the ORF thereof.

Still another object of the present invention is to provide a transformant including the genome DNA of VZV MAV/06 strain or the ORF thereof.

Still another object of the present invention is to provide a method of preparing protein coded by the genome DNA of VZV MAV/06 strain or the ORF thereof.

Still another object of the present invention is to provide a vaccine composition containing the protein of VZV MAV/06 strain as an active ingredient.

### [Technical Solution]

In order to achieve the objects, the present invention provides genome DNA of VZV MAV/06 strain and an ORF thereof.

Hereinafter, the present invention will be described in detail.

As used herein, the term 'open reading frame' or 'ORF', which is a DNA base sequence means a DNA base sequence translated into an amino acid sequence, means a base sequence from a translation initiation codon (for example, ATG) to a termination codon (for example TGA, TAA, and TAG).

In one general aspect, the present invention provides genome DNA of Varicella-zoster virus (VZV) MAV/06 strain having a base sequence of SEQ ID NO. 1.

In another general aspect, the present invention provides an ORF of the genome DNA of VZV MAV/06 strain. In more detail, the ORF may be selected from an ORF 0 having a base sequence of SEQ ID NO. 2, an ORF 17 having a base sequence of SEQ ID NO. 3, an ORF 29 having a base sequence of SEQ ID NO. 4, an ORF 56 having a base sequence of SEQ ID NO. 5, and an ORF 60 having a base sequence of SEQ ID NO. 6.

In another general aspect, the present invention provides a protein coded by the genome DNA of VZV MAV/06 strain or the ORF thereof. In more detail, the present invention provides a protein coded by genome DNA of VZV MAV/06 strain having a base sequence of SEQ ID NO. 1 or an ORF selected from an ORF 0 having a base sequence of SEQ ID NO. 2, an ORF 17 having a base sequence of SEQ ID NO. 3, an ORF 29 having a base sequence of SEQ ID NO. 4, an ORF 56 having a base sequence of SEQ ID NO. 5, and an ORF 60 having a base sequence of SEQ ID NO. 6.

In another general aspect, the present invention provides a recombinant expression vector including the genome DNA of VZV MAV/06 strain or the ORF thereof. In more detail, the present invention provides a recombinant expression vector including genome DNA of VZV MAV/06 strain having a base sequence of SEQ ID NO. 1 or an ORF selected from an ORF 0 having a base sequence of SEQ ID NO. 2, an ORF 17 having a base sequence of SEQ ID NO. 3, an ORF 29 having a base sequence of SEQ ID NO. 4, an ORF 56 having a base sequence of SEQ ID NO. 5, and an ORF 60 having a base sequence of SEQ ID NO. 6.

As used herein, the term "recombinant vector", which is an expression vector capable of expressing a target protein in a suitable host cell, means a genetic construct containing an essential regulatory element to which a genetic insert is operably linked so as to be expressed. As used herein, the term "operably linked" means that a nucleic acid expression regulatory sequence and a nucleic acid sequence coding the target protein are functionally linked to each other so as to perform general functions. The operable linkage with the recombinant vector may be performed using a genetic recombinant technology well known in the art to which the present invention pertains, and a region-specific DNA cleavage and linkage may be easily performed using an enzyme generally known in the art to which the present invention pertains.

A suitable expression vector capable of being used in the present invention may include a signal sequence for membrane targeting or secretion in addition to expression regulatory elements such as a promoter, an initiation codon, a termination codon, a polyadenylation signal, and an enhancer. The initiation codon and the termination codon may be generally considered as a portion of a nucleotide sequence coding an immunogenic target protein, necessary to be functional in an individual to whom a genetic construct has been administered, and must be in a frame together with the coding sequence. The promoter may be generally constitutive or inducible. An example of the promoter available in prokaryotic cells may include lac, tac, T3, and T7 promoters, but is not limited thereto. An example of the promoter available in eukaryotic cells may include β-actin promoter, human hemoglobin promoter, human muscle creatine promoter, and human metallothionein promoter, as well as simian virus 40 (SV40) promoter, mouse mammary tumor virus (MMTV) promoter, human immunodeficiency virus (HIV) promoter such as the HIV Long Terminal Repeat (LTR) promoter, Moloney virus promoter, cytomegalovirus (CMV) promoter, Epstein Barr virus (EBV) promoter, and Rous sarcoma virus (RSV) promoter, but is not limited thereto. The expression vector may include a selectable marker for selecting host cells containing the vector. The selectable marker is to select cells transformed by the vector, and as the selectable marker, markers conferring selectable phenotypes such as drug resistance, nutrient requirement, cytotoxic agent resistance, or expression of surface proteins may be used. Since only cells expressing the selectable marker survive in the environment treated with a selective agent, transformed cells may be selected. Further, in the case of a replicable expression vector may include a replication origin, which is a specific nucleic acid sequence that initiates replication. As the recombinant expression vector, a vector having various shapes such as plasmid, virus, cosmid, and the like, may be used. A kind of recombinant vector is not particularly limited as long as the vector may serve to express the desired gene in various host cells such as prokaryotic cells and eukaryotic cells and produce the desired protein, but a vector capable of producing a foreign protein having a form similar to that in a natural state on a large scale while having a promoter having a strong activity and strong expression capacity may be preferable.

In order to express the protein of VZV MAV/06 strain coded by any one selected from base sequences of SEQ ID NOS. 1 to 6 according to the present invention, various expression host/vector combinations may be used. As an expression vector suitable for eukaryotic cells, an expression regulatory sequence derived from SV40, bovine papilloma virus, adenovirus, adeno-associated virus, cytomegalovirus, and retrovirus, and the like, may be used, but the present invention is not limited thereto. An expression vector usable in bacterial host cells may include a bacterial plasmid obtained from *Escherichia coli* such as pET, pRSET, pBluescript, pGEX2T, pUC vector, col E1, pCR1, pBR322, pMB9, and a derivative thereof, plasmids having a broader host range such as RP4, phage DNA exemplified as various phage lambda derivatives such as λgt10, λgt11, NM989, and other DNA phages such as M13, single-stranded filament type DNA phage, or the like. An expression vector available in yeast cells may be 2°C plasmid and a derivative thereof. A vector useful for insect cells may be pVL941.

The recombinant vector may be inserted in a host cell to form a transformant. In this case, a suitable host cells may be prokaryote cells such as *Escherichia coli Bacillus subtilis, Streptomyces* sp., *Pseudomonas* sp., *Proteus mirabilis or Staphylococcus* sp. In addition, the suitable host cells may be fungi such as *Aspergillus* sp., *yeasts such as Pichia pastoris, Saccharomyces cerevisiae, Schizosaccharomyces* sp., and *Neurospora crassa,* eukaryotic cells such as lower eukaryotic cells, higher eukaryotic cells, for example, insect cells, or the like.

The host cell may be preferably derived from plants and/or mammals. In particular, monkey kidney cells 7 (COS7), NSO cells, SP2/0, Chinese hamster ovary (CHO) cells, W138, baby hamster kidney (BHK) cells, Madin-Darby canine kidney (MDCK) cells, myeloma cell lines, HuT 78 cells, HEK293 cells, and the like, may be used, but the present invention is not limited thereto. Particularly, the CHO cells may be preferable.

As used herein, the term "transformation into host cells" may include any method for introducing nucleic acids into organisms, cells, tissues, or organs and be performed by selecting standard techniques suitable for the host cell as known in the art. These methods may include an electroporation method, a protoplast fusion method, a calcium phosphate (CaPO₄) precipitation method, a calcium chloride (CaCl₂) precipitation method, an agitation method using silicon carbide fiber, an agrobacterium-mediated transformation method, and PEG-, dextran sulfate-, or lipofectamine- and desiccation/inhibition-mediated transformation, but are not limited thereto.

The transformed host cells in which the recombinant vector is expressed are cultured in a nutrient medium, such that the protein of VZV MAV/06 strain coded by any one selected from base sequences of SEQ ID NOS. 1 to 6 according to the present invention may be produced on a large scale. Here, general medium and culturing conditions may be suitably selected according to the host cell. Conditions such as a temperature, pH of the medium, a culturing time, and the like, may be controlled appropriately for cell growth at the time of culturing the cell and mass production of the protein. The protein of VZV MAV/06 strain coded by any one selected from base sequences of SEQ ID NOS. 1 to 6 recombinantly produced as described above may be recovered from lysates of the medium or cells and be separated and purified by a general biochemical separation technology (Sambrook et al., Molecular Cloning: A Laboratory Manual, 2nd Ed., Cold Spring Harbor Laboratory Press (1989); Deuscher, M., Guide to Protein Purification Methods Enzymology, Vol. 182. Academic Press, Inc., San Diego, CA (1990)). As the separation technology, electrophoresis, centrifugation, gel filtration, precipitation, dialysis, chromatography (ion exchange chromatography, affinity chromatography, immuno-adsorption chromatography, size exclusion chromatography), isoelectric focusing, and various modification and composite methods thereof, may be used, but the present invention is not limited thereto.

In another general aspect, the present invention provides a vaccine composition containing the protein of VZV MAV/06 strain coded by any one selected from base sequences of SEQ ID NOS. 1 to 6 as an active ingredient. In this case, the vaccine composition may further contain a pharmaceutically acceptable adjuvant or excipient. Any adjuvant may be used as long as it serves to promote formation of antibody at the time of injecting the adjuvant into the body to thereby achieve the objects of the present invention. Particularly, at least one selected from an aluminum salt (Al(OH)₃ or ALPO₄), squalene, sorbitane, polysorbate 80, CpG, liposome, cholesterol, monophosphoryl lipid (MPL) A, and glucopyranosyl lipid A (GLA) may be preferably used, but the present invention is not limited thereto.

### [Advantageous Effects]

According to the present invention, the entire genome sequence of Varicella-zoster virus (VZV) MAV/06 strain isolated from a Korean patient was analyzed and compared with base sequences of 23 VZV virus strains, such that polymorphism of a length of the genome between species due to deformation of reiteration sequences and an origin of replication may be confirmed. In addition, the entire sequence of VZV MAV/06 strain virus may assist in understanding molecular characteristics of attenuated VZV vaccine virus strains through bioinformatic research, comparative genomic research, and the like.

### [Description of Drawings]

FIG. 1 shows an ORF map of VZV MAV/06 strain (hereinafter, represented by 'Suduvax' in the drawings) according to the present invention;

FIG. 2 shows a phylogenetic tree of 24 VZV virus strains based on the entire nucleotide sequences;

FIG. 3 shows a phylogenetic tree of the 24 VZV virus strains based on non-coding nucleotide sequences;

FIG. 4 shows a characteristic phylogenetic tree of vaccine virus strains based on 12 ORF differences between the vaccine virus strains and clinical virus strains;

FIG. 5 shows correlation between the lengths of the genomes and reiteration sequences of the VZV virus strains;

FIG. 6 shows mutants between an ORF0 of the VZV MAV/06 strain according to the present invention and Oka vaccine virus strains;

FIG. 7 shows a 3-bp deletion region in an ORF17 of the VZV MAV/06 strain according to the present invention;

FIG. 8 shows a 3-bp deletion region in an ORF56 of the VZV MAV/06 strain according to the present invention;

FIG. 9 shows a 15-bp deletion region in an ORF29 of the VZV MAV/06 strain according to the present invention; and

FIG. 10 shows a 3-bp insertion region in an ORF60 of the VZV MAV/06 strain according to the present invention.

### [Best Mode]

Hereinafter, the present invention will be described in detail with reference to Examples and the accompanying drawings. However, the Examples and drawings of the present invention have been disclosed for illustrative purposes, but the scopes of the present invention are not limited thereby.

[Example 1] Virus and DNA Sequencing

A MAV/06 virus strain was obtained by isolating Varicella-zoster virus from blisters of a 33 month old Korean patient infected by chicken pox in 1989 and primarily culturing the isolated VZV in human embryonic lung cells (Hwang, K.K., Park, S.Y., Kim, S.J., Ryu, Y.W., & Kim, K.H., Restriction Fragment Length Polymorphism Analysis of Varicella-Zoster Virus isolated in Korea. J. Kor. Soc. Virol. 21, 201-210, 1991).

The cultured MAV/06 virus strain was sub-cultured 55 times in human and guinea pig embryonic diploid lung cells at 34 to 36°C, and it was confirmed through cell sensitivity and temperature sensitivity tests that the sub-cultured MAV/06 virus strain was attenuated (Hwang et al., Marker test for attenuation of varicella-zoster viruses isolated in Korea). The attenuated virus strain was named as 'MAV/06' and used to produce Suduvax.

The attenuated MAV/06 virus strain was serially sub-cultured in human embryonic diploid lung cells to prepare a master virus seed, and the prepared master virus seed was serially sub-cultured to prepare a working virus seed.

In addition, "Suduvax", which is a final vaccine, was produced using a working virus seed sub-cultured 5 times, and sequence analysis was performed using the final vaccine.

DNA was extracted from the MAV/06 virus strain stock at a concentration of 5.5 *µ*g/100 *µ*ℓ using a QIAamp DNA mini kit (Macrogen). A DNA sequence was determined by a Genome Sequencer FLX standard system of Roche Diagnostics.

23,722 sequence fragments with an average length of ∼400 bp were obtained and the obtained sequence fragments were assembled and viewed using a Consed program (http://bozeman.mbt.washington.edu/consed/consed.html). An average quality of the sequence fragments was 99.99%. A total of 99.38% of 124,759 sequences was matched with the derived consensus sequence and the coverage was 83 reads per nucleotide. These were aligned against two reference virus strains Dumas (NC_001348) and Varilrix (DQ008354) and gaps between the contigs were filled by PCR Sequencing using primers obtained from the adjacent contigs.

The completed entire genome sequence of the MAV/06 virus strain was registered in NCBI Genbank database (August 9, 2011) and represented by SEQ. ID NO. 1. As shown in SEQ. ID NO. 1, it was confirmed that the genome of the MAV/06 virus strain has a length of 124,759 bp.

[Example 2] Analysis of Open Reading Frames

A location of open reading frames (ORFs) of the MAV/06 virus strain in the entire genome sequence was determined by Blast search for two reference virus strains Dumas (NC_001348) and Varilrix (DQ008354). It may be confirmed that the resulting data included the first and last nucleotide positions of each ORF in the MAV/06 virus strain genome and direction of the ORFs.

The ORF information was verified by ORF finding programs such as CLC Sequence Viewer (version 6.1 http://www.cicbio.com/index.php) and ORF Finder provided by NCBI. When the results of the blast search did not coincide with those of ORF finding programs, the nucleotide sequences of the corresponding ORFs were examined with BioEdit Sequence Alignment Editor (Department of Microbiology, North Carolina State University, version 5.0.9 http://www/mbio.ncsu.edu/BioEdit/bioedit.html) and manually edited, thereby determining positions of initiation and termination codons. Finally, all the allocated ORFs were confirmed by identification of the translated amino acid sequences.

Results of the above-mentioned analysis are shown in the following items (1) to (4).

(1) MAV/06 Virus Strain Genome Structure and ORF Mapping

The structure of the MAV/06 virus strain genome was a typical structure of the VZV virus strain in that the genome may be divided into 6 regions, that is, TRL, UL, IRL, IRS, US, and TRS, and lengths of the regions were 88 bp, 104,799 bp, 88 bp, 7,267 bp, 5,232 b, and 7,276 bp, respectively.

A G+C content of the MAV/06 virus strain genome was approximately 46.1%, and the total genome length was significantly similar to those of 24 VZV virus strains. VZV virus strains analyzed in the present invention, and GenBank accession numbers, genome lengths, and G+C contents thereof were shown in the following Table 1.

**[TABLE 1] INFORMATION ON VZV VIRUS STRAINS ANALYZED IN THE PRESENT INVENTION**

| Strain | Accession Number | Country | Length (bp) | | | | | | | **%**G+C |
|---|---|---|---|---|---|---|---|---|---|---|
| | | | Genome | TRI | Ul | IRl | IRs | Us | TRs | |
| Dumas | NC001348 | Netherlands | 124.884 | 88 | 104,836 | 88 | 7.320 | 5.232 | 7,320 | 46.0 |
| M2DR | DQ452050 | Morocco | 124,770 | 89 | 104,719 | 89 | 7.320 | 5.232 | 7.321 | 46.0 |
| CA123 | DQ457052 | USA | 124,771 | 100 | 104,698 | 98 | 7.322 | 5.232 | 7.321 | 46.0 |
| SD | DQ479953 | USA | 125,087 | 88 | 104,787 | 88 | 7.446 | 5.232 | 7.446 | 46.1 |
| Kel | DQ479954 | USA | 125,374 | 88 | 104,857 | 88 | 7.555 | 5.232 | 7.554 | 46.2 |
| 11 | DQ479955 | Canada | 125.370 | 88 | 104.906 | 88 | 7.529 | 5.232 | 7.527 | 46.2 |
| 22 | DQ479956 | Canada | 124,868 | 88 | 104,689 | 88 | 7.386 | 5.232 | 7.385 | 46.0 |
| 03-500 | DQ479957 | Canada | 125.239 | 88 | 105,299 | 88 | 7.266 | 5,232 | 7.266 | 46.1 |
| 36 | DQ4779958 | Canada | 125.030 | 88 | 104,850 | 88 | 7,387 | 5,232 | 7.385 | 46.1 |
| 49 | DQ479959 | Canada | 125.041 | 88 | 104,916 | 88 | 7.358 | 5.232 | 7.359 | 46.1 |
| 8 | DQ479960 | Canada | 125.451 | 89 | 105,020 | 88 | 7.510 | 5.232 | 7.512 | 46.2 |
| 32p5 | DQ479961 | USA | 124.945 | 88 | 104,760 | 88 | 7.389 | 5,232 | 7.388 | 46.1 |
| 32p22 | DQ479962 | USA | 125.084 | 88 | 104,791 | 88 | 7.443 | 5,232 | 7,442 | 46.1 |
| 32p72 | DQ479963 | USA | 125.169 | 88 | 104,870 | 88 | 7,446 | 5,232 | 7,445 | 46.1 |
| NH29_3 | DQ674250 | USA | 124.811 | 87 | 104,766 | 87 | 7,320 | 5,230 | 7,321 | 46.0 |
| SVETA | EU154348 | Russia | 124.813 | 87 | 104,772 | 87 | 7,319 | 5,230 | 7,318 | 46.0 |
| MSP | AY548170 | USA | 124.883 | 88 | 104.848 | 88 | 7,313 | 5,232 | 7,314 | 46.0 |
| BC | AY548171 | Canada | 125.459 | 88 | 105,326 | 88 | 7,363 | 5,231 | 7,363 | 46.2 |
| HJ0 | AJ871403 | Germany | 124,928 | 89 | 104,752 | 89 | 7,335 | 5,230 | 7,433 | 46.0 |
| pOka | AB097933 | Japan | 125.125 | 88 | 104,798 | 88 | 7,463 | 5.225 | 7,463 | 46.1 |
| vOka | AB0977932 | Japan | 125.078 | 88 | 104,822 | 88 | 7,427 | 5,232 | 7,421 | 46.1 |
| VarilRix | DQ008354 | Japan | 124.821 | 88 | 104.761 | 88 | 7,326 | 5,231 | 7,327 | 46.1 |
| VariVax | DQ008355 | Japan | 124.815 | 88 | 104,758 | 88 | 7,324 | 5,232 | 7,325 | 46.1 |
| MAV06 | This study | Korea | 124.759 | 88 | 104,799 | 88 | 7.276 | 5,232 | 7,276 | 46.1 |

The MAV/06 virus strain included 74 ORFs. It was confirmed that among them, 64 ORFs were UL genes, and 4 ORFs were US genes.

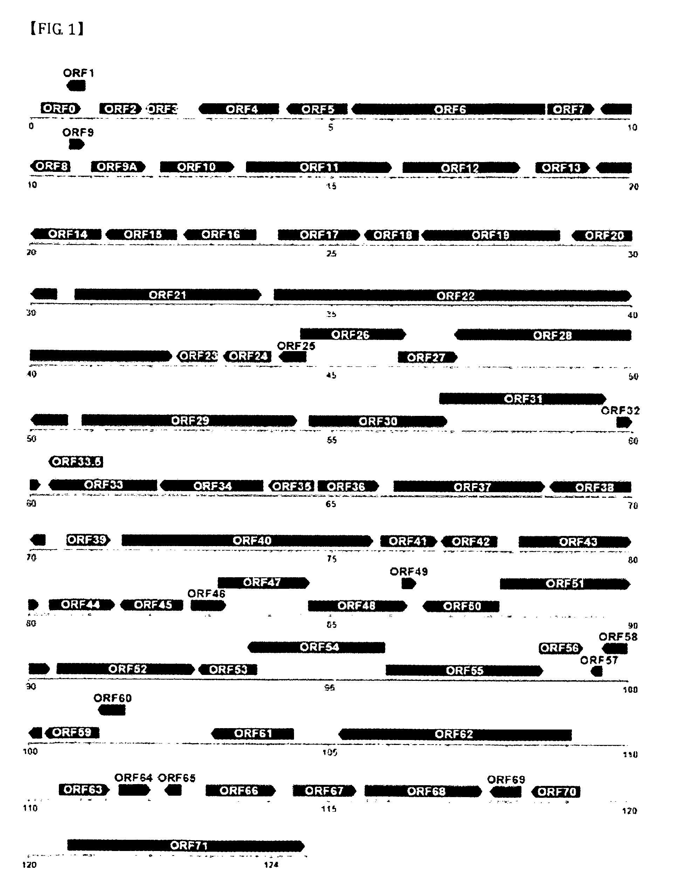

3 genes (ORFs 62-64) in IRS were repeatedly present in a reverse direction in the TRS (ORFs 69-71), and among 74 ORFs, 70 ORFs were present in a forward direction, 34 ORFs were present in the reverse direction. In addition, the directions of ORFs were 100% conserved among the VZV strains. The ORF map of the MAV/06 virus strain (represented by 'Suduvax' in all of the drawings) was shown in FIG. 1.

(2) Phylogenetic analysis of MAV/06 virus strain

Based on the entire nucleotide sequences of the virus strains of Table 1, phylogenetic trees were constructed using a neighbor-joining method.

The ORF nucleotide sequence of the MAV/06 virus strain was multiple-aligned with the nucleotide sequences and amino acids of 24 VZV strains of Table 1 registered in NCBI GenBank database, by way of ClustalW (ver. 2.0.1), followed by manual editing.

The resulting output files were used in order to construct the phylogenetic trees using Dnadist and neighbor programs included in Phylip package (version 3.69, http://evolution.genetics.washington.edu/phylip.html). Distance matrix was obtained by Kimura-2-parameter. Cluster analysis was performed by a neighbor-joining (NJ) method and a maximum-likelihood (ML) method, and the resulting tree files were viewed by the Treeview program (version 1.6.6). Significance of the phylogenetic trees was verified by bootstrap analysis. Phylogenetic trees were constructed from 1000 replicates generated by the Seqboot program and the consensus tree was identified by the Consense program.

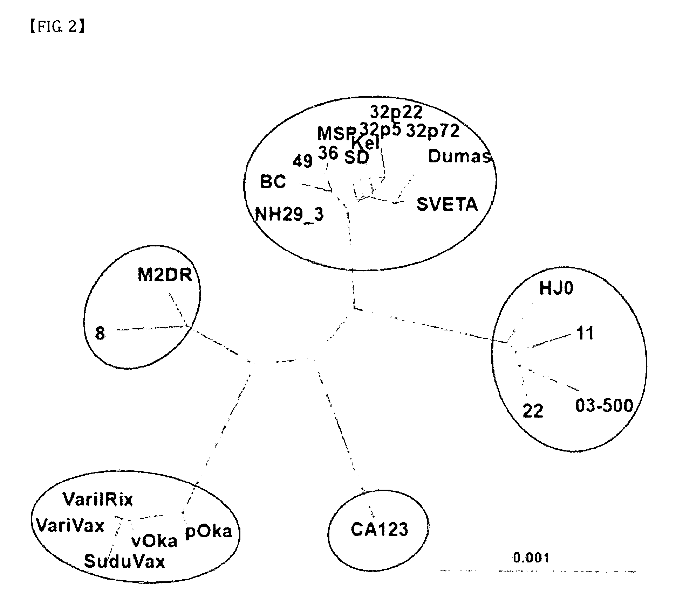

The phylogenetic tree was constructed by the maximum-likelihood (ML) method based on the entire nucleotide sequences. As a result, virus strains (vOka, Varilrix, and Varivax) including the MAV/06 virus strain, which were virus strains of 4 live attenuated virus vaccines, formed a cluster, and M2DR virus strain and 8 virus strains formed a cluster adjacent thereto, as shown in FIG. 2. In addition, CA123 virus strain independently formed a third cluster, 11, 22, 03-500, and HJ0 virus strains formed a fourth cluster, and the rest of the clinical virus strains formed the other cluster.

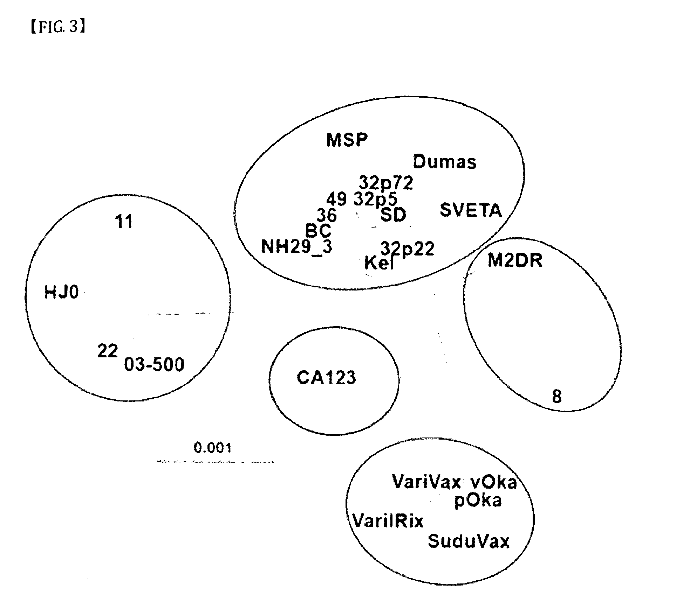

As the result obtained by constructing the phylogenetic tree using the maximum-likelihood (ML) method based on the non-coding nucleotide sequences, the MAV/06 virus strain isolated from a Korean patient and Oka virus strain isolated from a Japanese patient formed a unique cluster (Japan-Korean (J-K) cluster) as shown in FIG. 3. The pOka virus strain was positioned between 4 vaccine virus strains (vOka, Varilrix, Varivax, and MAV/06) and 19 clinical virus strains in the phylogenetic tree of the 24 VZV virus strains constructed based on the entire nucleotide sequences and common amino acid sequences but positioned between the vaccine virus strains in the phylogenetic tree of the 24 VZV virus strains constructed based on the non-coding nucleotide sequences. That is, in other words, it was confirmed that the 4 vaccine virus strains (vOka, Varilrix, Varivax, and MAV/06) formed a subclade in the Japan-Korean (J-K) cluster in the phylogenetic tree of the 24 VZV virus strains constructed based on the entire nucleotide sequences and common amino acid sequences but did not form the subclade in the phylogenetic tree of the 24 VZV virus strains constructed based on the non-coding nucleotide sequences.

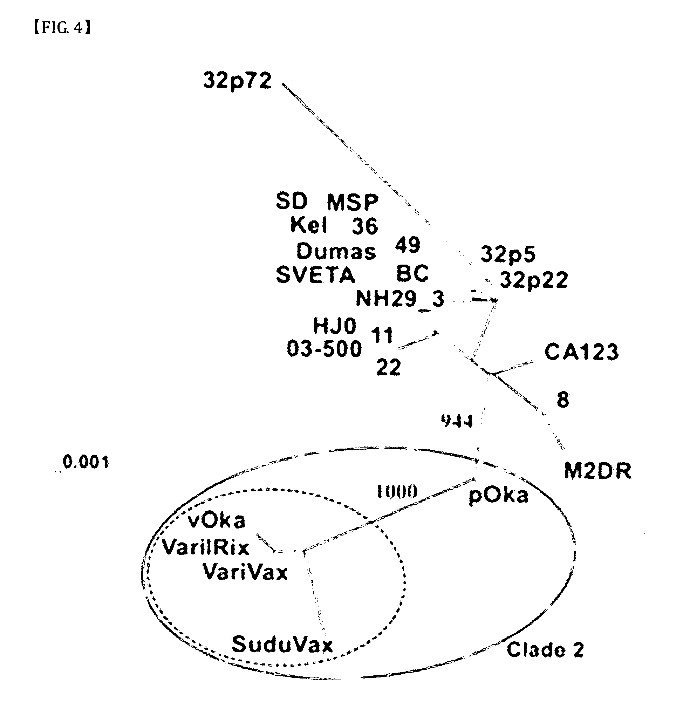

In order to confirm main ORFs for distinguishing the vaccine virus strains and clinical virus strains from each other, 74 phylogenetic trees according to the ORFs were constructed. Among them, vaccine virus strain-specific phylogenetic trees were confirmed in 12 ORFs, as shown in FIG. 4. As the ORFs capable of obtaining the vaccine virus strain-specific phylogenetic tree, there are ORFs 0, 1, 6, 18, 31, 35, 39, 59, 62, 64, 69, and 71.

(3) Reiteration Sequences and Polymorphism Analysis of Length of Genome

Lengths of the ORFs of the VZV virus strains were hardly changed. It may be confirmed that among 74 ORFs of the VZV virus strains, there was almost no difference in a length of the ORF 63 between the VZV virus strains. As shown in the following Table 2, it may be confirmed that polymorphism of the length of 3 ORFs (ORFs 11, 14, and 22) between the strains was relatively high due to lengths of the reiteration sequences.

**[TABLE 2] REITERATION SEQUENCE OF VZV VIRUS STRAINS**

| Strain | Length (bp) | | | | | | | | | Rank⁴ (R) | Rank⁴ (G) |
|---|---|---|---|---|---|---|---|---|---|---|---|
| | R1 | R2 | R3 | R4a | R4b | R5 | R¹ | G² | G-R³ | | |
| Dumas | 303 | 326 | 72 | 146 | 146 | 200 | 1.193 | 124.884 | 123.691 | 9 | 10 |
| M2DR | 303 | 284 | 27 | 146 | 146 | 200 | 1.106 | 124.770 | 123,664 | 3 | 2 |
| CA123 | 261 | 284 | 36 | 146 | 146 | 200 | 1,073 | 124.771 | 123.698 | 1 | 3 |
| SD | 258 | 326 | 72 | 281 | 281 | 200 | 1,418 | 125.087 | 123,669 | 16 | 17 |
| Kel | 321 | 326 | 72 | 389 | 389 | 200 | 1.697 | 125,374 | 123.677 | 21 | 22 |
| 11 | 225 | 326 | 225 | 362 | 362 | 200 | 1,700 | 125.370 | 123,670 | 22 | 21 |
| 22 | 195 | 284 | 72 | 200 | 200 | 200 | 1.151 | 124.868 | 123,717 | 8 | 8 |
| 03-500 | 225 | 284 | 657 | 92 | 92 | 200 | 1.550 | 125.239 | 123.689 | 20 | 20 |
| 36 | 321 | 326 | 72 | 227 | 227 | 200 | 1,373 | 125.030 | 123.657 | 13 | 13 |
| 49 | 321 | 410 | 54 | 200 | 200 | 200 | 1.385 | 125.041 | 123,656 | 14 | 14 |
| 8 | 258 | 620 | 36 | 335 | 335 | 200 | 1,784 | 125.451 | 123,667 | 23 | 23 |
| 32p5 | 243 | 326 | 54 | 227 | 227 | 200 | 1,277 | 124.945 | 123,668 | 12 | 12 |
| 32p22 | 291 | 326 | 36 | 281 | 281 | 200 | 1.415 | 125.084 | 123,669 | 15 | 16 |
| 32p72 | 291 | 326 | 117 | 281 | 281 | 200 | 1,496 | 125.169 | 123,673 | 19 | 19 |
| NH29_3 | 258 | 326 | 51 | 146 | 146 | 200 | 1,127 | 124,811 | 123,684 | 4 | 4 |
| SVETA | 243 | 326 | 72 | 146 | 146 | 200 | 1,133 | 124,813 | 123,680 | 5 | 5 |
| MSP | 258 | 368 | 90 | 146 | 146 | 200 | 1.208 | 124,883 | 123,675 | 10 | 9 |
| BC | 258 | 326 | 612 | 200 | 200 | 200 | 1,796 | 125.459 | 123,663 | 24 | 24 |
| HJ0 | 225 | 326 | 72 | 146 | 254 | 200 | 1,223 | 124,928 | 123,705 | 11 | 11 |
| pOka | 225 | 326 | 36 | 281 | 281 | 312 | 1,461 | 125,125 | 123,664 | 18 | 18 |
| vOka | 258 | 326 | 27 | 254 | 254 | 312 | 1.431 | 125,078 | 123,647 | 17 | 15 |
| VarilRix | 258 | 326 | 72 | 146 | 146 | 200 | 1,148 | 124,821 | 123,673 | 6 | 7 |
| VariVax | 258 | 326 | 72 | 146 | 146 | 200 | 1,148 | 124,815 | 123,667 | 6 | 6 |
| MAV06 | 273 | 326 | 99 | 92 | 92 | 200 | 1,082 | 124.759 | 123,677 | 2 | 1 |
| Avg | 263.8 | 336.5 | 116.9 | 209.0 | 213.5 | 209.3 | 1,349 | 125,024 | 23.675 | | |
| Stdev | 33.8 | 65.8 | 164.5 | 83.0 | 82.4 | 31.6 | 229.7 | 224.1 | 16.0 | | |
| CV | 0.128 | 0.196 | 1.407 | 0.397 | 0.386 | 0.151 | 0.170 | 0.00179 | 0.00013 | | |

| | | | | | | | | | | | |
|---|---|---|---|---|---|---|---|---|---|---|---|
| ¹ Sum of R sequences ² Length of the genome ³ Length of the genome after deletion of R sequences ⁴ Rank in the order of length from the shortest to the longest | | | | | | | | | | | |

That is, R1 was positioned in the ORF 11 and formed by combining various sequences of an 18-bp element (consensus: GGACGCGATCGACGACGA) and a 15-bp element (consensus: GGGAGAGGCGGAGGA). Since a 15-bp element in the ORF 11 was additionally repeated in the MAV/06 virus strain, the length thereof was different from that of the Oka vaccine virus strain.

In addition, R2 was positioned in the ORF 14 and configured various 42-bp elements (consensus: ACCTCGGCCGCTT/aCCCGAAAG/taCCCGATCCCGCCGTCGCGCCC, here, a small letter indicates a small deviation of the sum), and a part thereof (32-bp element) was further included in the R2. In the MAV/06 virus strain, the 42-bp element in the ORF 11 was repeated 7 times as in the Oka vaccine virus strain.

Further, R3 was positioned at the center of 3-end of the ORF 22, and a 9-bp element was repeatedly replicated. A consensus sequence of the 9-bp element was GC/tCCGC/tG/cCA/g (here, a small letter indicates a small deviation of the sum). A repetition number (n) was significantly different between the strains. In the 03-500 virus strain, the repetition number was 73, and in the vOka virus strain, the repetition number was 3. In the MAV/06 virus strain, a repetition number of a 9-bp element was 11, but in the Varilrix and Varivax virus strains, the repetition number was 8.

In addition, R4 and R5 were non-coding regions. R4 was configured of a 27-bp element (consensus: CCCCGCCGATGGGGAGGGGGCGCGGTA), and a part (11-bp element) thereof was further included in the R4. R4 (R4a) was positioned between the ORFs 62 and 63 in the IRS, and R4b complementary thereto was positioned between the ORFs 70 and 71 in the TRS. A length of the R4a was the same as that of the R4b, but in the case of the HJ0 virus strain, the lengths of the R4a and R4b were different. Since in the R4b of the HJ0 virus strain, the 27-bp element was further repeated 4 times, the length of the R4b was longer than that of the R4a by 108 bp. In the case of the Varilrix and Varivax virus strains, the 27-bp element was further repeated 5 times. In the MAV/06 virus strain, a 27-bp element was further repeated 3 times.

R5 was positioned between the ORFs 60 and 61 and configured of an 88-bp element including a 24-bp element. The two elements were different from other reiteration sequences. Two types of reiteration sequences were present. The MAV/06 virus strain included two 88-bp elements and one 24-bp element as most of the virus strains, but the vOka and pOka virus strains included three 88-bp elements and two 24-bp elements.

It was confirmed by comparing the lengths of the genome sequences of the 24 VZV virus strains with the lengths of the reiteration sequences that the polymorphism of the lengths of the genomes of the VZV virus strains was due to the length of the reiteration sequence. The BC virus strain includes the longest reiteration sequence as the longest genome, but the MAV/06 virus strain includes the second-shortest reiteration sequence as the shortest genome. A length (700 bp) between the longest genome and the shortest genome was similar to a difference (724 bp) between the longest reiteration sequence and the shortest reiteration sequence. If the reiteration sequence was deleted from the genome, the length of the genome was decreased together with a change in coefficient of variation (CV). When the reiteration sequence was deleted from the entire genome, 0.000129 was deleted, and the CV was calculated as 0.00179.

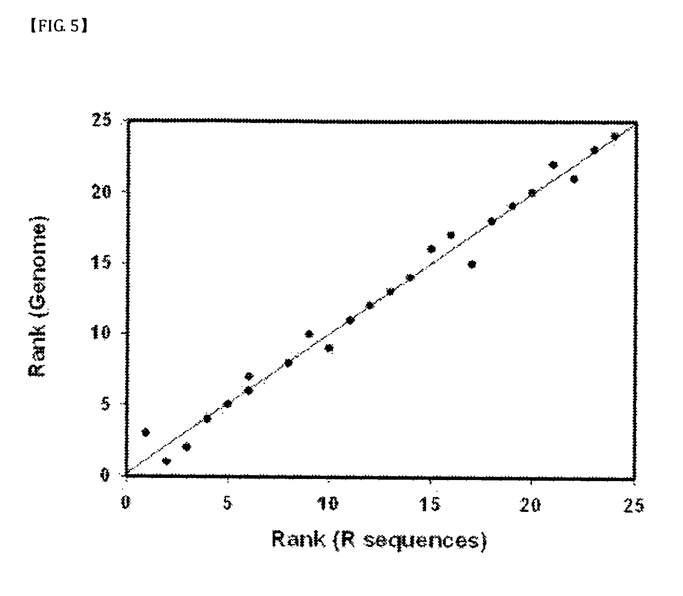

Actually, as shown in FIG. 5, when an order of the length of the genome was set as an order of the length of the reiteration sequences, correlation coefficient (r²) was 0.9854, and a linear relationship between the lengths of the genomes of the VZV virus strains and the reiteration sequences was almost established.

(4) Origin of Replication

An origin of replication (ORI) sequence of the VZV virus strains was positioned between the ORFs 62 and 63. A length of the ORI sequence in the 36 virus strain was 80 bp and a length of the ORI sequence in the HJ0 virus strain was 108 bp since repetition numbers of TA and GA dinucleotides in arrangement of the ORI sequence were different from each other.

As shown in the following Table 3, the ORI sequence in the MAV/06 virus strain was positioned between the 110,080th and 110,183th positions of the genome, and 15 GA tandem repeats and 11 TA tandem repeats were included therein.

**[TABLE 3]**

| Strain | Start | End | Length | (TA)n¹ | (GA)m² |
|---|---|---|---|---|---|
| Dumas | 110,166 | 110,263 | 98 | 15 | 8 |
| M2DR | 110,052 | 110,149 | 98 | 15 | 8 |
| CA123 | 110,050 | 110,147 | 98 | 15 | 8 |
| SD | 110,250 | 110,337 | 88 | 11 | 7 |
| Kel | 110,431 | 110,514 | 84 | 9 | 7 |
| 11 | 110,455 | 110,538 | 84 | 8 | 8 |
| 22 | 110,075 | 110,180 | 106 | 13 | 14 |
| 03-500 | 110,580 | 110,667 | 88 | 11 | 7 |
| 36 | 110,262 | 110,341 | 80 | 7 | 7 |
| 49 | 110,300 | 110,379 | 80 | 7 | 7 |
| 8 | 110,541 | 110,632 | 92 | 13 | 7 |
| 32p5 | 110,171 | 110,254 | 84 | 9 | 7 |
| 32p22 | 110,256 | 110,339 | 84 | 9 | 7 |
| 32p72 | 110,335 | 110,420 | 86 | 10 | 7 |
| NH29_3 | 110,094 | 110,191 | 98 | 15 | 8 |
| SVETA | 110,101 | 110,194 | 94 | 13 | 8 |
| MSP | 110,176 | 110,265 | 90 | 12 | 7 |
| BC | 110,708 | 110,795 | 88 | 11 | 7 |
| HJ0 | 110,085 | 110,192 | 108 | 11 | 17 |
| pOka | 110,268 | 110,371 | 104 | 11 | 15 |
| vOka | 110,264 | 110,359 | 96 | 8 | 14 |
| VarilRix | 110,096 | 110,191 | 96 | 8 | 14 |
| VariVax | 110,093 | 110,190 | 98 | 8 | 15 |
| MAV06 | 110,080 | 110,183 | 104 | 11 | 15 |

| | | | | | |
|---|---|---|---|---|---|
| ¹ **THE NUMBER OF TA TANDEM REPEATS** ² **THE NUMBER OF GA TANDEM REPEATS** | | | | | |

The numbers of tandem repeats in 110,235 positions of the genome were different, but in the Oka vaccine virus strain and MAV/06 virus strain, generally, A was substituted with G in the GA tandem repeats at one position of the nucleotide positions corresponding to 110,235 positions in the Dumas virus strain, which is a reference virus strain. The ORI sequence was accurately complementarily duplicated between the ORFs 70 and 71 except for in the HJ0 and Varilrix virus strains: (TA)3(GA)2 was deleted in the HJ0 virus strain, and one GA tandem repeat was deleted in the Varilrix virus strain.

(5) Analysis of Characteristics of ORF of MAV/06 Virus Strain

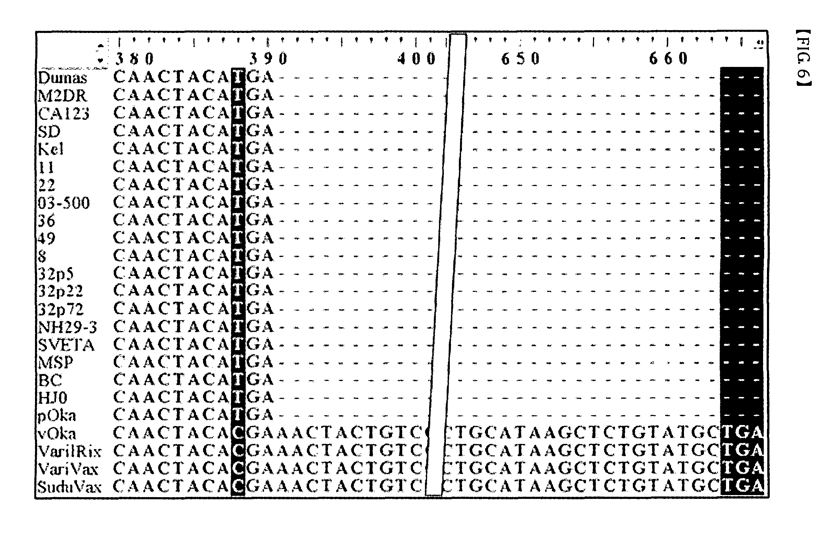

A length of the ORF0 of the MAV/06 virus strain was increased due to mutation. TGA (388-390th nucleotide position), which is a termination codon, was mutated into CGA coding arginine (Agr). TGA estimated as the termination codon was founded at a lower end region overlapped with the ORF1. The expanded ORF0 encoded a new protein having 221 amino acid residues. The same mutation was confirmed in other similar vOka, Varilrix, and Varivax vaccine virus strains (FIG. 6).

Among all of the clinical virus strains, the pOka virus strain included an ORF0 coding 129 amino acids and having a length of 390 bp.

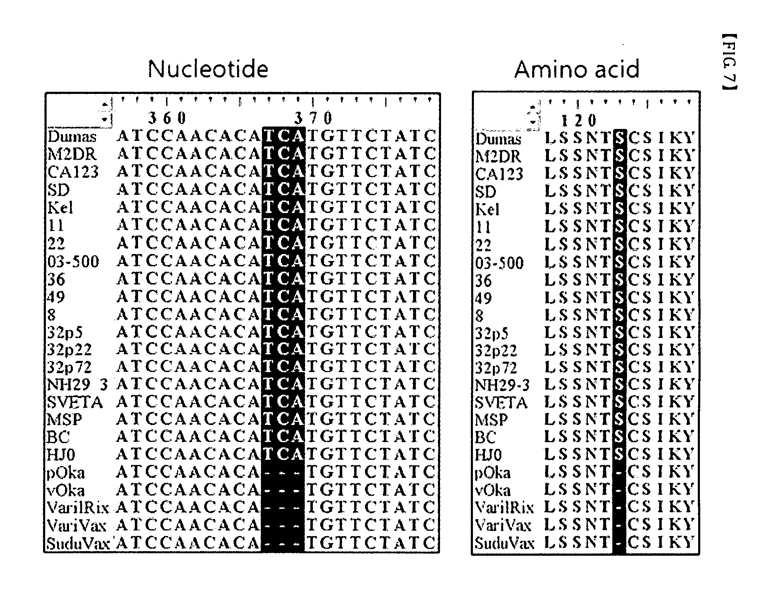

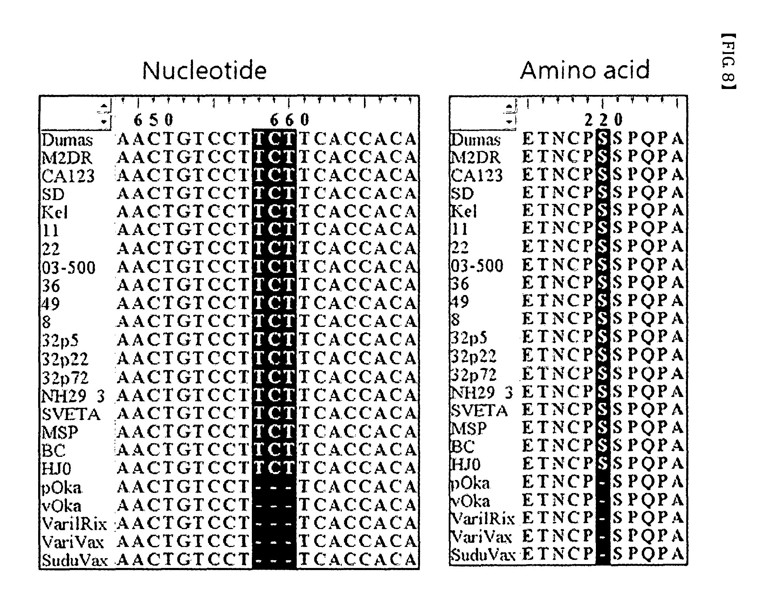

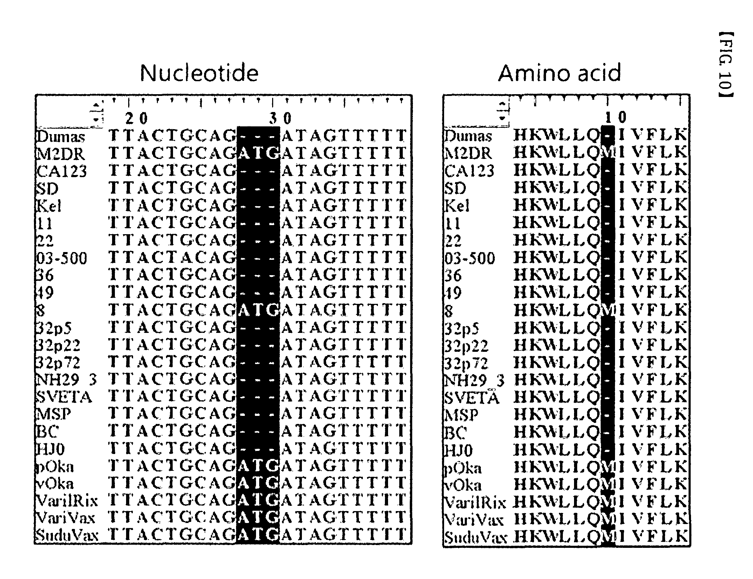

As compared with the Dumas virus strain that is the reference virus strain, since TCT at the 658th to 660th position and TCA at the 367th to 369th position were deleted from the ORF17 (SEQ ID NO. 3) and the ORF56 (SEQ ID NO. 5) of the MAV/06 virus strain, respectively, the lengths of the ORF17 and the ORF56 were shortened by 3 bp (FIGS. 7 and 8). All of the above-mentioned deletions caused deletion of a serine (S) amino acid residue. On the other hand, in the ORF60 (SEQ ID NO. 6) of the MAV/06 virus strain, three nucleotides (ATG) were inserted at a 28th position (FIG. 10). Interestingly, one insertion and two deletions were found in all of the Oka vaccine virus strains including the pOka virus strain.

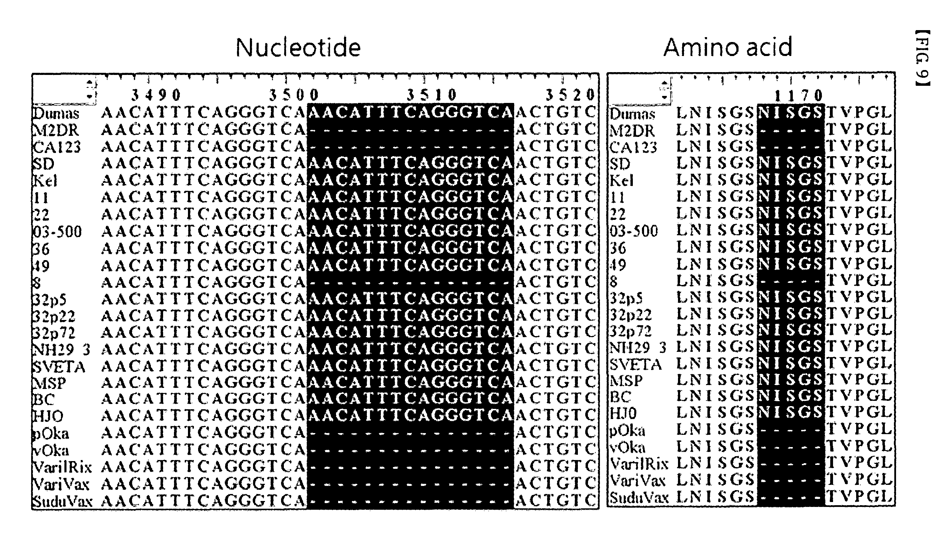

In addition, in the MAV/06 virus strain as well as the Oka vaccine virus strains, the length of the ORF29 (SEQ ID NO. 4) was shorter than those of the ORF29 in the Dumas strain and most of the clinical virus strains by 15 bp (AACATTTCAGGGTCA). The Dumas strain, which was the reference virus strain, and most of the clinical virus strains continuously and repetitively included a 15 bp-sequence. In the M2DR, CA123, and 8 clinical virus strains, the length of the ORF60 was the same as that of the ORF60 in the Oka vaccine virus strain and the MAV/06 virus strain (FIG. 9).

### [Sequence list pretext]

SEQ ID NO. 1 is a sequence of genome DNA Of VZV MAV/06 strain.

SEQ ID NO. 2 is an ORF0 of the genome DNA Of VZV MAV/06 strain.

SEQ ID NO. 3 is an ORF17 of the genome DNA Of VZV MAV/06 strain.

SEQ ID NO. 4 is an ORF29 of the genome DNA Of VZV MAV/06 strain.

SEQ ID NO. 5 is an ORF56 of the genome DNA Of VZV MAV/06 strain.

SEQ ID NO. 6 is an ORF60 of the genome DNA Of VZV MAV/06 strain.

## Claims

1. A genome DNA of Varicella-zoster virus (VZV) MAV/06 strain having a base sequence of SEQ ID NO. 1.

2. An open reading frame (ORF) of genome DNA of VZV MAV/06 strain selected from an ORF0 having a base sequence of SEQ ID NO. 2, an ORF17 having a base sequence of SEQ ID NO. 3, an ORF29 having a base sequence of SEQ ID NO. 4, an ORF56 having a base sequence of SEQ ID NO. 5, and an ORF60 having a base sequence of SEQ ID NO. 6.

3. A protein coded by the open reading frame of genome DNA of VZV MAV/06 strain of claim 2 or 3.

4. A recombinant expression vector comprising: genome DNA of VZV MAV/06 strain having a base sequence of SEQ ID NO. 1 or an ORF selected from an ORF0 having a base sequence of SEQ ID NO. 2, an ORF17 having a base sequence of SEQ ID NO. 3, an ORF29 having a base sequence of SEQ ID NO. 4, an ORF56 having a base sequence of SEQ ID NO. 5, and an ORF60 having a base sequence of SEQ ID NO. 6.

5. Host cells transformed by the recombinant expression vector of claim 4.

6. The host cells of claim 5, wherein they are selected from animal cells, plant cells, yeasts, *Escherichia coli,* and insect cells.

7. The host cells of claim 6, wherein they are selected from monkey kidney cells 7 (COS7), NSO cells, SP2/O cells, Chinese hamster ovary (CHO) cells, WI38 cells, baby hamster kidney (BHK) cells, Madin-Darby canine kidney (MDCK) cells, myeloma cell lines, HuT 78 cells, HEK293 cells, *Escherichia coli, Bacillus subtilis, Sireptomyces* sp., *Pseudomonas* sp., *Proteus mirabilis or Staphylococcus* sp., *Aspergillus* sp., *Pichia pastoris Saccharomyces cerevisiae, Schizosaccharomyces* sp., *and Neurospora crassa.*

8. A method of preparing a protein of VZV MAV/06 strain coded by any one base sequence selected from SEQ ID NOS. 1 to 6.

9. A vaccine composition comprising: the protein of VZV MAV/06 strain of claim 8 as an active ingredient.

10. The vaccine composition of claim 9, further comprising a pharmaceutically acceptable adjuvant serving to promote formation of an antibody at the time of injecting the adjuvant into the body.

11. The vaccine composition of claim 10, wherein the adjuvant is at least one selected from an aluminum salt (Al(OH)₃ or ALPO₄), squalene, sorbitane, polysorbate 80, CpG, liposome, cholesterol, monophosphoryl lipid (MPL) A, and glucopyranosyl lipid A (GLA).


<110>     Mogam Biotechnology Research Institute

<120>     Novel Varicella Zoster virus and vaccines against chicken pox and

<130>     P13070970966

<150>     PCT/KR 61/446284
<151>     2011-02-24

<160>     6

<170>     KopatentIn 2.0

<210>     1
<211>     124758
<212>     DNA
<213>     Varicella-zoster virus

<400>     1
ggcccagccc tctcgcggcc ccctcgagag agaaaaaaaa aagcgacccc acctccccgc        60

gcgtttgcgg ggcgaccatc gggggggatg ggattttttg ccgggaaacc ccccccgcca       120

gcctttaaca aaacccgcgc cttttgcgtc cacccctcgt ttactgctcg gatggccacc       180

gtgcactact cccgccgacc tgggaccccg ccggtcaccc tcacgtcgtc ccccggcatg       240

gatgacgttg cgacccccat tccctaccta cccacatacg ccgaggccgt ggcagacgcg       300

cccccccctt acagaagccg cgagagtctg gtgttctccc cgcctctttt tcctcacgtg       360

gagaatggca ccacccaaca gtcttacgat tgcctagact gcgcttatga tggaatccac       420

agacttcagc tggcttttct aagaattcgc aaatgctgtg taccggcttt tttaattctt       480

tttggtattc tcacccttac tgctgtcgtg gtcgccattg ttgccgtttt tcccgaggaa       540

cctcccaact caactacacg aaactactgt ccggaagggg aaggtattta ttctcgcttg       600

cagcttgtcg cgcgtgtatg cacaacaaaa gctatatatg tcaccaaagc caacgtcgcc       660

atctggagta ctacacccag tacattgcat aacctgtcca tctgcatttt cagttgcgcg       720

gacgcctttc tccgggatcg tggccttggg acatcaacca gcggaataag aaccgccggt       780

ggtcttgccc gaacgacgag tggcgacgcg ttgttctgca taagctctgt atgctgatac       840

ataaacacag agtctgtatc gctatcagat tcccgaacac cttccggtac cccatactcc       900

gataccctgg acattgcgga tcccaaaaat ataatattaa caggatttgc ttatactttg       960

ctacagctta tataaattta tgtgcgatac atcttaagtg catccgtacg ttatttatac      1020

attgcctgtc acgtgaaaag actgtgttac ccaataaagg ttctacaaaa aatgctttat      1080

tgggtgtttg tttaatagct attatcgtaa cccacccccg taaaatcata aaatgcatgt      1140

aatttctgag acacttgcat atgggcatgt tcccgcattt attatgggct ccactctggt      1200

gcgtcccagt ttaaacgcca ccgccgagga aaatcccgcg tcagaaacgc gatgtttatt      1260

acgagtgctt gcggggagaa ctgtagacct gccaggcgga ggaacgttac acattacctg      1320

taccaaaacc tatgtaatta ttggcaaata tagcaaaccc ggcgaacgtc ttagccttgc 1380

ccgtctaata gggcgtgcaa tgacgcctgg aggtgcaagg acatttatta ttttggcgat 1440

gaaggaaaag cgatccacaa cgcttgggta tgaatgtggt acgggcttgc atttactggc 1500

tccatctatg ggtacatttc tccgcacaca cggtttaagt aacagagatc tctgtttatg 1560

gcggggtaat atttatgata tgcatatgca acgtcttatg ttttgggaga atatcgcgca 1620

aaataccact gaaacacctt gtataacgtc gacgttaaca tgcaacttga cagaagactc 1680

tggtgaagcc gcacttacca cgtcagaccg acccactctc ccaaccctaa cagcccaagg 1740

aagaccaaca gtttccaaca ttcgtggaat attgaaagga tccccccgtc aacagccggt 1800

ctgtcaccgg gttagatttg ccgaacctac ggagggcgta ttgatgtaat cactaaataa 1860

aatacacctt ttttcgattg tacgtatttt tatttaaatg tgtagttcat agtccgccga 1920

cagccgctcg ggcttttccc ccacatacaa catgatcgta tgcctcggat gcaccggtcc 1980

aacactccgc cgagaagggg gatttacaat gacagtgata cccaatagcc gccagatgta 2040

cacccagctg tccggactcc agcatcatct gctgagttgc ggcgctgaag ggtgcatcgc 2100

atagggtgtt ataattagcc atttccggta acagtcgttg ggaatttagg aggctgcaaa 2160

acggctgtag gtcaacatac attggggatt cagatggttt atctcgacgt ccaagtccaa 2220

tcaaaaaagc gtgtaaatca tcagcccggc cgcatgttgc tcgaagagca cataacctct 2280

taacaccgta cagaggggat ggcgtcggtg catgtgagtt ggcagggcat gtccacgttg 2340

tttccaacgc cagtggcggt ataacttgtg taaacgacgc caacgggtca ggtttaagat 2400

tcactcggat gggttgactg ctttcggaag ctcccgttgt atccattaat taaacgttcg 2460

gtacacgtct ggtgtgtgtt ttacccgaat cagagacgga attgcaaaga tatcggtttg 2520

aaagcaatgt aatcccgccc atatatcccc aacgtcgcct taaaaactcc cacaatatta 2580

catttttatt agtcttttat taatatagaa tcacataaac aattgataaa atcaaggggt 2640

ggtgtataat gattaaaaat ataaattgat atgttttaca agcatgaaat aggtatttac 2700

tattctaaca ggtaaatatg cttaatgatt aaaaatacaa attagtatgt tttgacaagc 2760

atgaaaaagg tattttttat tttagcagtt aaaggtacta cacttaaaat atttaccgta 2820

tggacgggcg tcagaaagat gcccggccca agttgagagg gtacattcaa cacgaccaca 2880

ctcgcgttgg tgggtgatta gggcctctaa aacaccggcc agacatgacc cgggtgtata 2940

ttcttgtaac acttgaacgt tacaactgat atcatcatat tccacaaatt tagagccacg 3000

gacaactata ttagcaatgc gggcaatcat aacaaacata taagtagtaa tacacgtgat 3060

atcactaaaa cgttgctggc gcaacagttc ggggagagta cgagacccca aatcgttgtc 3120

cctgtttaga agaagacatc ttacaaaagg ccccagcttt aactttaaat tctccaaaag 3180

tgacttcgag gttgcaacaa tgggattatt tgtgtagatg ggcaagtttt ttgccgctaa 3240

cattttaatc cacgttaaca gttcatccgc agactccaac gcttcaatca aagattctcc 3300
acgtatgact ctctcacgca acgcgcgggc aatacgtgag tccattttat atgactcaaa 3360
ggtacgataa agttcatgtc cgtacaacat caactccggc caagatgtgt tttgttttat 3420
ccccggaaaa catccaccgg aagcccatga atcaccctct tgtattgtgg catatcggac 3480
taccagtttt tcaattgttt catctaaatg gcgtaccgag tcaatggtca cgctggctcc 3540
cgcggtggag acgacttcaa tagcacggcc cgtaattcga tcgaccggga tatcatactc 3600
ttttcgaata cgctctcggc gggcgtctct cttggaaaat cgcaacctgt acgattcgtc 3660
atgtgtctga tcatttcttt ctcccgtggt cattgcagga ggcgttgtag gacgccgtct 3720
tcgatttgac agggatcgat cacggtgttt tcttgaactt tgggtgttat aagatctgga 3780
tgatcgtcga tgtccccgtt cgatgcgtgc atatccagtc tccacgtctc ttcctccatg 3840
atggtttgaa tcgggtaata caacaaccaa agttttcggg cgattgtggt ggtagctttc 3900
acgccttccg tgccttcgtt tggaataccg tggattatat gctgtatctg cagtacgctc 3960
cacatacaca gttctagacg ttgtggagtc ctcgcctgga gtggagccaa tagcttcatc 4020
atttgcccaa tcggtgactt ccaatgcaaa gtcatccgaa ggttcgtctg gtagcaaatt 4080
cataaagtct tcacaaatag tagacacgtc tgggtcggtt ggaattgaag cagaggccat 4140
ggctgcaaaa tatctgacaa ttgcgtgttt gcagttgcct gtatcttccg ccaatgttgt 4200
agaatttata ggctcaccca accccgcaat gggcgtgttt agtcacatga ttaatgtttc 4260
tgggagtttt cactttcccc aaacaagctt acctgcaccc tttgttcgta atgcataaaa 4320
ataaccactg ctatagcaaa tatgacgata taaaaacatt ttatagcaag gccggacatt 4380
actgtagcgc aacatgttgt gcatatacca cgtattcccc ccgtattgat atgatttaaa 4440
tgattatcct tggttggttt tggtctaaca taagatataa gctctactat agcgagcgtg 4500
catacaacaa cccaggccag aatccgaatg tatgtggggt ataataacgc gcatggtgta 4560
tatgcaacgc caagcgttaa aagcacaata catccagatg atatatgagc gataacctcc 4620
aaaagcatca ataacgtaac acctttatgc atatataaaa aacttatagg gtcagcatta 4680
aatactttac tcataccatc ccgtcgcatg gaaacatcac ataacaacct tgccaacttt 4740
gtatatgggt aaccaagaag aatgttcgaa ataacccgtg ttacgtaatt cagtgaatat 4800
gatgtggggg atattaactc acaggatgat cggaatggcc caaacatacg acgtattcgt 4860
cgaaattgta aatacatacc atatacaaac catgcaaaaa aaatcatttt tagctgcacg 4920
caccaaaaat aagcgtgaca attacgtgtt cccagaacaa ttcgaatttt gtcatgcaaa 4980
ggtgtagaaa tagcggtttt taccatagta tctcctgata atagatttcc ccggcagctg 5040
taatcgtatc cagataggcc atccaaaaac gttgagtggt ttacaaacgt tacatatata 5100

agagagttgt tataagaccc ccatacaacc ggtccaccat taatcaccgt ggttgcatac 5160

acacactcat gttcaaactt tacacgagcg gtataccata gggtaaaaac agcatgtccg 5220

ctaagtagac acataattat aaaatgttct gtcttgattc ctaaagcctg catgacccgt 5280

ggaagatggc aattcaagca cgatgtagta tcacacggtt ggtgttaact cgaagttaaa 5340

tttggataat taggtacttc tagagtaaag attgtatgca tgcgattgct atcgcacttt 5400

gtagcaaaac attgttgtgc aagcgaaata cacaaacggt tgtgatgatc cactcgcaga 5460

gacacaaatg tccggggagc cgttcttcct ccgcgatggg gatatcgaag acaagtgaac 5520

ccttttgttc cgcatatgag ctgaaataac acccagtccc ttttgatggc gatacacttt 5580

gatgatgtta aggtatattc gcgatcacgc ccggggaaat gaacagcaat atgctccaca 5640

atagattcta atattgtgct gtcgacaaag gcctccagtg taaatgcgtc cagacaagtt 5700

accccgcgct cttttagagc ctttgttaaa gatatttgcg gggggctaaa tatttgttta 5760

ttacgcgcaa ccttacgttc aaaaaactct gcgtattccc ccccaaggtt atgtaaaata 5820

aattgcactg gaacattcga ctgcggtctt gaatgaaaat gaaagtttgc cgggtttcta 5880

tgtgatgtca caaacgctaa tatatcaata cactgctcag gtacaacata aaatgggagt 5940

agttgtccaa ccgccgtccc tgtggttgtt actttggaga aaaaaggcag tcttaaacta 6000

tgtccgtggc tataaacacc agtatctata aacgaaaagt cccgtaaata cggaccaata 6060

tattcaacaa attcccgttc cagcaacacc gcttgctgta atatttgtgc aaaccccttt 6120

aaagtggaag accccactaa cgcataggga tttgggattg gtacgcatac cctgaaacct 6180

attttctctt tacagttaca gggtagagtt tcatgcaagt tttcattgtt tgatacatcg 6240

gcgtgtgtat ggacttcaga cgttgtctgt gtatcaaaaa accatacatc ctctgtataa 6300

ttctcttcta cacacgtgta taattcgcca ttttctatgt aaaaatcgat gtcagaatgg 6360

ctggttatat ccaataaatt atcatcatcc aacacctcaa cggtaggttc aggacatgca 6420

gttttataaa aataacatgg gtctttgtta gggtttacca cggcctttgg aaaaagtaat 6480

tgcatggccg ttaaaatacc atgacgaaat gctcgcatgc cggcatgtaa aatacccaat 6540

gggatgggtt ttcttatatg aaagtctaca tcaagtatga ggtttgtgat tataagattt 6600

gtattaaata gctcattcct gtttatataa agctgatctt tgggtatgtt tgatgaaatt 6660

ttagaaacgt ttttaacaga cgtagataat agtaaagtca actgcatatc tcgtagtgaa 6720

gcggcaacaa aattacatgg attaatttgt ttaaggtcct ccgcaattaa tcgagcctcg 6780

tgcggtaaag tgtaacggtt tgttattgat gaccacgtat cattagcaat aacagcaaat 6840

gcttgggcgc cttgaggcaa ggctacccga tatacaggca ttggtccagt tacctcagaa 6900

tggccgatga gggcttctaa tggagtttta taactcagga tggatacatc atgtgtggct 6960

atcccagtgg cagcagagaa aaacagtaat agttttgtaa tccccgggct cgtatcaaaa 7020

ccagtacgac cactttggtt aggtgtatcg tttgcaaagt tggctgctcg taacgcctcc 7080

gcggaaacaa ccgaatcctc aaaattagac aattcgtcaa aaccgggtgg atttgaggga 7140

atagtggagg accatccata tggactaaat tgtttttcaa tgttttccac acgacgagtt 7200

agcgttgtag ctaggtcaca tacgcctata aacttgctag gttttgcggc atacgtaaga 7260

cttaaagtat atgttttagt aattgtatat ttatgtccaa tctcaggtcc aagttcagtg 7320

acatcacaaa ttacgttctt ttttatatag tcacgcatgt tgagacgaga acgtacatga 7380

ttaaaaaaat tagcagtagc tctttttccc aggttggatg attttaagag gaccggttta 7440

ttcacaaaat ctgagtatgt aaccgcttgt aggtggtctg cgatctgttt ccgattgaaa 7500

cattcaaaat gtgccagata aatataatca acaaattcac ggtctggaac tttaaggcct 7560

tttctatcgt tggtaatata ctccgatact gcgtgtattt ccgttgtgtc tgtatgtatt 7620

cgctgtaaaa tgtacgatag agcatttttg gctgtcaaac ctcgtgtata tgttgaggaa 7680

caacaaaaca tggaaagttt atcaaaagac aacaagtccg aaatattgta cccactacaa 7740

ttaggtaatg ctgggacttg gtaagttaaa aacaaatctt taattgcctg taagtcatat 7800

aagggggttt ccaacgtatt gtaacttgtg tccgtttgta acaagtaata gcgtgtagcc 7860

aacactagcg ttttttcaga gggtccaaat cgaacaatat accaaaacgg cgagcatcca 7920

tacccccagt agagtcgtcg atatgcagcc aatacttgac gttcgtaatg ggcatataat 7980

gatgttagct cctgacgacc aacggatttt ttaactaact tgcagagtgt tgcctctgtg 8040

atgcataggc cgttgtccga taatcccttt cggtttaaat ggtgtgttgt taccatcaga 8100

gtttgtataa cttccgagtg aatgtcaaac gtctccgata tacatagggt atcagatatt 8160

atatgcggat ttaggggtgc tccataccat aacgccttat ataaagcttt aaaatcagtt 8220

tgggttttaa aacaacaaaa aaatataggc cagacccggg atcgtacatc tccagttgaa 8280

aatccaccaa ttaaataaaa aataacgttg acgtccctac tacaaaataa atgcattatt 8340

tggttttctt catcgttttc agttacttca cgtgggcgtt tagttgggat tacttgcgtg 8400

atctcttccc tcccattttt gacaaagacg tcatctaagt cgggagtcca agtataactc 8460

accacataca gaggttctgt gcttatctgc ccggtaagca acaacagcga gtgggagatt 8520

gcacatccct ttgtggcaaa taataaccga atcgtcggtt tggaggattt atccatagtt 8580

caatacgttg gaaagccagt caatcatgca gacggtgtgt gccagcttat gtggatatgc 8640

tcgaatacca actgaagagc catcttatga agaggtgcgt gtaaacacgc acccccaagg 8700

agccgccctg ctccgcctcc aagaggcttt aaccgctgtg aatggattat tgcctgcacc 8760

tctaacgtta gaagacgtag tcgcttctgc agataatacc cgtcgtttgg tccgcgccca 8820

ggctttggcg cgaacttacg ctgcatgttc tcgtaacatt gaatgtttaa aacagcacca 8880

ttttactgaa gataaccccg gtcttaacgc cgtggtccgt tcacacatgg aaaactcaaa 8940

acggcttgct gatatgtgtt tagctgcaat tacccatttg tatttatcgg ttggcgcggt 9000

ggatgttact acggatgata ttgtcgatca aaccctgaga atgaccgctg aaagtgaagt 9060

ggtcatgtct gatgttgttc ttttggagaa aactcttggg gtcgttgcta aacctcaggc 9120

atcgtttgat gtttcccaca accatgaatt atctatagct aaaggggaaa atgtgggttt 9180

aaaaacatca cctattaaat cggaggcgac acaattatct gaaattaaac ccccacttat 9240

agaagtatcg gataataaca catctaacct aacaaaaaaa acgtatccga cagaaactct 9300

tcagcccgtg ttgaccccaa aacagacgca agatgtacaa cgcacaaccc ccgcgatcaa 9360

gaaatcccat gttatgcttg tataaatatt gaaataaaaa ctaaaaacgt ttctggtgta 9420

tgtttttatt ttgtatataa aattaaaaca ttgctggccg gcgtggttat tacatttaat 9480

gttttagtag aaaatcgaca tcgtttgttt ctttatcagt tgaaccaaat ccacgcgttc 9540

cccgttcgct gggtgtggct attagatcta acgttttagt aaaataccat tgtacacccg 9600

gtatgccaca tttaccgcgg atagcataag gaaatgcaat attacttaaa acgttgtgtt 9660

ttaagtgtat ttgggtgttg tgatctatta acaggacctg tgcaagacga tctcccgttt 9720

ttatacgtat gtcatcaccc gtgagattat atacgtagaa tttacagtgt tctcctgcag 9780

gccatgccgt tggacacacg ataatgcctg atcggctttt cgatgatctt ccaaaaatat 9840

aagcgtttat actcggatgt tgtaagtccc agtctcttat aatcggtaag acaattttta 9900

taaattcatt cctttttaaa tataggttat atggtacaca aatatcatat cccgcgtctt 9960

cttggcgttt tggattgatg atatgtttgt aggttaaggg aacatcgata tggtattctg 10020

cagaatccct atgtaaaggt tgcccctgct gtaccgtgga aatatcagca aattcagata 10080

taacgggttt ttcataattt gacggcgagt ttgataaggg ttgaacttgt atcgatttaa 10140

aaattggatc cagatgttta agaacgtttt ttgggagaag gcgactttgt cttaatttta 10200

ccgggaacaa gtagattgtt aaatgtccgg gtaaaataac ggttactcct ggccggtaat 10260

acaaaagggc tgaaattact cctctgtaac ccgcatcaat aactccgttg gcgacaaaaa 10320

aattgtcttc atcagcaagg gcagtatctt tgcattgaat taacaacagt gcgtattcat 10380

tgggaggcgc cgacttaacc aacagctcca actgctgcat ataaaaaccg ccccgtgtta 10440

cagatttttc agatggcagt tcgagtttct tgtggttccg gagtaacaac ggttgatgtc 10500

gacttacttt atcgtctaac acgcattgca gcgtatctgc acattcaggt tgaacttcta 10560

ttaaaattgt atcttttaaa caccgattcg gaatagtttg gctacaaaac atatcacctg 10620

tatttactgc cgtttccaag atgggatcaa ttaccgcttc gttcatatta ataacgatgc 10680

aaattttatt tttttgtgaa gacagcagtg gggagccaaa ctttgcagaa cggaattttt 10740

ggcatgccag ctgttcggct cgtggagttt atatcgacgg atcaatgatc accacccttt 10800

tcttctacgc atcccttttg ggggtgtgtg tagcccttat ttcgttagct tatcatgcgt 10860

gtttccggtt atttactcgt tctgtattac gcagcacgtg gtaaacccgt ttgcctataa 10920

aaggggcagg cgtgtataag agggcccctg tttaatacgc ggtctgccgt gtttggatat 10980

ttcacgaccc tatcgtttat ttacgtaatg gcatcttccg acggtgacag actttgtcgc 11040

tctaatgcag tgcgtcgtaa aacaacgcct agttattccg gacaatatcg aaccgcgcgg 11100

cgaagtgtgg tcgtaggacc ccccgatgat tcagacgact cgttgggtta cattaccaca 11160

gttggggccg attctccttc tccagtgtac gcggatcttt attttgaaca taaaaatacg 11220

acccctcgcg tacatcaacc aaacgactcc agcggatcgg aagatgactt tgaagacatc 11280

gatgaagtag tggccgcctt tcgggaggcc cgtttgagac atgaactggt tgaagatgct 11340

gtatatgaaa acccgctaag tgtagaaaaa ccatctagat cttttactaa aaatgcggcg 11400

gttaaaccta aattagagga ttcaccgaag cgagctcccc cgggagcagg cgcaattgcc 11460

agcgggagac caatttcctt cagcactgca ccaaaaaccg caacaagctc gtggtgcggt 11520

cctacgccat catataacaa acgcgtcttt tgtgaagcgg tccggcgcgt agccgccatg 11580

caggcacaaa aggctgccga agcggcttgg aatagtaatc ccccaaggaa taacgccgaa 11640

ttagaccgtt tgttaaccgg agccgttatt cgtattacgg tgcatgaggg tttaaattta 11700

atacaagccg ctaatgaagc agacctaggt gaaggagcat cggtatccaa acgtggacat 11760

aatcgaaaaa ctggagattt acaggggggc atgggtaatg aacctatgta cgcacaagtt 11820

cgtaagccaa aaagtcgaac ggatacacaa acgactgggc gtataactaa tcgaagtagg 11880

gcccgttcgg catcaagaac tgatgcgcga aaatagggat ataattacgc agtaacggtt 11940

tacccggtat tatgtataat aaataaacgt ataaaagaca gtcgtggttt gtgtttatta 12000

taaatgtgta ttatatgtca catattataa actgtttaaa tagtaccacg tggtattatg 12060

aacagtttat aatcagttgc taccaaacaa accccattag acggcgggtt ttgataaagg 12120

gaatcgctta tttaaactaa agattttact ctataagtat ggagtgtaat ttaggaaccg 12180

aacatcatag tacagatacg tggaatcgta gtaaaacgga acaagcggtt gtggacgcat 12240

ttgatgaatc gttgtttggt gatgtagcat cggatattgg atccgaaacg tcgttatatt 12300

cacatgcagt taaaactgct ccgtctccgc cttgggtagc tagccctaaa attttatatc 12360

aacagttaat acgggatctt gatttttcag aagggccgcg tttactatca tgtcttgaaa 12420

cctggaacga ggatttattc tcatgttttc ctattaatga ggacctatat tccgatatga 12480

tggttttatc cccggatcca gatgacgtta tctcaaccgt ttcaaccaaa gaccatgttg 12540

aaatgtttaa tttaacaacc cggggttccg ttcgattgcc tagtccacca aagcaaccga 12600

cggggcttcc agcttacgtt caggaggtcc aggattcgtt taccgtagaa ctacgcgccc 12660

gggaagaagc atacacaaaa ctactagtta cttattgtaa atcgattata cgttatctcc 12720

aaggaacggc gaaaaggacg acaataggtc ttaatataca aaaccctgac cagaaagctt 12780

acacgcaact caggcaaagt attctactta gatattatcg tgaggtggca agtttggcgc 12840

gtcttctgta cctacattta tatttaaccg taacgcgtga attttcctgg cgtttgtacg 12900

ccagtcaatc tgcacacccg gacgtgtttg cggctttaaa attcacctgg accgaacgtc 12960

gacagttcac gtgtgcgttt catcctgtat tatgcaacca cggcattgtg ttattagaag 13020

ggaaaccact aacagcgtct gccttgaggg aaataaatta ccgccgccga gaactgggac 13080

tgcctctagt tagatgtggt cttgttgaag aaaacaaatc tccgttggtt caacaaccct 13140

cattttcggt tcatttacca cggtcggtgg ggtttcttac ccaccacatt aagcgtaagt 13200

tagacgcata tgcggtcaaa catcctcaag aaccgagaca tgtacgagcg gatcatcctt 13260

acgcaaaagt tgttgaaaat agaaactacg gtagtagcat cgaagctatg attttagcac 13320

ctccgtcccc atccgagatc ctgccggggg acccaccacg cccacccacg tgtgggtttt 13380

taacgcgtta aacgtcattg gggtaaaggg tgtaaataaa ttacgaaaac gtgcatgcgt 13440

tttttatttt tacaatgcgc cgtatatggt atgtctgtca tgtgctctaa agtcccatat 13500

ataaaagaag ccccaacgag tgtatgcgta ttgcgtaccg cgaccctggg atgttttaca 13560

ggcgcgtttg tttgtctcgg ttataagtat gcagtcgggt cattataacc ggaggcaatc 13620

ccgccgacag cggatatcgt ctaataccac agactccccc cgtcacacac acggaacacg 13680

ttatcggtca accaattggt atacacaccc accccagata ttgtccaatt cagaaacatt 13740

agttgcggtt caagaactac tgaactccga gatggatcag gacagcagtt ctgacgcatc 13800

ggatgatttt ccgggatacg ccttacatca ttctacatat aatggatccg aacaaaatac 13860

atcaacttcc agacatgaaa atcgcatatt taaattaacg gagagggaag ctaatgagga 13920

aatcaacatc aatacggacg cgatcgacga cgagggagag gcggaggagg gagaggcgga 13980

ggaggacgcg atcgacgacg agggagaggc ggaggaggga gaggcggagg aggacgcgat 14040

cgacgacgag ggagaggcgg aggagggaga ggcggaggag gacgcgatcg acgacgaggg 14100

agaggcggag gagggagagg cggaggagga cgcgatcgac gacgagggag aggcggagga 14160

gggagaggcg gaggaggacg cgatcgacga cgagggagag gcggaggagg attatttttc 14220

tgtaagtcaa gtttgcagtc gagacgcgga tgaggtttat tttacgttag acccggaaat 14280

aagttacagt accgatcttc gcattgcaaa ggttatggag cctgcggtat caaaggaact 14340

taatgtatca aaacgttgcg ttgaacctgt taccctaaca ggctctatgt tagcgcataa 14400

tgggtttgat gagtcctggt ttgctatgcg cgaatgtacc cgtcgcgaat atattacggt 14460

ccaaggatta tacgacccaa ttcatttacg gtatcagttt gatacttccc ggatgacacc 14520

cccacagatt ttgagaacta taccagccct tcctaacatg acacttggtg aacttttatt 14580

gatttttcct attgaattta tggcccagcc aatttctata gaacgtattt tagttgaaga 14640

tgtattttta gataggcggg cttccagtaa aacacataaa tacggcccgc gttggaattc 14700

cgtctacgca cttccatata atgcgggtaa aatgtatgta caacacattc ctgggtttta 14760

tgacgtgtcc ttacgtgctg tgggccaagg aacggccatt tggcatcaca tgatattatc 14820

cacagcagca tgcgctattt ctaatcgcat ttcacatgga gatggattag gatttttgtt 14880

agacgcggca attcgtatta gcgcaaactg tatttttttg ggacgtaacg ataattttgg 14940

cgtgggggat ccatgttggt tagaagacca tcttgccgga ttaccacgag aagccgtacc 15000

cgacgtactc caagtgacac agttggtttt gccaaatcgg ggtccaacgg ttgccattat 15060

gcgtggtttt tttggggcgt tggcatattg gcccgaacta agaattgcta taagtgaacc 15120

atctacatct ttggtgcgat atgctaccgg tcacatggaa cttgccgaat ggtttttatt 15180

ttcacgtaca catagtttaa agccacaatt taccccaacg gaacgggaaa tgttagcgtc 15240

attttttacg ttgtatgtta ctcttggtgg aggaatgttg aactggatct gtagagcaac 15300

tgcaatgtat ttagctgctc cttaccattc ccgttcggct tacatcgcgg tctgtgaatc 15360

tctgccctat tactatatcc cggttaatag tgacctgtta tgtgatttag aggtattact 15420

gttaggcgag gtcgacctcc caactgtttg tgaatcctac gcaactattg cacacgaatt 15480

aaccggatat gaggctgttc gcacagcagc cacaaatttt atgatagagt ttgccgattg 15540

ttataaggaa agtgagaccg atttaatggt aagcgcgtac ctgggggccg ttttattgtt 15600

acaacgggtg ttgggtcatg caaatcttct tttgttgctt ctctccggtg ctgcgttgta 15660

cggaggatgt tcaatttaca tcccccgagg tattttagat gcatataata ctttaatgtt 15720

ggcagcaagt cctctttacg ctcaccaaac tttaacatcc ttttggaaag accgcgatga 15780

tgcaatgcaa actttgggga ttcgaccgac aacggacgtt ttacccaaag agcaagacag 15840

gatagttcag gcatcaccta tagagatgaa cttccgtttt gtgggattgg agaccatcta 15900

tccccgagaa cagcccattc cctccgtgga cctagccgaa aatcttatgc aatacaggaa 15960

tgaaattctg ggtttggatt ggaaaagcgt agccatgcat ttactacgaa aatattaagg 16020

gttgtgattt ttttcattag gatgaaaaga acgtttccta gccacaccca caaaggagtt 16080

tgtaaaataa aatctctgtt tagaccttaa aatttgttgt gtgtgttgtg tggggggtcc 16140

gtgaggatcg acctttacaa gatataattt gtccatatcg caatgttttc tcggtttgcg 16200

cgttcctttt ccagcgatga tagaacgcgt aaatcttatg atggtagtta ccaaagtttt 16260

aatgccggcg aacgtgattt gcccacacct acccgggact ggtgttctat ttcccaacgc 16320

ataaccagcg agcgcgtgag ggatggatgt cttattccaa cgcccggcga ggctttggag 16380

acggcggtaa aggctttatc tgaaaagacc gacagcctaa catcgccggt tttacaaagt 16440

accgaaagac acagtgttct gcttggatta caccataata atgttcctga atcgttggtg 16500

gtctcgtgta tgtctaacga tgttcatgac gggtttatgc agcgttatat ggaaacaatt 16560

caaagatgtt tggatgacct gaaactttct ggggatggac tttggtgggt ttatgaaaat 16620

acatattggc agtatctcaa atacaccaca ggagccgagg taccggtgac ttcagagaag 16680

gtaaataaaa agtctaaatc cacggttttg ttgttttcat ccgtagttgc caataaacca 16740

atatccagac atccttttaa atctaaagtt ataaattcgg attaccgggg aatatgtcag 16800

gagctacgtg aggcgttagg agctgtgcaa aagtatatgt attttatgcg tccagatgat 16860

cctacaaacc ccagcccgga tacaagaata cgtgtacaag aaattgcggc ttacacggct 16920

actggctacg ggtggatgtt atggttcttg gacgttgtgg acgccagggt atgtcgccat 16980

ctcaaacttc aatttcgacg gattcgaggg ccgcgcgcgt ctgttattcc agatgatttg 17040

cttagacgac atttaaaaac gggtcctgcg gtctcagcgg gcacaggagt tgcgtttatt 17100

ttagcagcaa caactgccag cgctcttact gcgcttttgc gtattagtgt attatggcga 17160

aaggaagagt ggcgggatgg tttaaatgga accgcagctg caattgttgc ggcggttgaa 17220

cttattacgc ttttgcacca ccattttcaa tacttaatta atatgatgct tattggatat 17280

gcatgttggg gggatggggg attaaacgat ccttatatat taaaggcgct acgtgcccag 17340

ggacggtttt tatattttgc gggtcagttg gttagaacaa tgtcaacaca cagttgggtt 17400

gtgttagaga ccagcaccca tatgtggttt tcccggccgg tggcgcagag tattttagca 17460

catgggggta aacccacaaa gtattatgct caggttcttg ccgccagtaa acggtatact 17520

ccgttacatt taagacgtat atccgaacca tcgagtgtgt ctgatcagcc gtatattcgt 17580

tttaatcgac tgggatctcc aatagggaca ggtataggga atttggaatg tgtctgttta 17640

acgggaaatt atttatctga cgacgtaaat gcaagttcgc atgtaattaa tacagaagca 17700

ccgttaaaca gtatagcacc cgatacaaat agacagcgga cttctcgcgt tttagttcgt 17760

ccagacacgg gtttggatgt aactgtccga aaaaaccact gtttggacat aggccatacg 17820

gacggtagtc cagttgaccc aacgtatcct gatcattaca cccggataaa ggcggaatat 17880

gaaggtccgg ttcgggatga atcaaacaca atgtttgacc aaagatcgga tttacgtcac 17940

atagaaaccc aagcatcttt aaatgatcac gtatatgaaa atataccacc caaggaagtg 18000

ggttttaact catcttcaga cctggatgtg gatagcctta acgggtacac ttccggagac 18060

atgcatacag acgatgactt atcaccagat tttataccca acgacgttcc cgttagatgt 18120

aaaaccacgg ttacgtttag gaaaaatacg cctaagagtc atcattaagt acagcggtta 18180

atagatagtt atggactagg cactttggcg gtcatttcca caaccaggtt aaaattgggg 18240

gatttgggag aaaatagtct attgcgtatt ttctgttcaa taattggact gcgttattta 18300

aaggtctgat tggttgattg ggttataaaa ggaattactc ctttaaattt tacttaatgt 18360

acccacaata tcaagtggtc gtttgtattt aacgattatt accggtacca tgggagactt 18420

gtcatgttgg acaaaagtgc cgggttttac gttaaccggc gaacttcagt acttaaaaca 18480

agtggatgat attttaaggt atggagttcg gaaacgcgat cgaacaggaa tcggaacgtt 18540

atctttattt ggaatgcaag ctcgatacaa tttgcgaaat gaatttcctc ttttaactac 18600

aaagcgtgtt ttttggaggg ccgtcgtgga agagttgtta tggtttatcc gcgggtcaac 18660

cgattccaaa gaactcgccg ctaaagatat acacatatgg gatatatacg gatcgagcaa 18720

atttctaaat aggaatggct tccataaaag acacacgggg gaccttggcc ccatttacgg 18780

cttccagtgg agacattttg gagcggaata taaagactgt caatcaaact atttacagca 18840

aggaatcgat cagctgcaaa ctgttataga tacaattaaa acaaacccag aaagccgacg 18900

aatgattata tcgtcttgga atccaaagga tatcccctta atggtactac ctccatgtca 18960

cacgttatgt cagttttacg ttgcaaacgg tgaattatcc tgccaagtat accagagatc 19020

gggggatatg ggccttgggg taccgttcaa cattgctgga tatgcacttc ttacctacat 19080

agtagcgcat gttacaggac ttaaaaccgg agatttaatt catacaatgg gggatgcaca 19140

tatttacttg aatcatatag atgctttaaa agtgcagcta gctcgatccc caaaaccttt 19200

tccttgcctt aaaattattc gaaatgtaac agatataaac gactttaaat gggacgattt 19260

tcagcttgat ggatataatc cacacccccc cctaaaaatg gaaatggctc tttaatggat 19320

ttttaaatgt tgtcaagaca gtagatgtgt tgcgaatgta ataaaatgat atacacagac 19380

gcgtttggtt ggtttctgtt tatgaacagc aacggatgca tagggttgcg ataactgcga 19440

taagacccaa tgtcccaagg atagatatca caccaattat aactgctaca acggaaaatg 19500

tagtggcgta ggtagatgca tcgtaggtat aaacggccga aaacggaggg aattttttag 19560

ggtaaccatc tagatgacac gaataggtga taggtccgtc gagttccgat gttggacaag 19620

aactttgcat gtttacaaac cgtttgtttt gatcacacac cccagtaatc tcactgtttt 19680

cgtggttgat gggagaatcg ttaacccacc atacgaaatg tacaacgcca cgtggcacac 19740

attttgccgt acatactatg tgtccatcaa taatacctat agacacgttg ggaaatggat 19800

agacgtcagg ggtaacgaca gcagaatatt tcatattaga gacgccatcc cgaatccata 19860

aaacattaca ttggatggct gggggtgggt aatccatttg tttttgctgt ggaattcgta 19920

ccgccgaaac ataactaaat aatccattgg catattcttg tattgcatcg gttataaaat 19980

tttttccgat gttaccaaac cttgaagtcc accgaacacg taccgagtgc ggtggataat 20040

actttgatac gttacagtag gctgcgtatg tctgtccggt taagactgga tcgccgacaa 20100

cggtaatatt tggacgataa tacgttgtaa ctgtaatact gtgttccgat atgacgttct 20160

tagtttttgt attaacgact cgccaaatat acgttccctc cgtggtagca tccatagata 20220

aaattgttac agaaaaatca gacgttgttt taacatctgg tattacataa ttttccttag 20280

cgtgtgtaaa tatctcaggg ttgtttatta agtttaaatc ggcactgttg ctatataaca 20340

taaccggtaa atctggcatg cgtattaacg cattgcccag ttgacggtgc ggatctataa 20400

ggtgacgcgt aaaccaaact tcaatatgaa gatcggggcg tataagcgac ttccaccttg 20460

ttatatttga accttccgga tctaaagaat attgttcata tgttttttgt tgctgcttaa 20520

aggccgcctg ttgtccggtc gttagacgca tgtaacaagg catgataaat gtgtgaaaat 20580

agggtatgga ttgtattccg ccgtgaacgc attgtatatt ttcaaataga aaaggtggtt 20640

gtgaatgttg ggcgttggct gcgggatcgg gctttcgggt agcggccgag gtgggcgcga 20700

cggcgggatc gggatttcgg gaagcggccg aggtgggcgc gacggcggga tcgggatttc 20760

gggaagcggc cgaggtgggc gcgacggcgg gatcgggatt tcgggaagcg gccgaggtgg 20820

gcgcgacggc gggatcgggc tttcgggtag cggccgaggt gggcgcgacg gcgggatcgg 20880

gatttcggga agcggccgag gtgggcgcga cggcgggatc gggctttcgg gaagcggccg 20940

aggtgggcgc gacggcggga tcgggctttc gggaagcggc cgaggtatat aattcagtta 21000

tacttacggg tgtgggttga gattcagtcg ataattgtat acacgcgatc gttaaaatta 21060

aatttatttg tatccgcttc atcctggttt ttattgacac atccacgctc cccttaaata 21120

aaagattaaa acacccaccg cggaatttaa atgatggaaa cgtttttttc gacattggga 21180

ataataaaaa cggcttttgc aactttaaaa actttattta tctcgattac gatacatatg 21240

taccacatag atagcataga tttattataa tataaacaca cacgtgatat actttagtga 21300

tatgagatgc cataaaacag tcaataggtt taacgcttaa tctcatcatc tgaatacacg 21360

tcaaacccgc cgcaactgtt gatgttagaa ttataatagc tccccatgaa atgccggcaa 21420

atgttacagc tatacccgtc accgaggtcg ttgtatataa tacaattacc cataggtttt 21480

tttttcttga tataaaacgg caaaaccctg taacccaaat gctataatat gacctcctat 21540

tgaaactgct aacgttactt gtgtaagttt gataaaatga tttaatttaa ttatatgtga 21600

gattgcccac attaatgggg taactatata taacaccggg ggtataacag acattatacg 21660

aattccttta aacacgcgtt taagggtccg ggaactttct ctatggtcac atactctccc 21720

gcggtcattt tgtgtatata caacggcaaa acctaaatct gtataagtgt ttaattgctt 21780

atggcgattt ttacgatata tacacgtatc ttgcaaatcg gtggcggcat cgacaattga 21840

aactagtgtg acaatagata tacacaatcc aataagaacc tcatatttac tgacatacat 21900

atataaaata acggttagta aacctcccaa cccagttccc aacatcataa cataaaaata 21960

aatatgcggt ccattgaatg tcgtaacaaa gttgtagtaa tggatatgca cagcagccac 22020

tgttccggta atcgcggata tggaaattcc cagtaattct acaaatggaa gatcccggga 22080

tattgggcaa ccaaccgccc ataacacagc aaaacccaac acgaccaccg tctgcaaaca 22140

tcgtcccaat tttgctaatg tgcgtagaaa tttcacggat gttggccata accccgaaac 22200

gacgatcaac cccataatag ttgcattgac ggcagcttcg cagacgtgat attgtaaaat 22260

taacccggac gtgataacac ttgcttgtag tcccacgaga aacaaccgcg atgctgaggt 22320

tattgcacac gaattacatt cttgagggtt tccgacacat ccttggattg attgagcgcg 22380

gattaattct ctgtctaaca cacccaggtt ttcatcatgg acagctcttt caccattcac 22440

ggccatgtct taagtttaat aattcaaaac agataaaaat gtgttcatct atggtacaca 22500

caagtttgta tgtaaaatat aagcaaaagt tgcacttatt taactgtaca tattacgtca 22560

gattcacgtg ataattcaga ataatccagg gttcctgcag ggtccactgg aggagccaca 22620

caatattcgc gaattccgat tccctcctgc catgtggttt cggggagttt cccccccatt 22680

ttatttccgg tatttttttc gtttcttttt gttaataaat tgcgtctttt ttttaatggt 22740

ggttcatcct tcacagattc cgtgttcgca aataattgca tcgaggttaa tttttcttta 22800

aggtctttgg gacttaagaa cgttgcataa aaaaaagaat gcacgggtgc ggaacgttgg 22860

atatacaatc caaccatggg ggagttagtt aaggcgagat aaaaattaat ataacacgtc 22920

tcatcccgtg ttaacttaag attttgtacg gcagaacgga atccactgtg tgtttccaat 22980

aatactccaa attcacgcat actcccgctg ccataaacaa cattattaag gatccttttt 23040

gaatttgtga ttgagcgtat taaattatat ggtgtaggct tgcttccgtt tatatccaag 23100

gaaacattaa atgagataaa accacccccg gcggtctgga tgtacatatc cgtggctgtt 23160

agaatgaagc atgttgtaaa cccaaaagtt ttaagtagtc gctgtaaacg ggtgaattga 23220

tcgcgtttta agcaaatgct tatatctgga gttagatttg ggaacatcat tgtataacaa 23280

gcgagttcac gttttacaac ttgtttgtaa cattgtactt gatcatctgg accacaatca 23340

cccgggcgtt gccataccat cgtttggata atactccgct cggggggttg tccggtaaat 23400

ttaaaatata accgtgttgg ggtcgacgga tcttttgtat ggcgaaacgc gtcaataagc 23460

gaggaccgtc cctccgttgc cgcgagtaca accattctcg gcccagtcca attatactgg 23520

tcaaacatat ttgccggtat aggaatatac agttgttctg tttccaaact acagtgaata 23580

attaatcctt cgtcgctgaa tattaaaata gaatccctta gtctattaac cagaggtgat 23640

atagacgaaa ttaaaccagt aagcgttttt tccgttaaaa cagctctggc gatttctggg 23700

gcgtcaaaac ccgcatgcaa ttccatgtcc aaagcatcgt ctgtacgcga cctcaaatcc 23760

ataatttact acttaaaatg tttactatag aaaaagtaat catatgtaaa cacacgagtt 23820

tcgttaatat gtttgtttaa cccgatccgg tgacttaagt acataaacag gcatgatatt 23880

tgaatagtac ggcccatggg agggaacatt tccacgtgtt ccaatacagg gggtgttcct 23940

taatagggac tgtgcaataa aatacgtaag aagttaccag atttgatgta atgtttgtca 24000

taaaaaatat gtacatcatt atatacgtct gtaattaaca caagatcaca tcgaagaatt 24060

actgaagccg ctgtgaaacc tttcacaaga cgatataaac ttggttaagt gtattgatgg 24120

ggctctttgg actgacacgc tttatccatg aacataaact ggttaaaccc agcatcattt 24180

caacgccacc cggagtttta acccccgtgg cggtagacgt atggaacgtc atgtacacat 24240

tgttggaacg tttataccct gtgggtaaac gcgagaattt acacggacca tctgtaacga 24300

tacattgtct tggagtctta ttgcggctat taacacaacg gtcatactat ccgatatttg 24360

tattggaacg ttgtacagac ggcccattat cacgtggagc caaggcaatt atgtcacggg 24420

ccatgaacca cgatgaaagg ggaacctcgg acttaacccg tgttctacta tcatccaaca 24480

catgttctat caagtataac aaaacatcgg aaacatatga cagtgtgttt cgaaactctt 24540

ccgcgagttg tattcctagc gaagaaaaca aatcccagga tatgtttttg gacggttgtc 24600

cacgacaaac tgacaagatg atctgcctgc gcgaccaaaa cgtatgcagt cttacctcta 24660

caatgccatc ccgaggacat cctaaccatc gattatatca caaattgtgt gcaagtctta 24720

ttagatggat ggggtatgca tacgtcgagg cggttgacat tgaggcggac gaggcatgtg 24780

caaacttatt tcatacgcgt acagtggctt tggtttatac gacagatact gatttactct 24840

tcatgggctg tgatattttg ttagatgcaa ttcctatgtt tgctccagta gtacgatgtc 24900

gcgatttgct tcaatattta ggaattacat accctgaatt tttggttgcc tttgttcgct 24960

gtcagaccga tttgcataca agtgacaacc taaaatctgt tcagcaagtt attcaggata 25020

ccggcctgaa aattccacat caaatggaca cttcaacgcg ctcccccact tacgactcgt 25080

ggagacatgg cgaggttttc aaaagtctta ccgtagccac gtcgggtaaa acagaaaacg 25140

gagtgtccgt ttccaaatat gcatctaacc gatcggaggt gacagtagac gccagttggg 25200

ctttaaacct tctgccaccc tcatcctccc cattggataa tttggaacgc gcatttgttg 25260

aacatataat cgccgtggta actccattga cccgcggtcg cctaaagtta atgaaacgtg 25320

taaatattat gcaaaatacg gcagacccat atatggttat taacacctta tatcataact 25380

taaaggggga aaaaatggct cgccaatacg cacgtatttt taaacagttt attcctactc 25440

cactcccact aaacactgta ttaacaaaat attggaatta aaacacacat aagagcgact 25500

taatggttca ttgttttatt ttgctcgtat atacatgtta taaatcgttt atcactgtgc 25560

ccgcataaga tgtactgtgt ctctcaaaaa aatttgtgtt tttatctgca atcataaatg 25620

caagtggaaa gtccgaatcg ggaggtgggg tgttaaatag ttttggtaca ttaatcgctg 25680

ataaaagcct gtccgcgctg aatttcacgt attgtgtaat tgcatcgacg ttcaccaaac 25740

gggttttggg tgcatgggat tttaaaaacg cacactcgat ttcaacggct tccgaaaaca 25800

gttgatgtat tctggtgata gcgggttttt cgggtacata gttattgtat atacaacacg 25860

atgcgctggt atgtatggct tcatctcggc ttataaggtc gttaaattga caagttacaa 25920

caaatagtcc gttattgcgt aaatatgcaa tagccgcgaa cgatgataca aaaaaaatgc 25980

cctctataag aatcattagt atatattttt ctgcaacgga tgggttgtcc cgtacctttt 26040

cttccaacca ttgtactttt tgttggatcg acggattatt aatagtgacg tttacgtatt 26100

gtacccgcaa cgattcatcc cctctgaaca acattagttg aatttgacta tagacacgcg 26160

cgtggacaac ctcgatgcac tcttgttcaa tgtagtaatg gtgaatatcc ttttgggaaa 26220

agagttgggt tagagagccc aaattaacat ttaccagatc atctgccgcc gataaaaatg 26280

taaaaataaa tctgtagaat attagttcat cttccgttaa acagtccaag tattgataat 26340

catcttcaat gataaaatcg ctttctaacc aacgattcga aatgctcagg gcacgtaaat 26400

tgtttatatc tggacactcc ggcctgtaaa aaaaatgact gcaatctttc tgatccattt 26460

tggaatagtt tcccgtgtaa atttataaag cacaactggt acaggttaat tcgcctcccg 26520

caaacagtcc gctgttcgta gctttacgaa ttttacagta gtacataccc gttttaaggc 26580

cggctttata ggcacgtata agcaaattca ttattttgga ggcgggaatt gtcccgtctg 26640

ggcgttcctc aataaataaa gtcattgatt gactttggtc aataaatggc gccctttctg 26700

cacacatatc aacgagatcc tcttgctcat attcaaacgc tgttttatat tttaagagtg 26760

ggtgactatt agataaacag ccaaacgaac gtattactga ccattggttt ttctcaagta 26820

tgtttataac ttccagtcgt ttttcttcac atgaatacat atctcttagt tcgtccataa 26880

ggtctaagtt gggtctaagt aactcacccg aggtggtgac cttactaaac atattattat 26940

aaattggaga gaaaccctca ctgcactccg ttacctgtgc agatgaaact gtgggcatta 27000

acgctaagaa ctgcgagttg tataacccat aagcgcaaat atcatctcgc agggtacacc 27060

atggtaaatc taaataactt atcgtagaaa acccatcttg gtgtaaccat cccttagcat 27120

atttactttc ggtaaaaccc ttaaacgggg ctaagccgcc aatcttacac atttccatgc 27180

ttgttttcat tgtctcatac aacattaact ccgctatttg tacatttaac cgtctagctg 27240

gttgggaagt taaatcaaat cctaagcgga gacaagttgt atgtaaccct tgtatgccaa 27300

tgccaagtga tcggttgttt tttacacctt tacatgattt tttacatgga aagttcccag 27360

ccgccaggac cccgtttaaa aaaataacag tcgttcttgc tgtcaattga aggtcgttta 27420

aattaaatga cactgggcct ttggataagc acgttgtaag atttatgctg gcaagattac 27480

atacgccgtg ttgatgagcg tctgcctttt gaacaatttc cgtacacaaa tttgaccccg 27540

tgatagcatt tccttgggta ttcatatgat aattacgatt acaggcatct ttgaacatta 27600

aaaaggggct tcctgttaca gcagcactgc gtatgattgt gaatgcgata tcttgaatgg 27660

gaacagaaga aacgcctaat ccttctctct ctaaacgtaa ataggttgaa gtgaatgcct 27720

ccccgtgtaa tgttcgaagg atatcggctc tgttatcaaa aagagtccac tgaacattac 27780

tagccccttt tagatagctt aggtatcttt caaaaaataa atctggggtc cataaacaac 27840

aaaatatgtt atcacatcga aatatttcat cacgaaccaa cattccacgt gtggccaaaa 27900

cagtttgtag atcgacgtgc catggttcta tgtaaacaca aactccagtt ggtcgttcac 27960

aatcactgtt aattgccata accatgcaat ctaaaagttt taaaactgca agaagacctt 28020

tcgtttgatt ttccgtaggt attaaattca gactctgtag agaaattccc actccacctc 28080

gactttgtaa taccgttccc acatcgcctg tgatagctcg aacagctctc ccaacagtga 28140

tggattccgg gtccattaaa taacaactgg ccgttgcccc ggtctctcga cctaaaaaca 28200

tcataaccgg tgtagccggg acaatttctt gacatgccaa cgctgtgaaa aatacccgac 28260

agacatcagt ccatgtataa ccatcattta ttccgggaat aagagttgcg attttaggca 28320

ggtttacgat ttctgttgtc acggtggccg ccagtcttaa aaagaattgg caaagcgact 28380

ctaatttacc ttcctctaac ttagttaaat aaaagtcttc gtactttaaa gcagactgta 28440

gtccaagggt agctaaagcg gggtattgat ctttcaaaaa cggttctaat atagcccgac 28500

gaatttcgtc cctccgccct tcaattgctt ggcggactcg gggagttaaa cagagaattg 28560

gggaagtcaa ccacgtttcc atggaaacgg atcgtaggtt aatacggcaa tggataagtt 28620

ctccacaaca tcggtacact cgctcatctt gtcgcgtcac cgccttaagt tttgagacga 28680

tagtgctaat atactccatt aattccaccg gtgtggttga ttcgggcgga atgatgtatt 28740

ccttgtagcc atgttgacat aatcggttta taatgtcatg aaccgtatta aaaattcttt 28800

tgaactccat aacggataac gtatttaggc tccggaataa acctttaaac cctaaactca 28860

cagctgagtt agttctacaa tattgtagac tcccttatat atggttacgt acagcctgcc 28920

cctccccagt atataatatc acgcaaaacc cacgctatgt taaattcagt ttattttaca 28980

tacatgcttt aataataaca ttcgttccat gtatttgtac ccccccacac aaccccctct 29040

aaccaaatag ttggcacgtt ataacctccg aaccgttcca tgcgtcttgt ataacgcaca 29100

gactctgatg gaattgttcc aattaacgta tatgccgcat acatgcagga taattgtgtg 29160

ggaaggcccc gaaaatcgcc ggtccattga tacaatcgct gtctagccaa gttccaattt 29220

actcctgtaa tttcgccaat actacatcga gggcttgtcg ggtcattgga taactgcaca 29280

agcggcaacg cccttgtgtt atatggctgg tgggtatttg caaccccttc agtcccccag 29340

gcggcatttt cagctcgtat gcgtcctaac aggaagccaa taccacgacc aaaacattgt 29400

tcgtttagtt ggcttaatgc aagatgcagt cttacacctt ctcgttggcg tcgctgtgta 29460

tatacaaaaa ccaagaacac atgcttcagt ccgtccgcgg aaagatgtaa atctttgtca 29520

acgtcccaaa atacgcaggc cgggatgttg gctgtgaccc tgcgagttga agttttgtct 29580

gtacgtgcag cttcttgggg acctttggcc acggcggtta tattgcataa attatcctga 29640

atggtatatt ccagcaggga cccaaaaaaa cttataaatc gatgtggaaa tacatgacat 29700

tgtaccatcg cacgtaaaca ctccgaaaac cttatgagcc gcgtttccat acgactgcat 29760

ccataggcag aaacaattgc tgttctgttg gcatccgctg cctgtttatc cgtatattct 29820

tctgcccggc atgcggcgat gaaacttaat gacgttacat atgctctaag ccccccacct 29880

tctccaacgg tccaaggagc cgtgcaggca ttgaataggt ttcgtaaacc ctctagtagt 29940

acatcggggt cacgtccagc ctgtgtaagt gtattagctt ctccaatcat gtcagatgga 30000

tgacgaagga ttaagacgat tgacccagca tgctcaatgt ccggacgaaa aaaatcggtt 30060

aatgacactt gttggattag ctgtgtcgtt gatttaaaat tatttaacgg gagtctaatg 30120

gtaacttgcg ggttaccaat tgaagttgga tttatttgaa tgttgttcat acgattaata 30180

acaattgaac ggggggttac ttgaatagac gcggtttctg tacgttttgg tggtacatgt 30240

atcggttgtt tgttcagacc tccaaagcga gggccaattg ttaaatcgcg actccaattt 30300

ccgaagaagc ccggagcata agtcatatga agcccgttcc ctatttgaat aaaacggtta 30360

tttcctaaaa gactgatatt agttccacat agcgtttgtt cgtttaaagt aaaatgcgag 30420

ttggttggtt gactccccat agctgagggg ttaaattcac acaatgcaat cgtgacgtgg 30480

tactatctga aatgttgcct ggggtatgtg tacacattat acagtcgtag taccgtttat 30540

ataatgttag gtaggaggag cctataaaaa tattttgatt ggcgttaaaa ggttcttcaa 30600

cttaccgtga cgtccttttt attaacatgc gtttttattg atgttacatt tatgtctttt 30660

cattccggac ggatgtagct ttttcatatc acgttataaa gttaagtcag cgtagaatat 30720

accatggaag aaccaatttg ttatgataca caaaaacttt tggatgattt aagtaacttg 30780

aaagtacaag aagcggacaa cgaaagacca tggtcaccag agaaaacaga aatcgccaga 30840

gttaaggtag ttaagttttt acgatctacc cagaaaattc cagctaaaca ttttattcag 30900

atatgggaac ccctgcattc taatatctgt tttgtatatt ccaatacatt tttggcggag 30960

gctgctttca cggccgaaaa tttacccgga ctgttgtttt ggagactaga tctagactgg 31020

acgatagagg agccaggtaa tagcttaaaa attttaaccc agctatcaag tgtagtacaa 31080

gattccgaga cgttacatcg tttatcggcc aataaattac gaacctcgtc taaatttgga 31140

cccgtttcga tacacttcat tataacggac tggataaata tgtacgaggt cgccttaaag 31200

gatgcaacaa cagccattga atcaccattc actcacgctc gtattggaat gttggaaagc 31260

gccattgcag ctttaacaca acataaattt gcgatcattt acgatatgcc atttgttcaa 31320

gaggggattc gtgttttaac acaatatgca ggatggcttc ttccgtttaa tgttatgtgg 31380

aatcagattc aaaatagctc actcactcct ctaacacgag ccctttttat aatctgtatg 31440

attgatgaat atctcacgga aacgccagta catagcatat cagaattatt tgcagatact 31500

gtaaatttaa ttaaagatga ggcgttcgta tccatcgaag aagcggtaac gaatccacga 31560

acggtgcacg agtcacgaat ttcctcagct ctggcttatc gagaccctta tgtttttgag 31620

acatccccgg gaatgcttgc taggagactt agattagaca atggtatatg ggaaagcaac 31680

ctcttatcgt tgtccacccc cggaattcat attgaggcgc tgttacattt actaaactcc 31740

gacccggaag cggaaaccac atctggaagt aatgtagcag aacacacccg tggcatttgg 31800

gaaaaggttc aggctagtac atcgcctagt atgttaataa gcacccttgc cgaatccggg 31860

tttacaagat tttcatgcaa attgctacgt cggtttattg ctcaccacac actcgccggt 31920

tttattcacg gaagcgttgt agcagacgag catattacag atttccaaca aacactagga 31980

tgtctcgctt tagtgggtgg actggcatac caattagtgg aaacgtacgc tcctactacc 32040

gagtatgtgt taacatatac acggacagta aacgagaccg aaaaacggta tgaaacgcta 32100

ttacccgcct taggattacc accgggaggc ctgggacaaa ttatgcggcg ctgttttgct 32160

ccacgacccc ttattgaaag tatacaagcg acacgcgtaa tactacttaa tgaaatttca 32220

catgcagaag ctagagaggc aacatatttt aagcaaacac ataatcaatc ctcaggtgcg 32280

ttattaccac aagcaggaca aagtgccgta cgcgaagccg tactaacctg gtttgaccta 32340

cgtatggatt caagatgggg tattactccc ccggtggatg tgggtatgac acctcctatt 32400

tgtgttgatc caccggctac agggttggaa gctgtcatga taacagaagc actaaagatt 32460

gcatatccta ccgaatataa tcgctctagc gtgtttgtgg aaccgtcgtt tgtgccttat 32520

attattgcaa caagcacgct tgatgccctt tcggcaacaa tagctttgtc ttttgataca 32580

cggggaatac agcaagcctt gtctattctt cagtgggctc gcgattatgg atccggaacc 32640

gtgcccaatg cagatggata tcgcacaaaa ctatctgctc ttataacaat attagaacct 32700

tttacccgta cacacccccc agtactttta ccatctcacg tttctactat agattccctt 32760

atatgcgaac ttcatcggac tgttggcatt gccgttgacc tgcttcccca gcacgtccgt 32820

cctttggttc ctgaccgtcc ttctattaca aatagcgttt ttttagcaac tctctattat 32880

gatgaacttt acggtcgttg gacccgactg gataaaacat cgcaggcgtt ggttgaaaat 32940

tttacatcca acgcgttagt ggtttctcgg tacatgttaa tgttacaaaa attttttgcg 33000

tgtcgttttt atccaacgcc agatcttcag gctgttggta tctgtaaccc aaaggttgaa 33060

cgcgatgaac aatttggggt atggcgttta aacgatcttg ctgatgcggt tggtcatatt 33120

gttgggacaa tacaaggaat ccgaacgcaa atgagagtgg gaatatccag cctgcgcaca 33180

attatggccg atgcttcctc agcccttagg gaatgtgaaa atttaatgac taaaacctcc 33240

acttctgcta ttgggcctct tttttcaacg atggcttccc ggtatgcacg gtttacacag 33300

gatcaaatgg acattttaat gcgtgttgac aaactaacaa caggagaaaa tatacccggt 33360

cttgcaaatg tagagatttt tttaaatagg tgggaacgaa tagcaacagc ttgtaggcat 33420

gccacggcag tcccgtcggc cgaatctatt gcaaccgtgt gtaatgaatt gaggcgcggt 33480

ttaaaaaata tacaagagga tcgtgtaaat gccccaacct catatatgag tcacgcccga 33540

aatctggaag atcacaaggc agcagtttca ttcgttatgg actccaggca acagtttatt 33600

gtggattctg gacctcagat gggcgcggtt ttaacttcac aatgtaatat aggaacatgg 33660

gagaatgtaa atgcaacgtt tttacacgac aacgttaaaa taacgacaac ggtcagagac 33720

gtaatttcag aggctccgac gctgataata ggacaaagat ggcttcgtcc agatgagatt 33780

ttatctaatg tagatttgcg tcttggcgta cccgggaata caagtgggag tgacccttaa 33840

tataaaacag gcgtgtttat gtacattaaa gtatttgtgg tttttattga ctgggcgttt 33900

cgtttgtata acgctgttgt tgctagtatt ttcataacct cctaggtttt tggagctaca 33960

cgtgcttatt caacgctctt tgggatttga atcatcgtaa acgtagcgtc cctaccagtt 34020

gagcgcgtaa ttttcgtaag caataaaatg gatataattc cgcctatagc tgtcactgtt 34080

gcgggagtgg gaagccgtaa tcaatttgac ggtgccctgg gaccggcgtc aggtctgtca 34140

tgtttaagaa catctttatc gtttttgcat atgacatatg cgcatggaat taatgcaacc 34200

ctgtcatcag acatgattga tggatgttta caagagggtg cagcatggac tacggatctg 34260

tctaatatgg ggaggggtgt cccagatatg tgtgctcttg ttgatctccc caatcgaatt 34320

tcatatatta aactggggga cactaccagt acgtgctgcg ttttgtctag aatatacggc 34380

gatagccatt tttttaccgt tccagacgag ggttttatgt gcacacaaat tcccgctaga 34440

gcgtttttcg atgatgtgtg gatgggacgt gaagagtcgt atacaattat aactgtagac 34500

tcaacgggaa tggccatcta tcgtcaggga aacatatctt ttatttttga tccacatggc 34560

catgggacta taggacaggc tgtagttgtt cgggtgaata ccacggatgt gtactcttat 34620

atcgcatcgg agtataccca ccgccccgat aacgtagaat cccaatgggc cgctgcatta 34680

gttttttttg tcaccgcaaa cgacggtccc gtaagcgaag aagcgctatc ttcggcagta 34740

acgcttatat acggaagctg tgatacatat tttacagatg aacaatattg cgaaaaactg 34800

gttacagctc aacatccgtt gcttctttca cctcctaatt ccacgacaat tgtgcttaat 34860

aaatcgtcta tagtacctct tcaccaaaac gttggtgaaa gtgtatcctt ggaagcaacc 34920

ctacattcaa cgttaaccaa cacggttgca ctggacccta gatgtagtta cagcgaggtt 34980

gatccttggc atgcggttct agaaacaacc tcgactgggt ctggcgtttt ggattgtcgt 35040

cgtagacgcc gtccttcatg gactcctcct tcaagcgagg aaaatttagc ttgtatcgac 35100

gatggcttgg taaataatac acattccacg gataatttac ataaacccgc taaaaaggtt 35160

ctcaaattta aaccaactgt agacgtgccg gataaaacac aagtggcaca tgtattaccc 35220

cgcctacgag aagttgctaa caccccagac gttgtgttaa atgtatccaa tgtagatacg 35280

cctgaatcca gtcccacttt ttcacggaac atgaatgtag gaagcagttt gaaagatcgg 35340

aagccatttc tatttgaaca gagtggtgat gtcaacatgg ttgtcgaaaa actactacaa 35400

catgggcatg aaattagcaa tggatacgta caaaatgcgg tgggtacgtt ggatactgtt 35460

attaccggtc atacaaatgt tcccatttgg gtaacaaggc ccttggtcat gccagacgaa 35520

aaggatccat tggagctttt tattaacctc accattttgc gtttaacggg atttgtggtg 35580

gaaaatggaa cacgtacaca tcatggtgct acaagcgttg tatcagactt tataggtccc 35640

cttggggaaa ttttaacagg atttccctcc gccgcggaac ttatacgcgt tacaagtttg 35700

atattaacaa acatgccggg ggcggaatat gctattaaaa ctgttctccg gaaaaaatgt 35760

acaattggca tgctcattat cgctaagttt ggtctagttg ccatgcgggt tcaggataca 35820

accggcgctt tacatgccga actagatgtg ttagaagcgg atctaggagg ttcgtcgccc 35880

atagacctct attctagact gtcgacaggt cttataagta tactaaattc gcctattatt 35940

tctcatcccg gactttttgc cgagcttatt ccaacccgta cagggtccct gtctgaacga 36000

atacgtcttc tttgtgaatt agtctcggcc cgggagacac gctatatgcg tgaacacacc 36060

gcgcttgttt ctagtgtaaa ggctttagag aatgcattac ggtctacccg caataaaatt 36120

gatgccattc aaataccaga agttccccag gaacccccgg aagaaaccga cattccaccc 36180

gaagagttaa ttcggcgtgt atatgagata cgatccgaag ttacaatgct attgacctcg 36240

gctgttacag aatacttcac ccgcggagtg ttatatagca cacgggcctt gatcgctgaa 36300

caatccccta ggcgttttcg ggtcgcgacc gcaagtacgg cacccattca acggctttta 36360

gattctcttc cggaattcga cgctaaatta acggcaatca tatcgtccct gtctatacac 36420

cctcctcctg agactataca aaatctcccc gtcgtatctc tgttaaaaga gcttattaaa 36480

gaaggggaag atttaaacac agacacggct ctcgtatcgt ggttatctgt agtcggggaa 36540

gctcaaaccg caggttactt atccagacga gagttcgatg aattatcacg tacaattaaa 36600

accattaata cacgcgcaac gcaacgggct tccgcggaag cagagttgtc ttgctttaat 36660

acgctaagcg cggccgtaga ccaagccgta aaggactatg aaacatataa caatggtgag 36720

gtcaagtatc ctgaaataac acgggatgat ttattagcaa caattgtacg tgctacagac 36780

gatttggtgc gacagataaa aattttaagt gatccaatga tccaatccgg tttacaacct 36840

tcgattaaaa gacgattgga aacaaggctt aaagaggttc agacgtatgc aaacgaggcc 36900

cgaaccacac aggacacaat aaagagtcga aaacaggcgg catataataa actcgggggg 36960

ttacttcgcc cggtaaccgg ttttgtggga cttagggctg cagtagattt attaccggaa 37020

cttgcttctg agttagatgt ccaaggagcc ctggtaaatc tcaggaccaa agtcttagag 37080

gcgccggtag agatccgttc tcaacttacg ggtgatttct gggcgttatt taaccaatat 37140

cgagacattt tagaacatcc cggaaacgca cgcacatctg tcttaggagg actgggagct 37200

tgttttacag ctattatcga aattgtgccg atacctacgg agtatagacc atcattgctt 37260

gcgttttttg gtgacgtggc agatgtgctt gcatccgaca tcgcgaccgt atctactaac 37320

ccggaaagtg agtccgccat aaacgctgtt gttgcaactc ttagtaaagc gacgttagtt 37380

tcatctacag tgccagcctt atcctttgtg ttgtcgttat ataaaaaata tcaggcttta 37440

caacaagaaa ttacgaatac ccataagttg actgaattac aaaaacaact tggagatgac 37500

ttctccaccc tagctgtctc atctggacac ttgaagttta tatcatcttc aaatgtagat 37560

gattatgaaa taaacgatgc gatattatca atacaaacaa atgtgcacgc cctgatggat 37620

acggttaaac ttgttgaagt tgaactgcaa aagctacccc cccattgtat tgctgggaca 37680

tctaccttat ctcgagtagt aaaggatctt cataaactcg tcacaatggc acatgagaag 37740

aaggaacagg caaaagtgtt aattaccgat tgtgaacgtg cacataaaca acaaacgact 37800

cgggttttgt atgagcgttg gacacgtgat attatagcat gtctggaggc aatggaaacg 37860

cgccatgtat ttaacgggac agaactggca cggttgcgag atatggccgc tgcgggaggg 37920

tttgatatac acgcagttta cccacaagca cgtcaggttg tagcggcatg tgaaactaca 37980

gccgttacgg cattagatac cgtgtttcgc cacaatccac atacccccga aaatacaaat 38040

attcccccac ctttggcttt gttaagaggg ttaacatggt ttgatgattt ttcgattacg 38100

gctcccgtat tcaccgttat gtttccaggt gttagtattg aaggactcct tctgcttatg 38160

cgtattcgcg cggttgtgtt attatccgcc gatacgtcta ttaatggaat acctaactac 38220

cgagatatga tattacgaac ctcgggggat ctattacaaa tacccgcatt ggctgggtat 38280

gttgattttt acacacggtc ttatgatcag tttataaccg aaagtgtaac gttaagtgaa 38340

cttagagcag acatcagaca ggctgccggg gctaaactta cagaagcaaa taaggctttg 38400

gaggaagtaa ctcatgttcg ggcacacgaa acggctaaac ttgcacttaa agaaggtgtc 38460

ttcattacat taccaagcga aggtttattg attcgggcta tagagtattt tacaactttc 38520

gatcataaac gatttatagg aacggcatat gaaagagttt tacaaacaat ggtagaccgc 38580

gatctaaagg aggccaacgc agagcttgca cagtttcgta tggtgtgtca ggcaacaaag 38640

aaccgtgcaa tacaaatttt acaaaacatt gttgatactg ctaatgccac tgagcaacaa 38700

gaagacgtgg atttcactaa cctgaagacg ttattaaaac taaccccccc tcccaaaaca 38760

attgcattgg ccattgatag atctacttcc gttcaggaca ttgtcacgca gtttgcattg 38820

ctgttagggc gtctggaaga agaaactggt acgttggaca ttcaggcggt tgactggatg 38880

taccaagctc gcaatattat tgactcccat ccactaagtg tgcgtataga cggtaccggc 38940

cccctgcata cttataaaga tagggtggat aaactttatg cgttacggac taaattagat 39000

ctcctacgac gacgaataga aaccggtgag gttacgtggg acgatgcatg gacaacattt 39060

aaaagagaaa cgggggatat gttggcatcg ggggacacgt acgctacttc cgtagatagt 39120

ataaaggcac tccaggcatc ggcgtctgtg gttgacatgc tttgttccga acccgaattt 39180

tttttattgc ctgtggaaac gaaaaaccgt ctccaaaaaa agcaacaaga acgtaaaacg 39240

gcgttggatg ttgtgttgca aaaacaaaga cagtttgaag agaccgcgtc tcgcttacga 39300

gctttaattg aacgtattcc aacggagagt gaccatgacg ttcttcgtat gttattacat 39360

gatttcgatc aatttacaca tttgcctata tggataaaaa cacagtatat gacatttcga 39420

aatttactca tggtacggtt aggcttgtat gcaagttatg ctgagatttt tccacccgcg 39480

tctccaaacg gagtgtttgc tcctattccc gccatgtcgg gtgtatgtct agaagaccaa 39540

tcccgatgca ttcgcgcgcg ggtggccgcg tttatggggg aggcgtctgt ggtgcaaacg 39600

tttagggaag ccagatcttc tatagacgct ttgtttggaa aaaatttaac cttttacttg 39660

gatactgatg gggttccact tcgatataga gtgtgttata aatcagttgg ggttaaactt 39720

ggaaccatgc tatgcagtca gggtggatta tctttacgac cggcacttcc cgatgaaggt 39780

attgtggaag aaactacact atcggcatta cgcgtggcca atgaggtcaa tgagctacgc 39840

attgaatacg aatccgctat aaaatccggg ttttctgcct tttccacctt tgttaggcat 39900

cgccacgccg aatggggtaa aaccaacgca cgcagagcca ttgcagagat atacgccggc 39960

cttataacaa caacattgac acgacaatac ggggttcatt gggacaagct tatttattct 40020

tttgaaaaac accacctaac ttctgtaatg ggcaatggac taactaaacc aatccagaga 40080

aggggtgatg tacgcgtatt agagttaacc ctatctgata ttgtaactat tttggttgcc 40140

acaaccccgg tacatcttct caattttgct agattggatt taattaaaca gcatgagtat 40200

atggcccgta ccctcagacc cgtaatcgag gccgcattta gaggtcgttt actcgttcgc 40260

tcattggatg gagacccgaa aggcaatgcc cgggcctttt ttaatgccgc cccatccaaa 40320

cataaactcc cgttagctct tggatcaaac caggatccta ccggcgggag aatatttgca 40380

tttcggatgg cagattggaa acttgttaaa atgccacaga aaataacgga tccttttgcg 40440

ccatggcaac tttccccccc ccccggggta aaggccaatg tcgatgcagt tacccgtata 40500

atggcaacag atcgtcttgc gaccattact gtacttgggc gcatgtgtct cccgccaatt 40560

tccttagtgt caatgtggaa tacgctgcaa ccggaggaat tcgcatacag aacacaagat 40620

gatgtggaca ttatagttga tgcgagactg gatttgtcat ccacgcttaa tgcaagattt 40680

gataccgctc ccagcaatac cacgttagag tggaatacag accgtaaagt aattacagat 40740

gcttatattc aaaccggggc aacgacagtt tttacagtaa cgggggcggc accaactcac 40800

gtttctaatg taacagcgtt tgacatagca actacggcta ttttatttgg ggctcctttg 40860

gttattgcca tggaacttac atccgttttt tcacaaaatt ccggacttac tttggggtta 40920

aaattattcg attcccggca tatggctaca gattcgggta tatcctcagc cgtatctccc 40980

gatattgttt cttgggggtt acgtttactg catatggatc cccacccaat tgaaaatgca 41040

tgtttaattg tccaactaga aaaactgtcc gcgctcattg caaacaaacc tcttacaaac 41100

aatcccccgt gtttactgct attggacgaa catatgaatc cctcttatgt tttatgggaa 41160

cgaaaagact cgattccagc tccggattat gtggtctttt gggggccaga atctcttatt 41220

gatttgccgt acatcgactc cgatgaggac tctttcccct cgtgtcccga tgatccattt 41280

tactcgcaaa ttattgccgg ttatgcgccc caaggccccc caaacctcga cacaactgat 41340

ttttacccaa cggagccact atttaagtct cccgttcaag ttgttagaag ttccaaatgt 41400

aaaaaaatgc ccgtccagcc cgtccagccc gcgcagcccg tccagcccgc gcagcccgcg 41460

cagcccgcgc agcccgtcca gcccgcgcag cccgtccagc ccgcgcagcc catagaaccg 41520

ggcacacaaa tagtggtaca aaattttaag aaaccccaaa gcgtaaaaac aacccttagc 41580

caaaaagata ttcccttgta tgtggaaact gaatcagaaa cggctgtgct tatacctaag 41640

caattaacca cctccattaa aacaaccgtt tgtaaaagta ttaccccacc aaataaccaa 41700

ttgtcggatt ggaaaaataa tccacagcaa aaccaaacgt taaaccaagc gttcaataaa 41760

ccaatacttg agattacctc cattccgaca gatgactcga tatcttaccg gacttggatt 41820

gaaaaatcaa atcaaacaca aaaacggcat caaaatgacc ctcgaatgta taactccaaa 41880

acagtattcc accctgtaaa taaccaatta ccttcttggg ttgacacggc agccgatgcc 41940

ccccaaacgg acctattgac aaactataaa acaagacagc cgtcgccaaa ctttccgcgg 42000

gacgtacaca catggggcgt atcttctaac ccgtttaact caccgaacag agacctatat 42060

gaaagtgatt ttagtgaacc ttctgacggc tatagcagtg agagtgaaaa ttctatcgta 42120

ctaagtctcg acgaacatcg gtcatgtcgc gttcctaggc acgtacgtgt tgttaatgcc 42180

gatgtagtca ccggtcgacg ttatgtccga gggaccgcct tgggagcact ggccctgtta 42240

agccaggcat gtcggcgtat gatcgacaac gttagatata cacgtaaact tttaatggac 42300

cacacggaag atatatttca aggcctgggg tatgttaaat tgttattaga tggaacatat 42360

atataaagta gcgcctatta aagaaaaaaa aaacaacgat tattttctgt gtatttttat 42420

ttacacccta cgacttcttg aagcgtttcc agattgtccc gtgtgggaca aggtctgtcc 42480

cttacccccc tggggggtat tttgggttgg gggcggggta gactgtggca cgccttgggc 42540

cgcgggcggt gatccggttg ttggctggac agtgcttgac tgtgctccct gttgcggttg 42600

ttgtccagaa gaccccgaca ccacgtgttg ctgttgtcca acggatgccg acgtcgtttg 42660

aggtgggggg tgttgcgggg atgatcccga aaacgccaac gcggcgggct gttgtaaagc 42720

agactgatcg gcgctctgtg ttttttgcgg caatatagta ggccccgaga ttcccaaact 42780

catggatgga tttgggggtt gtggtcgtat aatacgcggg ttaaacgtac gttttaagcc 42840

aaccgttggt cttaaccatg tcatagggtc agtctcggca aacatggccg ttcggcgtat 42900

cgtatttgca ttatggttag cgcgtgcacg cgcggcactg gccgcggctc ccacggtgta 42960

aatgcttctg gcatcagcga tgtccacacg gtgaccaggt tgcaaaggtc cactggcgtt 43020

taaaagtcgt attaaagcaa cgggggtgta agccgcaatt gcttccaccg aaaatgtggt 43080

ggggttgctg ggatcaaaga ctacacgaga cgatgcgggt tgtgtcatcg tttattagtt 43140

tacgggacaa tcgataacag catacacgta catctgcgca ggatatgtac ggaaaggcaa 43200

tttatttcca gaaaagcacc gcccctaata caactaccag tacaattaca acgaacaggg 43260

catatgtcac gttagctacg ggtagagcaa gtttccagac acgcgtagtt tgggtatcgg 43320

gtaacgcagg tttaatgtca ctttgcattt gaacagacgt gtttggactt ccgttctcgg 43380

gtggggatct gaatgaaggc cgccagcgta tatattcatc caaattattg ccagtttcct 43440

tatacatgta tgcatccgtg gcgcgggcca taagtttaat ggtgcgagat ggatcttccg 43500

gtcccataaa acgaaaggat aactgaacat atggcattcg cacaaagcag ttcacccaca 43560

ttaaagcctg gagaggtcgg cggtcaatac ccccacctcg tttaattgat tccaaagcag 43620

ataggttgat accggtactt aacgttgaac taagaatcac gttattactg tcaatggaca 43680

cttcagccac tggtgcgtta gtcggacgaa aaaaaaaacc ttgaaatagc acagacaccc 43740

ccgtattttg aatttttatg taagggtcac aatctacttg cgcccaattc gccattaaac 43800

gcataatata ctctaccgga aaggcttcgg atacgttgtc ttcgccgtta aactgaaaaa 43860

cacaacgggc gggggggcgt tgtggatcaa atattggaag atccccatcg caacattgaa 43920

gagcgcttgg taccaccaac cgaatacgtt gtaaaagatt atctccgcaa cccctcctgc 43980

gttcactccg tacatacgtt ctccgtgaca tattgatcta aggttgcaaa ccaaggcaca 44040

cgcgtgaagt atttagacca tttatcgtgg gatataggag gagtttggag tgatccaccc 44100

cctgacgact tattaatgcg tttattttcc ccatgtatta agcatccttc aatatttcat 44160

gcaaatctag aaatttggcc atgactcccg caaagcgttc acggcgacgg gtcacgctgg 44220

cactatgttc acatggaaca acataagcag atttttctga atcgttactt tctttatgtt 44280

ttaaaacgga cgccaggcga ctggtaaatg atatataatt taattgagcg tcagttgtag 44340

gtagaattgc ttctatttcc gggggaatta aattttcaaa ccaaacggaa agagtaaagg 44400

tgctatcagc aggaaaatac tttgactcca gtgcatcgat atttaataga ttaacatcgg 44460

tgtctgtaat taaatcgcgg gccctcatcc cagagatgga tcgggtagaa tcagaagaac 44520

ccatggatgg attcgaatcg cccgtattct ccgaaaatac atcttctaat tccggatggt 44580

gttccgacgc attttccgat tcgtacatcg cttataatcc agcccttctg ctaaaaaacg 44640

atttgttatt ttcagaattg ttatttgcct cccacttaat aaatgttccc cgtgcaatag 44700

aaaacaacgt cacttatgag gcctcttcgg cggtaggtgt ggataatgaa atgacctcaa 44760

gtaccactga atttatagaa gaaattggag acgttttggc gttagacaga gcctgtttgg 44820

tctgtagaac gcttgatttg tataaacgta aatttggact gacaccggaa tgggttgcgg 44880

actacgccat gttatgtatg aaaagtctgg catccccgcc ctgtgcagtt gtcactttta 44940

gcgctgcctt tgaatttgtg tatcttatgg atcgttacta cctgtgccgt tataacgtta 45000

ctttggttgg gtcctttgcc aggcgcacgc tttccctgtt agatatacaa agacattttt 45060

ttttgcatgt atgttttcgt accgatggag ggttaccagg tatacgaccg ccccccggta 45120

aggaaatggc caacaaagta agatattcca attactcctt ttttgtacag gcggtagtta 45180

gggctgcatt actatcgatc agcacgtctc gtttagacga aaccgaaacg cgtaagtcat 45240

tttactttaa tcaggacgga ctgactggag gccctcaacc tttagcggcc gccttggcta 45300

attggaaaga ttgcgcgcgg atggttgact gttcatcatc ggaacatcgc acaagtggga 45360

tgattacctg cgcggaacgt gcattaaaag aggatataga gtttgaagat atattaatag 45420

acaaacttaa aaaatcgtct tacgtagaag cagcttgggg ttacgcagac ttggctttat 45480

tattactgag tggggttgct acttggaatg tagacgagcg tacaaattgt gctatagaaa 45540

ctcgcgttgg atgtgttaaa tcatactggc aggcgaaccg gattgaaaac tccagggacg 45600

ttccaaaaca attttccaaa tttacgagcg aggatgcctg tcccgaagta gcatttgggc 45660

ctattttgtt aactacctta aaaaacgcaa agtgccgtgg tcgcacgaat accgaatgca 45720

tgttatgttg tttattaacc atagggcact attggatcgc tttgcggcag tttaaaaggg 45780

atatattagc atactcagca aataacacaa gtttatttga ctgtatcgaa cctgtaatca 45840

atgcatggag cctagataac cccattaaac ttaaatttcc atttaatgat gagggtcgat 45900

tcataaccat tgtaaaagca gcaggttccg aggccgtata taaacattta ttttgcgatc 45960

tcctatgcgc tctctcggaa ttacagacaa accctaaaat ttatttgccc catcctacaa 46020

ccgcggataa ggaagtgttg gagttatata aagcccaact ggctgcacaa aacagatttg 46080

aaggtcgtgt atgtgctggc ctgtggacat tggcgtatgc atttaaagcc taccagattt 46140

ttccacgcaa accaaccgcc aatgccgcat tcatacgaga tggaggactt atgcttcgac 46200

gacatgcaat atcgctggtc tccctcgaac acaccctatc gaagtatgtc taggcgatat 46260

aaatccgtat ctcggagcgg gccttcgatg cgtgtacgct ccagaacgcc atgccgccgt 46320

caaaccattc gaggaaaact tatgtcaaag gagcggtctg tgtaccgcca ttattttaat 46380

tacatcgcaa ggtccccccc agaagaacta gctaccgtta gaggcttaat cgtgccaatt 46440

attaagacga cccctgtcac ccttccgttt aacttgggtc agacagtggc ggataactgc 46500

ctgtcgttat ccggaatggg ttatcattta ggtctcggag gttattgtcc gacatgcact 46560

gcatctggag aaccgcgtct atgtcgaacc gatcgggcgg ctctgatact agcatatgtt 46620

cagcagctta acaacatata cgaatatcgt gtgtttcttg catccatttt ggcgctatca 46680

gaccgagcca acatgcaagc agcgtccgct gaacccctat tgtcgagcgt attggcacaa 46740

ccggaattat tttttatgta tcatattatg agggaggggg gcatgcgaga tatacgcgta 46800

cttttttatc gtgatggaga tgccggaggg tttatgatgt atgttatatt tccggggaaa 46860

tctgttcacc tccattacag actaatcgat catatacagg ccgcgtgtcg ggggtataaa 46920

atagtcgcac acgtttggca gacaacattt ttactgtcgg tatgtcgcaa cccagaacaa 46980

caaacagaga ctgtggtgcc atccattgga acatcggacg tttactgtaa aatgtgtgac 47040

cttaactttg atggagaatt gcttttggaa tacaaaagac tctacgcatt atttgatgac 47100

tttgttcctc ctcggtgatt tcagcttcag tgttcatttt attatcccag cgcggggcgt 47160

gtatacaaac aaagcctgcc gcctgcaagc ggtttagcat tttaacgtta acaactcgtg 47220

tctctggaat aaaacgtttt aaaagccgtt ctgtgagttt agtgtcgttt ccaaataacg 47280

ccttaaaagt tacactcgcc gtcccaatga gatgagaaaa ataatagtca atgtttaaag 47340

acagcccgtg tgatgttacg tgaatgggat cttccgctaa gtcagatatt attaacttac 47400

gctttgcttc cccacaccgt ttacctgcgg tattctgtaa aggatctcca cgtagcaaag 47460

ctacactttt tgcatcagcc tccacttcgt ctgtgggggc cacaataaca taagggatgc 47520

gttctcgaac gtttgggatt tgaccctgtc tcattactaa tttataatat actgttaagt 47580

gagccaagcg acggtttatg taggcggatg gtggacgact aagctcggcc gtcataacaa 47640

acttattaat atccaatttg ggtgatgtaa tctggcgatg tgcatctgca attatgcgtc 47700

caaacccggc catcccagac ggcatggccc gtctattcca ttcagcaatg gaaacacacg 47760

acgcctccgc cgcagcacgc gagacggtgt cgtcatataa caacagttct acaagtttgc 47820

gggcataatc gttaataaat tgacagttgt tttttctaac caagtcgact cccttcatta 47880

aaacctttcc gccgtaaatt accccaatgt actttttctt tgttataagt aaaagtttta 47940

taaaagtttt ttcacactcc aactttatag gaggacaaaa cagagccgtt gaaattatat 48000

gtgccatttt ctcgccgatt ttagctatcc cctcaacacc aacacccttg aatcggataa 48060

acacagaatc cgtatctcca tatataacct ttacctcgta cgctttttgg gagagaacgc 48120

tactttcaat gtctggaaac gctgtaataa aacgttcaaa tgcggcccag ttattatgaa 48180

tataatctct ggtacttaat aacatttgac ggccaattgt agtgacagtg gccgctacgt 48240

ataaacatgg cagaaatccc tgcgcaactc cagtaaaacc gtacacggaa ttacaaacta 48300

cttttatcgc ggcttgttgt ttgtctaata acactgcttc atctgaagaa cttccgggta 48360

tgcgcgctct aatagccttg cgcatagcca accagtcttt taaaagaaca cccagcagac 48420

tttctcgaac gttagagcgc acaaaaaaaa gacgttttcc tccaactgta aaggtggcat 48480

aatcggatgg attcaaacgt ttaaccgtct caaaatttaa cgttagcgtg gtaaaacata 48540

agttatgggc ctgaattata cttggatata aacttgcaaa atccaatacg accaccggat 48600

cgatataaaa tcccgtatca gggtcaaaaa ccctggctcc tttatatcct acatttcgcc 48660

cacttgacgt accagtggga gaaacgctct cgtcttcatc catctcttcc tcaacatccc 48720

cgacatcggg aataacatcc ttatattcaa aagtagctgg gtatccccca tcgggtaaaa 48780

taaatcctcg agacgaagcc agtcctaata aacaagtgta aatcctaacc tgctgtccgt 48840

cgtaaatagc cttggttaaa gtaattctag ctagccttgc aaccgcggat aactcaaggt 48900

gtggtaaata tttaaaaaac agtttcccca caagagccga gtcttgtata caatattcac 48960

caataattcc tcgtgtattc ggtccactag cgtaatatcc cggaatgtct ttgtagggca 49020

aatctctctt ggactcattt agagcttcac gtgcaaccga atctaattta taactcgaga 49080

gttttaattt ttcagttgca attgcataca tatccagaga tatgagaccg ttgatcttta 49140

ccttgcttcg tcgctgaaat ccggatttgc caacatccca tatcttaaac agacccccac 49200

ggtttatact gccataacca tcaagcttga gactgtatat agaattaagt ttctccataa 49260

taaacgccca atcaaaatta acaatgttat aacctgtggc aaactcggga gcgtactgtt 49320

ttacgagggt cataaatgca attaatagct cgaattcact atcaaactcc agcacagtcg 49380

gctccggtaa ccccgcgtcc ttcatttctt gtacatacct ttgtggtaag tcacaagagc 49440

caagggaaaa cagtaaaatg tgttctaaag actgtcgagg gattgaatat aatagacaag 49500

aaatttggat tacaagatcc tccaaatgtg ttgcatcggg aaacgccagc tcattagatc 49560

ctcctgattt acattcaata tcgaaacata acaacttgta gtcaggccat gagtcatcgt 49620

ttggtatagc ctgcagatta tccgacatgc agtcaatttc aacgtcgctt aacgttaatt 49680

ggcgacttgc cggtcgaact cgaacacgtt ccccatcaac tccaggtttt agttgatacc 49740

aaccaaaact aacaaagccg ggattatcca ttagaaaacg agtggtagcg tctacccgac 49800

cttcatactt tttcaactcc gggtgaaagt tatcacaaag ataatttgta aatttagatg 49860

agggagaata caccctgtaa aacgcacatg gctgtgtatc gtagtaataa acatctgtgc 49920

gctcaataac ctcaacgcga aagctttctg gagatgcgct tttaaacgag gtaccatgaa 49980

aagcgttctt gtctccattt aacgttgcat cattttgtgt tatcatagaa ctgcgtaaac 50040

actcggcaag taatacagat aactcgctac aggaacgtat gccacaagcg gtatccacct 50100

cggctttgtt tatataaaaa tattgacaga tgccgtatac atgaactgcc accctttttc 50160

cacatcggga catgccaagt aaagtaataa cggtaccaag cggtcgtgtt gcagttgcaa 50220

accgggatac atctccatta gacgcggctt ctgttgtttc gacaatatca tatacatgga 50280

atgtgttaaa gcgggggtca aacttatccc cacgaaagtc gatttccccc caaatattca 50340

cgcgtctagg ccaggggctg gaacaacgaa aatccagaat cggaacttct tttccattac 50400

agtaaacttt aggcggtcga ctaagtgtac cgacgtgaac cccctttcgt tcttccatgg 50460

gcacatcttc atctaaacat ttaggggcca aaaattgaaa cgatgacatg gtagttttgt 50520

aactatgaag aaattctctg ttactaccgc gcccggttct tgggttatat ttaatccctg 50580

atgcttgggt taaaaaggga ttacaaaacc ccgttctgat cgccatttta tgttaacgat 50640

tgataatctt gtaaaaagcc agtgttactg agtaacacaa ccccacgccc ttctaataca 50700

taaagtgtaa tcacgtgatt tgttgtggtt tccgcatatg taatacccgt ttaaaagcct 50760

ctcttcttaa tgtatcgaca gactgggttt tgggtggtca tttgaccctg ccaacaaccc 50820

cccattatta cgagtacttc accaaaatgg aaaatactca gaagactgtg acagtgccca 50880

cggggcccct gggttacgtt tatgcgtgcc gggttgaaga tttggatctg gaggaaattt 50940

catttttggc cgctcgtagc acggactctg atttggcttt attacctttg atgcgtaatt 51000

tgaccgtgga aaaaactttt acatccagcc tggcggtggt ttctggagca cgcactacgg 51060

gtcttgccgg agctggtatt accttaaaac tcactaccag tcatttctat ccatctgtct 51120

ttgtctttca cggaggcaaa cacgttttac ccagctctgc ggccccaaat ctcacacgcg 51180

cgtgtaacgc ggctcgagaa cggtttgggt tttcacgctg ccaagggcct cctgttgacg 51240

gtgctgttga gacgaccggc gctgagatat gcacccgcct tggattagag ccagaaaata 51300

caatattata cttggtggtc acggcattgt ttaaggaagc cgtatttatg tgcaacgtgt 51360

ttctgcatta tggaggactc gatattgttc atattaacca tggggatgtt atacgtatac 51420

cgttatttcc ggtacaactt ttcatgcccg atgttaaccg tctggtaccc gacccattca 51480

acactcatca caggtctatc ggagagggtt ttgtataccc aacacccttt tataacaccg 51540

ggttgtgcca tttaatacat gactgtgtta ttgctcccat ggccgttgcc ttgcgcgtca 51600

gaaatgtaac tgccgtcgcc cgaggagcgg cccaccttgc ttttgatgaa aatcacgagg 51660

gggcagtact cccccctgac attacgtaca cgtattttca gtcctcttca agtggaacca 51720

ctaccgcccg tggagcgcgt cgaaacgatg tcaactccac gtctaagcct agcccatcgg 51780

gggggtttga aagacggttg gcgtctatta tggccgctga cacagccttg cacgcagaag 51840

ttatattcaa cactggaatt tacgaagaaa ctccaacaga tatcaaagaa tggccaatgt 51900

ttataggcat ggagggcact ttgccaaggc taaacgctct ggggtcatat accgctcgtg 51960

tggccggggt cattggtgcg atggttttca gcccaaattc tgcgttgtat ctaactgagg 52020

tggaggatag cgggatgacc gaagccaagg atgggggacc gggtccatca tttaatcgat 52080

tttaccagtt tgccggacct catttagctg cgaatcccca aacagatcga gatggccacg 52140

ttctatccag tcagtctacg ggttcatcaa acacagagtt tagcgtggat tatttggcac 52200

tcatttgtgg atttggagca cccctgttgg cgcgactgct tttttatcta gaacgctgtg 52260

acgctggtgc gtttacaggg ggtcacgggg atgcgttaaa atatgttacg gggacctttg 52320

actctgaaat tccatgtagt ttatgtgaaa aacacacgcg gccggtatgc gctcacacaa 52380

cagtacaccg acttagacaa cgcatgccgc gatttggaca agccacccgt caacctattg 52440

gggtgtttgg aacaatgaac agccaatata gcgactgcga tcctctagga aactatgctc 52500

catatttaat ccttcgaaaa cccggggatc aaacggaagc agcaaaggca accatgcagg 52560

acacttatag ggctacacta gaacgcttgt ttatcgatct agaacaagag cgactactgg 52620

atcgcggtgc cccatgttct tccgagggac tatcgtctgt cattgtggat catccaacgt 52680

ttcgtcgcat attagacaca ctgcgtgcgc gtatagaaca gacaacaaca caatttatga 52740

aagtgttggt tgagacccgc gattataaga tccgtgaagg attatccgaa gccacccatt 52800

caatggcgtt aacgtttgat ccatactcag gagcattttg tcccattacc aattttttag 52860

ttaaacgaac acacctagcc gtggtacaag acttagcatt aagccagtgt cattgtgtat 52920

tttacggaca gcaagttgag gggcggaact ttcgtaacca attccaacct gttttgcggc 52980

ggcgttttgt tgacctgttt aatggggggt ttatatcaac acgctctata accgtaacat 53040

tatctgaagg tcctgtatcc gccccaaatc cgacattggg acaagacgcg cccgcggggc 53100

gtacctttga tggggattta gcgcgcgtaa gcgtggaagt tattcgggat atacgagtta 53160

aaaatagggt cgttttttca ggtaactgta caaatctctc tgaggcagcc cgggcaaggc 53220

ttgtaggcct tgcaagtgcg taccaacgcc aagaaaaaag agtggatatg ttacacgggg 53280

ccctagggtt tttgcttaaa cagtttcacg gcctgttatt tcctcggggt atgccaccaa 53340

acagtaaatc ccccaacccg cagtggtttt ggaccctgtt acaacgcaac cagatgccgg 53400

cagataaact tacacacgaa gagattacca ctattgcagc tgttaaacgg tttaccgagg 53460

aatatgcagc actaaacttt attaatctac ccccaacctg cataggagaa ttagcccagt 53520

tttatatggc aaatcttatt cttaaatact gcgatcattc acagtacctt ataaatacct 53580

taacttctat aattacgggt gccaggcgcc cgcgtgaccc atcatccgtt ttgcattgga 53640

ttcgtaaaga tgtcacgtcc gccgcggaca tagaaaccca agcaaaggcg cttcttgaaa 53700

aaacggaaaa cttaccggaa ttatggacta cggcttttac ttcaactcat ttagtccgcg 53760

cggccatgaa tcaacgtccc atggtcgttt taggaataag cattagtaaa tatcacggag 53820

cggcaggaaa caaccgcgtc tttcaggcag ggaattggag cggtttaaac gggggtaaaa 53880

atgtatgccc gctatttaca tttgatcgca ctcgccgttt tataataaca tgtcctagag 53940

gaggttttat ctgccccgta acaggtccct cgtcgggaaa tcgagaaacc accctatccg 54000

accaagttcg cggtataatt gtcagtggcg gggccatggt tcaattagcc atatacgcca 54060

cggttgtgcg tgcagtgggc gctcgagcac aacatatggc atttgacgac tggttaagtc 54120

ttacagacga tgagttttta gccagagact tggaggagtt acacgaccag attatccaaa 54180

ccctggaaac gccctggacc gtagaaggcg ctctagaagc agtaaagatt ctagatgaaa 54240

aaacgacagc gggagatggg gaaaccccca caaacctagc atttaatttt gattcttgtg 54300

aaccaagcca tgacaccaca tctaacgtat taaacatttc agggtcaact gtccctggtc 54360

ttaaacgacc ccccgaagat gacgaactct ttgatcttag tggtattccc ataaaacatg 54420

ggaacattac aatggaaatg atttaacctc cctctttatc caattaaagc ccacacgcgg 54480

gtgagtgtac gtaataaaca agtcaatatt acatattctg ttgtgttttc ttttttgcgt 54540

gtagtcctta cccatatgac ctgtaatata gtgtgtctcc aaccattcag cttacagtcc 54600

agtggacagt aacagcccga taacatggaa ttggatatta atcgaacatt gttggttcta 54660

ctgggtcaag tttatacgta catctttcag gttgaactgc tacgtcgatg tgatccaagg 54720

gtggcgtgtc gctttttata tcggttagcg gctaactgtt tgacagttcg ttatttatta 54780

aagctgtttc tccggggatt taatacccag ctaaaatttg gaaacactcc cacggtttgt 54840

gcactgcatt gggcattatg ttatgtaaag ggagaaggtg agcgtttgtt tgagttgcta 54900

caacatttta aaacgcgttt tgtttatggt gagactaaag actcaaactg tatcaaagat 54960

tactttgtct cagcgtttaa cttaaaaacc tgccaatatc accatgagct gtcgttaaca 55020

acatacggag gttacgtatc gagtgaaatt cagtttttac acgacattga gaatttttta 55080

aaacagctta attactgcta tattatcacg tcttctcgtg aggcgctaaa cacattggaa 55140

accgtgacgc ggtttatgac agatactata ggaagcggtc taataccacc cgtggagttg 55200

tttgatccgg cgcatccatg tgctatatgt tttgaagaat tatgtataac agctaaccaa 55260

ggtgagacct tacatcgtag attattagga tgtatctgcg atcacgttac taagcaagtt 55320

cgggttaacg tggatgttga cgatattatt cggtgtttac catatatccc tgatgtaccg 55380

gatatcaaac gtcaatccgc cgttgaagcg ttacgaacac ttcaaaccaa gacggtagtc 55440

aatcccatgg gagcaaagaa cgatacgttt gaccaaacat acgaaattgc gagcaccatg 55500

cttgattctt ataatgtttt taaacctgcc cctcggtgta tgtacgccat cagcgagctt 55560

aaattctggt taacgtctaa ttccactgaa ggaccccaac gtactttaga cgtgtttgtt 55620

gataatttgg atgtattaaa cgaacatgaa aaacacgcag aacttacagc cgtaacggtt 55680

gagttggcgt tatttggaaa aactcccata cactttgata gggcgttttc tgaagaactc 55740

ggatctctgg atgcaattga tagtattttg gttggcaatc gctcatcctc accggacagt 55800

cagatagaag cattaattaa agcctgttat gcccatcatc tatcgtcgcc tctcatgcgt 55860

cacatttcta acccgagtca tgataacgaa gccgccttac gccaactttt agaaagagtt 55920

gggtgtgagg atgatttaac caaagaggcg agtgacagcg ctacagcatc cgaatgtgat 55980

ctgaacgatg atagtagcat aacttttgct gttcatggat gggaaaacct gttatccaaa 56040

gcaaaaattg acgctgcgga aagaaaacga gtatatcttg aacatctgtc taagcgctct 56100

ctaaccagcc tcggtagatg tatccgcgaa cagcgccaag agctagaaaa aacactcagg 56160

gtaaacgttt atggagaggc cttattgcag acatttgttt cgatgcaaaa tgggtttggg 56220

gcacgaaacg tgtttttagc taaggtttcc caggcagggt gtattatcga caatcgcatt 56280

caggaagcgg cctttgatgc acatagattt ataaggaata ccttagttcg acatacagta 56340

gatgcggcta tgttacctgc acttacacat aaattttttg agttggtcaa cggcccattg 56400

tttaatcacg atgaacaccg ttttgcacaa ccccctaaca ccgccttatt ttttaccgtg 56460

gaaaacgttg gcctatttcc gcacttaaaa gaggaattgg caaagtttat gggcggtgtc 56520

gttggttcca actggcttct cagtccattt aggggctttt attgcttttc tggggtagaa 56580

ggcgttactt ttgcacagag acttgcctgg aaatatatta gggagcttgt gtttgcaacc 56640

acactattca cctctgtttt ccattgtggg gaggtgcggt tatgtcgcgt tgaccgtcta 56700

ggtaaggatc cacgcgggtg cacgtctcaa cctaaaggta taggcagttc ccacggaccc 56760

ttagacggca tttatttaac gtacgaagaa acatgtcccc ttgtggctat tattcaaagt 56820

ggagaaacag ggatcgacca gaataccgtc gtaatctacg attcagacgt tttttctctt 56880

ctatacaccc taatgcagcg gctggctccg gattcaacgg acccggcgtt ttcataacct 56940

ccgttacggg ggtgtggtta tgctttttat gcatattttc tatgtttgtt acggcggttg 57000

tgtcggtctc tccaagctcg ttttatgaga gtttacaagt agagcccaca caatcagaag 57060

atataacccg gtctgctcat ctgggcgatg gtgatgaaat cagagaagct atacacaagt 57120

cccaggacgc cgaaacaaaa cccacgtttt acgtctgccc accgccaaca ggctccacaa 57180

tcgtacgatt agaaccacct cggacatgtc cggattatca ccttggtaaa aactttacag 57240

agggtattgc tgttgtttat aaagaaaaca ttgccgcgta caagtttaag gcgacggtat 57300

attacaaaga tgttatcgtt agcacggcgt gggccggaag ttcttatacg caaattacta 57360

atagatatgc tgatagggta ccaattcccg tttcagagat cacggacacc attgataagt 57420

ttggcaagtg ttcttctaaa gcaacgtacg tacgaaataa ccacaaagtt gaagccttta 57480

atgaggataa aaatccacag gatatgcctc taatcgcatc aaaatataat tctgtgggat 57540

ccaaagcatg gcatactacc aatgacacgt acatggttgc cggaaccccc ggaacatata 57600

ggacgggcac gtcggtgaat tgcatcattg aggaagttga agccagatca atattccctt 57660

atgatagttt tggactttcc acgggagata taatatacat gtccccgttt tttggcctac 57720

gggatggtgc atacagagaa cattccaatt atgcaatgga tcgttttcac cagtttgagg 57780

gttatagaca aagggatctt gacactagag cattactgga acctgcagcg cggaactttt 57840

tagtcacgcc tcatttaacg gttggttgga actggaagcc aaaacgaacg gaagtttgtt 57900

cgcttgtcaa gtggcgtgag gttgaagacg tagttcgcga tgagtatgca cacaattttc 57960

gctttacaat gaaaacactt tctaccacgt ttataagtga aacaaacgag tttaatctta 58020

accaaatcca tctcagtcaa tgtgtaaagg aggaagcccg ggctattatt aaccggatct 58080

atacaaccag atacaactca tctcatgtta gaaccgggga tatccagacc taccttgcca 58140

gaggggggtt tgttgtggtg tttcaacccc tgctgagcaa ttccctcgcc cgtctctatc 58200

tccaagaatt ggtccgtgaa aacactaatc attcaccaca aaaacacccg actcgaaata 58260

ccagatcccg acgaagcgtg ccagttgagt tgcgtgccaa tagaacaata acaaccacct 58320

catcggtgga atttgctatg ctccagttta catatgacca cattcaagag catgttaatg 58380

aaatgttggc acgtatctcc tcgtcgtggt gccagctaca aaatcgcgaa cgcgcccttt 58440

ggagcggact atttccaatt aacccaagtg ctttagcgag caccattttg gatcaacgtg 58500

ttaaagctcg tattctcggc gacgttatct ccgtttctaa ttgtccagaa ctgggatcag 58560

atacacgcgt tatacttcaa aactctatga gggtatctgg tagtactacg cgttgttata 58620

gccgtccttt aatttcaata gttagtttaa atgggtccgg gacggtggag ggccagcttg 58680

gaacagataa cgagttaatt atgtccagag atctgttaga accatgcgtg gctaatcaca 58740

agcgatattt tctatttggg catcactacg tatattatga ggattatcgt tacgtccgtg 58800

aaatcgcagt ccatgatgtg ggaatgatta gcacttacgt agatttaaac ttaacacttc 58860

ttaaagatag agagtttatg ccgctgcaag tatatacaag agacgagctg cgggatacag 58920

gattactaga ctacagtgaa attcaacgcc gaaatcaaat gcattcgctg cgtttttatg 58980

acatagacaa ggttgtgcaa tatgatagcg gaacggccat tatgcagggc atggctcagt 59040

ttttccaggg acttgggacc gcgggccagg ccgttggaca tgtggttctt ggggccacgg 59100

gagcgctgct ttccaccgta cacggattta ccacgttttt atctaaccca tttggggcat 59160

tggccgtggg attattggtt ttggcgggac tggtagcggc cttttttgcg taccggtacg 59220

tgcttaaact taaaacaagc ccgatgaagg cattatatcc gctcacaacc aaggggttaa 59280

aacagttacc ggaaggaatg gatccctttg ccgagaaacc caacgctact gataccccaa 59340

tagaagaaat tggcgactca caaaacactg aaccgtcggt aaatagcggg tttgatcccg 59400

ataaatttcg agaagcccag gaaatgatta aatatatgac gttagtatct gcggctgagc 59460

gccaagaatc taaagcccgc aaaaaaaata agactagcgc ccttttaact tcacgtctta 59520

ccggccttgc tttacgaaat cgccgaggat actcccgtgt tcgcaccgag aatgtaacgg 59580

gggtgtaaat agccaggggg tttgttttaa tttattaata aaaatgtgta ttacgttact 59640

catgtgtctc cattacgcat cacagggggt atttataccc gataatatac aaaacgcgtt 59700

ttgtacctct accgcacccg atatcttaac gggggttatt atggaatcgt ctaacattaa 59760

cgcgctacaa caaccgtcgt ctatcgcaca tcatccgtcc aaacagtgcg cttcaagtct 59820

caatgaaaca gtaaaagatt ctccccccgc gatttatgaa gataggttag aacacacgcc 59880

ggtacaatta ccccgcgacg gtacaccccg agacgtatgt tctgtgggac agctaacctg 59940

tcgagcatgt gcaacgaaac cttttcgcct taaccgcgac agccaatacg actacttaaa 60000

cacatgtcca gggggccgtc atatttcact ggcactggag attataacgg gtcgatgggt 60060

ttgcatcccg cgtgtgtttc cggatacccc agaggaaaaa tggatggcgc catatattat 60120

tccagaccga gaacaaccat catcagggga tgaagattct gacaccgatt aaatttaact 60180

taaataaaac cttaccaccc ataaaaacgc cttctgtttg tttaacacga caccgcttaa 60240

caaaaaaaaa acccaaacac gccttttatg aatgtaatac ttttatttgt tggttaacac 60300

cgccccacca tcatctgatt tgcaaacata tcggcgtcgt ctgccgtgga cccctgtatt 60360

aaaggggcct tggaactagc ctccactgca tttacatctt gtccaactgt atctgtatgt 60420

ggggtgcttg ttgtattttg ggatgagcat agacccgaaa cgctttgaag ctgttttaat 60480

aaaatcgata ttcgaggatc ccgtgtcccc tctggtatat ttgtatggtg cgacaaaggc 60540

atttgtgtcc cattttgtga ttttagctct gtaacctcct gttgcagttt tgccacaacc 60600

ccagcaagct cttcgtgctg accattagaa actctgtgtc tcctctgcca atatgatgga 60660

gaaactcgac gtctccgatg cgttatatac gttggttcac cgggaaaata tatatttgag 60720

ggaaactctc cgtccatttg agactcccca ctagaaaaag aatccaattc cctttgatcc 60780

atgctcttga aatcccgttt tcctggacga cggacatcgg ttttgtctgg aaaatttaca 60840

cacggggtct gcaagtcaat accccgttcg gcggccaatg cgttcataaa tgcggacatt 60900

tgcatttcca aacgattggg tggtggatat cccggaaacc cgtacggtcc cccgaagtgt 60960

cccggagggc aaccataacc ccctgtatta aatgggaagg caggcgggtg tggagatcca 61020

tatggcccga cgatatactg tccgttattt ggagctccaa ttgatacctg cggattttta 61080

gtctgcccgg ttaacagctg tgaataatac gcggtaggta tcagtacaaa ttcccctccg 61140

gttggaacgc ccgacggggg ctgtggtgag ataccactag cgttacctgc tacagaagcc 61200

atatcgctgt cgttcctaca caactgcgta acctttaaat gcggaacagt cttttcacaa 61260

tcttcatttg attccccaac acccaacgcg agatcgtata tgggcccgcc ggggtggaat 61320

gtggcgttta taacacccgc gttgggtaat ttagactcca ccccattaac gttggttatc 61380

cgagcaagtc catatccggt gctagcctga agataaacgt gacccataat tccggcttcg 61440

cgtctacgtt ttgcaaccac gtcccatcta tctcttaaaa gcatattgtt cacggctgtg 61500

gataataaca ccttggcgag tttatcttcg ctaaccttcc atactttatt taaacccgcg 61560

tagtctttaa ccagcgacaa taaccgcgct ttactttcca tcgataaaac ccggaatggt 61620

tcaattgaag attccggggt acagtcataa ttgaccactg ttccaacgcg tcttccaaca 61680

acacataacg caacatgggt aaaaaaatta ccgtctggta tctcattcgg ggacaatcgt 61740

tttgaagaca gggatacgga gggtaagtaa tttgtgacca agtataacgc acgttctagc 61800

ggagataata cagaatctct atttccaaaa aaattcgaat gggccgcttc aaacagcacc 61860

gcatgtagtt gagggcatct aacgataccc aaaaaaaaag gtccgcgtat gtcctcaatg 61920

attgcgatta cttcacccac gacacagtct tttcgatgat cgatgtttat tggtatttta 61980

ctagtaggcg gcaaagcgga ccgcacaatc tctggggtaa tatttaattc cccttcgtcc 62040

tttgaatata aggctaaata cccagccacg tataacgctt cacagttctc ttcgtcagct 62100

tcagcagcca ttataaacac cccacggacc ggatagtgaa tactcacggt gtggaggcaa 62160

actgaggaat gacacccaaa cagacaaaat atagaagatc atagtcactg ttaacgttga 62220

actgcgcaag gcggcgactt tcttccaatg ccgcccttac acgcggttgg tgcattaaca 62280

ttccaagtcc ccgttcatat tgcaacataa cactgtcatg tattgatacc acggcggcta 62340

tgggtaggga tgtaacattt tgtcggcggt gttctaattc caatgcaatt aagcttatga 62400

gccgatcttg gtactgtcca gaagaaatat ctattacggt tcttcctaaa cttccacgac 62460

taagctgggt atgcgcgtct aaacaaagag caactaatcc aggaaacatt tcagtcagct 62520

ctgtggtccg atttaacgta tacagtggtg ctatatatcg ttcacataaa aattgaaagt 62580

tattattacc gcttttaaac ttcccatcaa accccgtcgc tccgcgcaag attacattgt 62640

tggtaggggt tcctgttgct tctgacacaa tcaaacccag ttgaaaatta ttttttagtt 62700

tatctccgta tacgttcccg ttccataata agcgccttaa taataataac gccgtaatcg 62760

tgtcaattgt taaccttaat agagtttggt cttccataag aaacacgttt tgggcccgtt 62820

ctaaatacgc cgcggccgcc tgttgaatct tgtccacata tgcggtatga ttgcgatcaa 62880

taatgtcatt aaccccagga ttaaactgtc caggtgcagg cggtaggacc tgcaaccgta 62940

taagcgcatc cataacagaa tgtgacgtta aggcgccttg atcataccgc cccccacgag 63000

catgaaactg gtcgcgtggt agacgatcat agcaaaattg ataactgttt ttattttcgt 63060

gtgttgtcat ataattcaca aatgtctcag tatattccgg taggtgctct ataaggttcc 63120

cgaaggacga aacttgaggt tcgtggacac tattagatgt cctatacatt aaatataaac 63180

ataataccgc acactcgaac gcggagtacg ctctatctcc aacatacatt ctcccggcgg 63240

actgtagaca tgttaccgtt gtgttcataa acgtacggga aatgcgcccg tctttacaat 63300

caactccgcg tgcagctacg ggcctatcta acacaagccg ttcctgcaga gtacgatacc 63360

atggcccgaa aacaatccct ggagagttat tgccccttgc ccttcccaag tacaccaggg 63420

tgataaaatc cacttgaaag tttgtatcgt actgcaacgg tgcatcattt ttggcaatct 63480

gtacctcggg gtgtatagac tcattgcgta ttatttctgt acgtgtacat tcctcagatt 63540

gtgcatctgc ttcttccgcc tcggcagcag ccgtctccag ggaatccaaa accttggcca 63600

tgcgcgttag ttgttcttcg aggggcttta aacgacgatc tatttccgtt ggtaacgtaa 63660

tcgtttcccc gcgaaggttg tctaatgcgg caacggccgc cgcatttttt aacgttaacg 63720

tatttttttc caaatcggga ttcatacgcc ctcttaactc aaacgcggga gccgtccagt 63780

agtgtatggg gaagttgggg gctataaagt tcttagtggt agacaaaaat atcccacatt 63840

tattcggaaa cgagatagat ccgaacccat atctcgccgt catggtgtct gcagcaaaca 63900

aagtcaactg gcgtgaatat aaaccggtac tgctttaaaa gctgttttct tacccatggg 63960

aaaacatccc ggttatactt tgtaaaattc caccacaagc acctaaagaa ggccttctaa 64020

ggggtaaatc caccccacag gctgcatttt cttcaaactt tgttaaagcg gaacgatggc 64080

atgatttcgc acgctttttc gcaagagagc atacgtgaat tttctttttg catagacgtc 64140

ttcgctctct aacggacctt atcggggggg tatattccgc tacattctcc aaatgcgacg 64200

ctagcataac aaggtttcca tgaatcacct tcgggggtaa ccgagttacc tgtaacaggt 64260

tcagaccccg ttgagataca aacacaagga ggggggtcac cattatttca tcagatcccg 64320

tgggtgtggt ttcctttatt aaagccacgg tatccctcag ctggcgcata ccctcgcaaa 64380

actggtgata cttagtaggg gtatgtatat tagcgctaaa acggcaagat tttaattcca 64440

ctataaaaca aacggtcttt ccggcaccac tggattccgt ttgtataata caaacacaat 64500

cggggcgtcg gcgtcccaaa tttacttcaa acgacattga tatgcgtaca gccctttgaa 64560

catccacgtg ggataacggc gacaggagtt ttgccagcct cgggttgaac gcgtccgcga 64620

aacctcgacg tacgttatca atatcctttt tgagtacatc gtaaaaacga gtgtggcaac 64680

gttgtcccaa acgaaaacac ttggcccgaa ttcgactagc ggacatattt gaagttccgt 64740

cccagaagat aacctaagac gcgtttgtct acaataaaca tgtcaacgga taaaaccgat 64800

gtaaaaatgg gcgttttgcg tatttatttg gacggggcgt atggaattgg aaaaacaacc 64860

gccgccgaag aatttttaca ccactttgca ataacaccaa accggatctt actcattggg 64920

gagcccctgt cgtattggcg taaccttgca ggggaggacg ctatttgcgg aatttacgga 64980

acacaaactc gccgtcttaa tggagacgtt tcgcctgaag acgcacaacg cctcacggct 65040

cattttcaga gcctgttctg ttctccgcat gcaattatgc atgcgaaaat ctcggcattg 65100

atggacacaa gtacatcgga tctcgtacaa gtaaataagg agccgtataa aattatgtta 65160

tccgaccgac acccaatcgc ctcaactata tgttttccct tgtccagata cttagtggga 65220

gatatgtccc cagcggcgct tcctgggtta ttgtttacgc ttcccgctga accccccggg 65280

accaacttgg tagtttgtac cgtttcactc cccagtcatt tatccagagt aagcaaacgg 65340

gccagaccgg gagaaacggt taatctgccg tttgttatgg ttctgagaaa tgtatatata 65400

atgcttatta atacaattat atttcttaaa actaacaact ggcacgcggg ctggaacaca 65460

ctgtcatttt gtaatgatgt atttaaacag aaattacaaa aatccgagtg tataaaacta 65520

cgcgaagtac ctgggattga agacacgtta ttcgccgtgc ttaaacttcc ggagctttgc 65580

ggagagtttg gaaatattct gccgttatgg gcatggggaa tggagaccct ttcaaactgc 65640

ttacgaagca tgtctccgtt cgtattatcg ttagaacaga caccccagca tgcggcacaa 65700

gaactaaaaa ctctgctacc ccagatgacc ccggcaaaca tgtcctccgg tgcatggaat 65760

atattgaaag agcttgttaa tgccgttcag gacaacactt cctaaatata cctagtattt 65820

acgtatgtac cagtaaaaag atgatacaca ttgtcatact cgcgtgtacg tgtttttctt 65880

ttttatatat gcgtcattta ttaccacatc ctttaatccc gcctttatct ccctaaaacg 65940

gagtggtaat attaaaagcc gccaagcctg ttggtgggtg aggaggggta aaggcacgct 66000

gtgtgcataa cgttgcggtg atattgtagc gcaagtaaca gcgactatgt ttgcgctagt 66060

tttagcggtg gtaattcttc ctctttggac cacggctaat aaatcttacg taacaccaac 66120

ccctgcgact cgctctatcg gacatatgtc tgctcttcta cgagaatatt ccgaccgtaa 66180

tatgtctctg aaattagaag ccttttatcc tactggtttc gatgaagaac tcattaaatc 66240

acttcactgg ggaaatgata gaaaacacgt tttcttggtt attgttaagg ttaaccctac 66300

aacacacgaa ggagacgtcg ggctggttat atttccaaaa tacttgttat cgccatacca 66360

tttcaaagca gaacatcgag caccgtttcc tgctggacgt tttggatttc ttagtcaccc 66420

tgtgacaccc gacgtgagct tctttgacag ttcgtttgcg ccgtatttaa ctacgcaaca 66480

tcttgttgcg tttactacgt tcccaccaaa cccccttgta tggcatttgg aaagagctga 66540

gaccgcagca actgcagaaa ggccgtttgg ggtaagtctt ttacccgctc gcccaacagt 66600

ccccaagaat actattcttg aacataaagc gcattttgct acatgggatg cccttgcccg 66660

acatactttt ttttctgccg aagcaattat caccaactca acgttgagaa tacacgttcc 66720

cctttttggg tcggtatggc caattcgata ctgggccacc ggttcggtgc ttctcacaag 66780

cgactcgggt cgtgtggaag taaatattgg tgtaggattt atgagctcgc tcatttcttt 66840

atcctctgga ctaccgatag aattaattgt tgtaccacat acagtaaaac tgaacgcggt 66900

tacaagcgac accacatggt tccagctaaa tccaccgggt ccggatccgg ggccatctta 66960

tcgagtttat ttacttggac gtgggttgga tatgaatttt tcaaagcatg ctacggtcga 67020

tatatgcgca tatcccgaag agagtttgga ttaccgctat catttatcca tggcccacac 67080

ggaggctctg cggatgacaa cgaaggcgga tcaacatgac ataaacgagg aaagctatta 67140

ccatatcgcc gcaagaatag ccacatcaat ttttgcgttg tcggaaatgg gccgtaccac 67200

agaatatttt ctgttagatg agatcgtaga tgttcagtat caattaaaat tccttaatta 67260

cattttaatg cggataggag caggagctca tcccaacact atatccggaa cctcggatct 67320

gatctttgcc gatccatcgc agcttcatga cgaactttca cttctttttg gtcaggtaaa 67380

acccgcaaat gtcgattatt ttatttcata tgatgaagcc cgtgatcaac taaagaccgc 67440

atacgcgctt tcccgtggtc aagaccatgt gaatgcactt tctctcgcca ggcgtgttat 67500

aatgagcata tacaaggggc tgcttgtgaa gcaaaattta aatgctacag agaggcaggc 67560

tttatttttt gcctcaatga ttttattaaa tttccgcgaa ggactagaaa attcatctcg 67620

ggtattagac ggtcgcacaa ctttgctttt aatgacatcc atgtgtacgg cagctcacgc 67680

cacgcaagca gcacttaaca tacaagaagg cctggcatac ttaaatcctt caaaacacat 67740

gtttacaata ccaaacgtat acagtccttg tatgggttcc cttcgtacag acctcacgga 67800

agagattcat gttatgaatc tcctgtcggc aataccaaca cgcccaggac ttaacgaggt 67860

attgcatacc caactagacg aatctgaaat attcgacgcg gcatttaaaa ccatgatgat 67920

ttttaccaca tggactgcca aagatttgca tatactccac acccatgtac cagaagtatt 67980

tacgtgtcaa gatgcagccg cgcgtaacgg agaatatgtg ctcattcttc cagctgtcca 68040

gggacacagt tatgtgatta cacgaaacaa acctcaaagg ggtttggtat attccctggc 68100

agatgtggat gtatataacc ccatatccgt tgtttattta agcaaggata cttgcgtgtc 68160

tgaacatggt gtcatagaga cggtcgcact gccccatccg gacaatttaa aagaatgttt 68220

gtattgcgga agtgtttttc ttaggtatct aaccacgggg gcgattatgg atataattat 68280

tattgacagc aaagatacag aacgacaact agccgctatg ggaaactcca caattccacc 68340

cttcaatcca gacatgcacg gggatgactc taaggctgtg ttgttgtttc caaacggaac 68400

tgtggtaacg cttctaggat tcgaacgacg acaagccata cgaatgtcgg gacaatacct 68460

tggggcctct ttaggagggg cgtttctggc ggtagtgggg tttggtatta tcggatggat 68520

gttatgtgga aattcccgcc ttcgagaata taataaaata cctctgacat aaaaaacatg 68580

tataataaaa agtcactata aacgtattct ctacaatact ttattcgcga ataatacaca 68640

ctacctttgg gtttttttcc cgtccccaaa tggtgtttgg tgcactctac caaaaaatag 68700

agcgcctaaa tatgctatat aacgcctccc agcaaaatac ggttcaaagg cattacccga 68760

tattgtattg tagtacaggg caatgggaat tgatgatccc aataaacggc atagacgcac 68820

agcgccgtta tagcaggggt ctccagagta cagggtatct aagtaccggg atatctcata 68880

ctcatgcctt tccgtgacag aaacatcaac cggaacagta tccgataaac caactcctgt 68940

ttttgcaagg cgtaaaattc gcacaccttc cttttttgca agatgtgacg tttccttgta 69000

acagggaagc tgggggagtg gtaagaacaa caaagtttca gccaacgtgc caataaagcc 69060

cacttccctc aagaggctgt ttgctgtatc cacaatggtc cgtattaaat cttgagcaac 69120

ttgatccgtg tcatcatcac tgggtaacgc gttaacataa ctacgcgtta aatcttcaat 69180

aacggcataa caattaaacg cttcccaccg agacagtata tattgaacaa tcacgaaccg 69240

ttgacaggac gtcagatcac gtccgtaagc atgcccgaaa aatggaagtt ccccccgttc 69300

gccatatacc gcaacaactg cggtatatat cgtctcacgg gcttcattaa gttcatcttc 69360

aagtccaggc cattttctgg ctttaaatat aacctcgtcc gcaaaaaaaa ccgcacatga 69420

taacgcgcgg atacaatgag tagtggcttt atggcgagga tcccaaatgt ccattacccg 69480

ggggatggtc ctaatctgta caaagttact tagtgtaata tgatcggact tcttacgccg 69540

tctaggctgt ttctcagaat acggttcacc cgaaatcggc acatcatctg cttttacgtc 69600

ttccgtaacc acatcagcag cgcgccgact aacaattata cttgtttttt catcgtcgtt 69660

acttccgtta agcgcgtctc gtatctcggg cgtcccgtcg aataatccac tcactagctc 69720

ctgcaaacct tctggtaact ccaacatacg catatacacc aatgaaaaac tggcttcgtt 69780

tggtacgtac ataaagccat ttgtggtatt aatggcggtg ggtgttggaa acaattttag 69840

cttattctcg cgcgtaacat ctacccccgc caccaatgtt aaatgcgtca cggggaggga 69900

cacgagataa tctgcgagcg tagggtcctc cacttcaaca tcaaatgttc cgcaaaggtc 69960

gcgatccacc gcccccgatc ccgctgcaag taaggccact cgatccaaaa acacgcagtt 70020

attattggat gataccgccc atgtcttccc ggtgcgattg agctcacttc gaacgtaact 70080

ggcaacagat ctgtcaccgg gtccgacccc gcgaacaaca tgtccaaatt ttgcgatctc 70140

gcctccatgt ttgcggggta tggaaattaa gcatcccccg catataaaat acgccctggt 70200

agcacgctcg ttaaaataaa acgttacgcc gttataagat acggttgaat gatatggaaa 70260

ttccatatta aagcgtttat cggaacatta acctcgaact tgccgtcccg tgatcgtgtg 70320

atcgccaacc ttaggtccac accgaatatg agaaatatat aactacacgc aaacattcaa 70380

aacaccgtgg tatcattaac gtcatatgaa aagatccaat caatccaatc aaccacacct 70440

cctaccgttt agcacgtcag ctatgtgaca tgctccaaac atacgtaaac atttagagag 70500

ggtgttataa cagtctgtca ggcggggtat attctacata atacaaggat cggctttaac 70560

tttgtcaaca tttttacttt ggactataaa ctgcgactga acgttatgaa cccaccccaa 70620

gcccgcgtct cggaacagac aaaggacttg cttagcgtta tggttaacca gcaccccgaa 70680

gaggacgcaa aagtgtgtaa atccagtgat aattcaccgc tttataacac catggttatg 70740

ttatcgtatg ggggtgatac ggacttacta ttaagctctg catgtacccg cacatctacc 70800

gtaaacaggt cggcgtttac gcaacactcc gtgttttata ttatatccac ggtgttgatt 70860

caaccaatat gttgtatctt cttttttttt tactataaag cgacacgctg tatgctctta 70920

ttcacagccg ggttacttct gacgattcta catcactttc gacttattat tatgttattg 70980

tgtgtctaca gaaatatacg atcagacctg ctacccttat ctacatccca gcaactgctg 71040

cttggaatta ttgttgtgac tcgaacaatg ctattttgta ttacggcgta ttatactctt 71100

tttatagaca cccgggtgtt ctttttgatt accggacact tgcaaagtga ggttattttt 71160

ccagatagcg tttcaaaaat acttcctgtg tcgtggggtc caagtccagc cgtgttactg 71220

gtaacggcgg cagttattta cgctatggac tgtttggtgg acacggtatc ctttattggg 71280

ccaagggtgt gggtccgtgt tatgttaaaa acatctattt cgttttagtc catttcaata 71340

aatgtactat aattgttcag tctaaaaata atgttgggta tttataatta ccgcccccgt 71400

gttacttgga aacacccata catatgttcc actctacatc aaacttctcg cagttttctt 71460

gttcccgcac acgtttacac gtccggattc aagtcgcaac gctgctgaca aaatgacaac 71520

ggtttcatgt cccgctaacg tgattactac aacggaatct gatcgtattg ctgggttatt 71580

taacatccca gcggggatca ttccaactgg aaatgtgctg tcaaccatag aggtgtgtgc 71640

acaccgttgc atttttgatt tttttaaaca aatacgatca gatgataaca gcctttactc 71700

ggctcaattc gatattcttt tggggacata ctgcaataca ttaaactttg tgcgttttct 71760

agaacttgga ctgtctgtcg cttgcatctg tactaaattt ccggagctgg cttacgtgcg 71820

agatggcgtt attcaatttg aggtacaaca acccatgata gcacgtgatg gcccacatcc 71880

cgtcgatcag cctgttcata attatatggt taagcggata cacaagcgtt cgttaagcgc 71940

tgcgtttgca attgcatcgg aagcgttgag tttgttaagt aacacatatg tcgatgggac 72000

agagattgac tcatcgttac gtataagagc tatccaacag atggctcgta atttacgcac 72060

cgttttggac tcatttgaac gaggcactgc cgatcaactt cttggtgttc tattggagaa 72120

agccccaccg ctatcgctgc tttcaccaat taataaattc caacccgagg gacatctaaa 72180

tcgtgttgca cgcgcggccc tactttcgga cctcaaacgt agagtctgtg cggatatgtt 72240

ttttatgacc cgacacgcca gggaacctag gctgatctct gcgtatctgt cggatatggt 72300

ttcgtgcacc caaccatcgg tgatggtatc acgaataact catacaaaca ctcgcggacg 72360

gcaggttgac ggtgtgttgg taacaacagc aaccttaaaa cggcaactat tacagggaat 72420

tttacaaatt gacgacaccg ccgctgacgt accagtaaca tatggcgaaa tggttctaca 72480

ggggacaaac ttggtaaccg cccttgtgat gggaaaggcc gtccgcggaa tggatgatgt 72540

agcccgccat ctccttgata taaccgaccc taacacgtta aacataccgt ctataccccc 72600

acaatccaac tccgattcaa cgacagctgg gcttccggtt aacgcccgtg ttcctgcgga 72660

tttagtgatt gttggggata aacttgtatt cttagaagca ttagaacggc gggtctacca 72720

agctacgcgc gttgcctacc ctcttattgg aaatatagat attacgttta tcatgccaat 72780

gggagtgttt caggcaaact ccatggacag atatacacga cacgccggcg atttttcaac 72840

tgtatccgaa caggatccac gtcaatttcc accccaaggg attttttttt ataataaaga 72900

tgggatatta acacagttga ctcttcgtga tgcaatgggt accatctgcc acagttcatt 72960

gcttgatgtt gaggccacac ttgttgccct ccgccaacaa catttagatc gtcagtgtta 73020

ttttggtgta tacgtggccg agggtacaga ggacacattg gatgttcaaa tggggaggtt 73080

tatggaaacg tgggcagata tgatgcctca tcaccctcat tgggtaaacg aacatttaac 73140

aattctacag tttatagctc cgagcaaccc gcgtctaagg tttgaattaa accccgcctt 73200

tgattttttt gttgcaccgg gggacgtaga ccttcccgga ccgcagcgtc ccccggaagc 73260

catgccaacc gttaacgcaa cattacggat tatcaacgga aacattcccg tgcctctatg 73320

tcccatttca tttcgagact gtcgcggaac ccaactcggt ttgggaagac atacaatgac 73380

cccggcaacc attaaagccg taaaggatac atttgaagac cgcgcatacc caactatttt 73440

ctacatgcta gaggctgtta ttcatggaaa cgaaagaaac ttctgtgcgt tactgcgact 73500

gttaacacag tgtattcgcg ggtattggga gcaatcccac agggtggcat ttgtaaataa 73560

ctttcacatg ttaatgtaca taactacata tctcggaaac ggtgagcttc ccgaagtctg 73620

tattaatata tatcgggatt tactgcagca tgtaagagca ttacgccaaa ctataaccga 73680

ttttacaata caaggagagg gccataacgg cgagacctcg gaagcgctaa ataacatcct 73740

tacggatgac acgtttattg cacctattct atgggattgt gatgcgttaa tataccgtga 73800

tgaagccgcc cgagaccgac tccccgcaat tcgtgtaagc gggcgaaacg gataccaagc 73860

ccttcacttt gtggatatgg ccgggcataa cttccaacga cgcgataatg tgttaatcca 73920

cgggagaccc gttcggggag acacgggtca gggtattccc attaccccac accatgaccg 73980

tgaatggggt attctctcca agatttacta ctatattgtc attcctgcat tttcccgcgg 74040

ttcctgttgt acaatgggcg tgcgttatga tcgcctatac cctgcgttac aggcagttat 74100

cgttccggaa attcccgctg atgaagaagc ccaactaccc ccagaagatc caagacaccc 74160

tcttcacgca caccaactcg ttccgaactc tcttaacgtt tacttccata atgcacacct 74220

aaccgttgat ggtgatgcat tgctcacact acaagagtta atgggagata tggctgaacg 74280

aacgacggcc attttagtat caagcgcccc cgatgcggga gccgccacgg caacaaccag 74340

aaatatgaga atatatgacg gagcgcttta ccatggcctt attatgatgg catatcaggc 74400

gtacgatgaa accattgcaa cgggtacttt tttttatccc gttccggtca accctctgtt 74460

tgcatgtccg gaacatttgg catcattgcg tggaatgaca aatgctaggc gggttttggc 74520

aaaaatggta ccaccaatcc ctccttttct gggagccaac caccacgcaa ctatacgcca 74580

acccgttgcc taccatgtaa cgcatagtaa gtcggatttt aatactctta catattctct 74640

tcttggaggg tattttaagt ttacaccaat atctcttaca catcaactac gaacggattt 74700

tcaccccggg attgccttta ccgtagtgcg ccaggatcgc tttgccacag agcaactttt 74760

atatgccgag cgtgcttctg aatcgtactt tgtcggacaa atccaagtac accatcatga 74820

tgctattggg ggggtaaact ttaccctaac ccaacccaga gctcacgtgg acctgggagt 74880

cgggtataca gctgtatgtg ccacagcagc cctgcgatgc cctctcacgg atatgggcaa 74940

tactgcccaa aatctttttt tttcacgagg aggagtgcca atgttacatg ataacgttac 75000

cgaatcgttg cgtcgtataa cagcatcggg gggtcgctta aatcccaccg aacccctacc 75060

catcttcggc ggactacgtc ctgctacatc ggcaggaatt gcacgagggc aagcctctgt 75120

gtgtgagttt gtggccatgc cggtgtccac tgacctacaa tattttagaa ctgcatgcaa 75180

tcctagaggt cgagcatctg gaatgttata tatgggtgac cgtgacgccg acatagaggc 75240

tataatgttt gatcacacac aatcggatgt tgcttataca gatcgagcaa ctcttaaccc 75300

atgggcatca caaaaacatt catacggtga caggctatac aacggaacat acaaccttac 75360

aggcgcttct cctatctaca gcccatgctt taagtttttt acaccagcgg aggttaacac 75420

taattgtaat acactggatc ggcttctaat ggaggcaaag gctgtggcgt cgcaaagctc 75480

caccgacact gaatatcaat ttaaacgccc tcccggttct accgaaatga cacaggatcc 75540

gtgtggcctt tttcaagaag catatccacc actatgctca agcgatgcgg ccatgttacg 75600

aacggctcac gcgggagaaa ccggggcaga tgaagttcac ttagcccaat atctgattcg 75660

agacgcgtcg ccccttaggg gatgtcttcc tcttccgcga taatttcacc acgcccacat 75720

acccactccc aataaaagcc ctgtagagcg cattggcatc ttacttgaga tttggatacg 75780

ctcggccgac ttggtctgtt tcacgcttcc ttaaacaaca tggctatgcc atttgagata 75840

gaggtattgt taccaggaga actatccccg gcggaaacat ctgcattaca gaaatgtgag 75900

ggaaaaatta ttaccttctc aaccctgcgt catcgagctt cactggtgga tatagcgctg 75960

tcgtcatatt acattaacgg tgctccacca gacacgctct cgctgttaga ggcataccga 76020

atgcgattcg cggcagttat aacacgggtc atcccgggaa agttgttggc gcatgccatt 76080

ggcgtgggta ctcctacacc cgggttgttt attcaaaata catcccccgt tgatctttgt 76140

aatggcgatt acatctgctt acttcctccg gttttcgggt ccgcagactc aattcgcttg 76200

gactctgtag gactggaaat tgttttccct ttaaccatcc cccagacctt aatgcgagaa 76260

atcatcgcca aagtggttgc acgggccgtt gagcgcacgg ccgcgggtgc tcaaatttta 76320

ccccacgaag ttctacgagg cgcggatgtc atttgttaca atggaaggcg ttatgaactc 76380

gaaacaaatt tacaacatcg ggacggatcg gatgcggcta ttcgcacatt ggttttaaat 76440

ctaatgtttt ccataaacga gggatgtctg cttttattgg cgctgattcc aactttgtta 76500

gttcaaggag cacacgacgg ttatgtaaat ttattgatac aaacggccaa ttgcgttaga 76560

gaaaccggcc agttaattaa tataccgcca atgccgcgga ttcaagacgg ccatcgccga 76620

tttcccatat atgaaactat ttcatcttgg atatcaacat catctagact gggggatacc 76680

ttgggaactc gcgcaatttt acgcgtctgt gtgtttgatg gaccctctac tgttcatccg 76740

ggagaccgca cggccgtgat tcaagtgtaa acaggtgtta ataaaaacac aaccagtcta 76800

gttacatttc acgcgtcttg tttttattta ataggcataa acacggaatc cggtatacat 76860

gaactgccaa tatacacgga cataattaat gcaaccatca gatcatctga cattgttccc 76920

gtggtacctt tacccgtgta agtttttgtg tctagattac ccataccgcc tttaattacc 76980

tctgtcaggt tatccaactg tttacataga tactccacgg ggtctacacc taactttact 77040

gttagggata caagctcctg tgaggctatt atatttccgg agttaaatcg tttaacaaaa 77100

tagtctacgg ccggcgtttt ttgtttttgt aataaaaaaa aagggtacgc cacgctacat 77160

ccgggaggta tggaatgata aaacagtaac actggagcgg aagatagcac gtttcccttt 77220

tcgaggacag caaactgttg tgctatagcc aacgatatgg caactgcaga atcctggctg 77280

ctgtttccct ctatagaaac gtgtacgttt gtaaatgtat tggggtgtaa agcgagtatg 77340

tggcctaagc attgagtaac gcaacgccct atctcactgg aagacgtgcc agttaaagct 77400

ctaagaaaaa agtgctccaa tccaaatata atccaatccg acttataacg accaacaatc 77460

gctacaccag taccagacgc tcgtgtattt gaggtaaatg cagggtctac gtaaacgtac 77520

aacactgacg ataatatagc acaattcgca acggttgacg gccgatataa aataaacctc 77580

tcacgggcag tttttgtaaa taatggccgg tcaaacccca cacccccaga attctgttta 77640

cgcccaccta caatttcctg cacgaaggag tcggccataa ataaatctgc agtgcgccgc 77700

atggctccat ccattgtgat gaaaaccggc ttatttaata cataacacga acaagctgtg 77760

acatcgctat gtgctaaaac acgcggcatg tgatcgtcgc atacatatgt aacaacgttt 77820

aacaactgat ccgacgatcc acgtaagtta tacaaaaaac ttgtacttgc ttttccggta 77880

tttgttgatg aaacaaaaat aattttacaa ttggtttgat ttaaaaatcc gactatagtt 77940

tgtacagcat caggtcgaat aaaattagct tcatccacaa acagaagatt aaaatcttga 78000

cctcggatac cctggaacga tagaaagata tatagttacc ccaccaaagt ttaaatgtat 78060

ccttaaatac cacgtacgta aaaaatgttt gaatacgtac atatttcttt ttttttccag 78120

tacaaccata tccggtgtat aatggaagcc catttggcaa atgaaaccaa acatgcactt 78180

tggcataatg atcacacaaa aggattacta cacgttgtga tacctaacgc ggggcttatt 78240

gcggccggaa tagatcccgc attactgatt ttaaagaaac ccggacaacg cttcaaggtt 78300

gaagtacaaa caagatatca tgctacaggt caatgcgaac cgtggtgtca agttttcgcc 78360

gcgtacattc ccgataacgc cttaacaaat ctcttaatac caaaaacgga accatttgtt 78420

tcacacgttt tttcggccac gcataattca gggggattga ttttatcatt gcctgtttat 78480

cttagccccg gtttattctt tgatgcattt aacgttgtag cgatacgaat aaatactgga 78540

aaccgcaagc accgtgatat ttgtattatg tatgcagaac taatcccaaa cggaacgcgt 78600

tattttgctg atggacaacg ggtactttta ttatgcaaac agctgattgc gtatatccga 78660

tgcacccctc gtcttgcatc gtctataaaa atatacgcag agcatatggt ggcagccatg 78720

ggtgaatcac acacgtcaaa tggggacaat attggacccg tttcatccat aatcgatctt 78780

gatcgacagt taacttctgg aggtattgat gactcccctg ctgaaacacg catacaggaa 78840

aataatcggg acgtccttga gctaataaaa cgggccgtaa acattgttaa ctccaggcac 78900

cccgtccgac cttctagttc ccgcgttgca tctgggttgc ttcaaagtgc aaagggccac 78960

ggagcgcaaa cttccaacac agatccgatc aataacggtt cctttgatgg cgtccttgag 79020

ccgcctggac aagggcgatt tacgggaaag aaaaacaatt cgtccgccag catcccacct 79080

ttacaagacg ttctattgtt taccccagct tcgacagaac cccaaagtct tatggaatgg 79140

ttcgacatct gttatgccca attagttagc ggggacactc cagcagattt ctggaaacgg 79200

cgtcccctat caattgtacc gcgacattac gcagaatccc ccagtccgtt gattgtagta 79260

tcttacaacg gatcctctgc ctggggagga cgtattaccg gaagtccaat tttatatcac 79320

tctgcacagg ctattattga tgctgcgtgt ataaatgccc gggttgacaa tccccaaagc 79380

ctacatgtga cagctcgcca agagctagtc gcgcgtttac cgtttttggc taacgtccta 79440

aataatcaaa cccccttacc cgcctttaaa ccaggcgccg aaatgttttt aaaccaggta 79500

tttaaacaag cgtgtgtgac atcgctaacc caaggtctta taacggagtt acaaacgaac 79560

ccgactctac aacaactcat ggaatatgat attgcagatt cttcccaaac ggttattgat 79620

gaaattgtag cccgcacacc agacctgatt cagactatag tttcggtgtt aacggaaatg 79680

tcaatggatg cgttttataa cagctccttg atgtatgcgg ttttggcgta tctgtcatct 79740

gtatatacac gaccacaagg tggggggtat ataccctacc ttcacgcttc cttcccatgc 79800

tggttaggta atcgttctat atatttattt gactattata attcaggagg ggaaatactt 79860

aagctttcca aggtccccgt tcccgtagcc ttagaaaagg ttggtattgg taattccaca 79920

caactgaggg gtaaatttat acgcagcgcg gatattgttg atattggaat ttgttctaag 79980

tatttacccg gtcaatgtta cgcgtacatt tgtctaggat ttaaccagca attacaatcc 80040

attttagttt taccgggggg atttgcggca tgtttttgta ttaccgatac cctacaggca 80100

gcactacctg catcgttaat cggacctatt ctagacagat tctgcttctc tattcccaac 80160

ccccataaat aaattagtgt cactataaaa acataacacc agaatctctt catatttaat 80220

tttacgtcat ttctcccgtt tccaccccct cttaaaatat aaaataaccg ggtgggtggc 80280

attaaaccca caagtacccg ggcggcaatc cgctagactg tttttctgct catggaatta 80340

caacgcatat ttccgctgta caccgctacg ggtgcagcgc gcaaattaac ccccgaggca 80400

gttcagagac tctgcgatgc attaacgctg gatatgggat tatggaagtc catcctgacc 80460

gatccccggg tgaaaataat gcgatcaact gcttttataa ctttaaggat cgctccgttt 80520

atcccccttc aaacggatac tactaatatt gccgttgttg tagccacaat ttacatcacg 80580

cgcccacgtc agatgaactt acctccgaag acttttcatg taattgtaaa ttttaattac 80640

gaggtctcgt acgcaatgac ggcgacttta agaatttatc cggttgaaaa catagaccat 80700

gtttttggag caacgtttaa gaacccgatc gcgtaccccc ttccaacatc tattccggat 80760

cctcgagcag atcccacccc cgcagatctt acaccaacgc caaacttaag cgactactta 80820

caacccccgc ggcttccgaa aaatccatac gcatgtaaag ttatttctcc gggagtgtgg 80880

tggtcagacg aacgaaggcg tttatatgta ctggctatgg aacctaattt aatagggcta 80940

tgtcccgccg gatggcatgc tcggatactt ggctctgtat taaatcgact cctcagccat 81000

gcggacggat gtgatgaatg taatcataga gttcacgtgg gggcactgta tgcgttaccc 81060

catgtcacaa atcatgcgga aggttgtgtg tgttgggctc cgtgtatgtg gagaaaggcc 81120

ggtcagcggg aattaaaagt ggaggtagac attggcgcta cgcaggttct ttttgtagat 81180

gtcaccacct gcattcgaat tacgagtact aaaaatcctc gcattaccgc aaatcttggc 81240

gacgttatag cgggaaccaa cgccagtggt ctctctgtac cagtaaattc atctgggtgg 81300

cagctttata tgtttggaga aacattaagc cgggctatta ttaacggctg tggtctgctt 81360

cagcgaattt gcttccccga gacacaaaga ttatcgggtg aaccggaacc tacaaccacc 81420

tagtatacct taactcaacc gccgttgtgg aaaggtatat gtcaacattt acagtaatat 81480

attaaaggtt aaatttataa aacactcacg tttgtgttgt gacttgacgc gaacaccgct 81540

gtgctgtaag acccgtcggt aaatgaaaac gtaatagatt cgccttttac atgatccacg 81600

taatttgccc caaaccactg ttccaggcga gacttgatac cctcaaacac gggttccgtt 81660

gctttgcgta tatgagccgt ataacccact ttaattcctc taaacgtggc cattactaaa 81720

gctattaatg gtacaagaaa ccatgttttc ccatgtctac gtggtaccaa aaacacagtt 81780

gatttttgtt tgaagtgttc taaaacactg tcagaaacac ttggcgtgtt aaacactgta 81840

cgcagaaagc agtcaactct gtcggcatga tcgcccaata gcaccgatga aataaaatgc 81900

gtggtgtgca tgaggatcat tttttgaaac agttccaacg tccccttata tctgccatag 81960

attggaacgt caacctttgc gcgtttgcca tgacttccac actcttcaat actctcaaaa 82020

gatgtttcca caaggtacga aaaccgttgt gtaaaggtag acaactgaca gaaactatcc 82080

gacagagaaa acgcgcgaaa tgtgttcata acaccgctat acgcatttcg atgaggtgct 82140

gcttcttccg gtgaatattc ataaaactgt acactactga cagccttttt taattcgggg 82200

cttacgtttg catttaccga atatcgccat ggtttcaaaa ctacattggg ggtacagttg 82260

taccctgttg acgatagaaa cgcgccaaac attgcccgtc gagcagtagc cgagaacagt 82320

ggaatatatt cacaacagtt gtgaagcgtt ccaattccgg gaataacggc ctgatgacgt 82380

cgggttacat ctatagcaaa attcagaaac gggatttggg ttgcgtttcc cagagaccct 82440

tgccgcgtgg aacacggggt aggggactcc aacgtcccaa agcgttcatc cctacgacgc 82500

tttagacgtt caaaatatct tacagattct tcaccaagcg tacgaccaaa cattatcaat 82560

gacatttaac atcaattcac ggaatccgcc tcatctcttg taagcagtaa aacaggaagc 82620

cgcgtcatct tacgtactcg ttacgtatat atcataaaca ttttcagggc cgcattcatt 82680

cactttggtc atgtcaggcc acactccaac ctacgcttct cataggcgta accgtgtcaa 82740

actagttgag gcgcataacc gcgcggggtt atttaaagaa cggaccctcg atctaatccg 82800

tgggggtgcg agtgtacaag atccagcatt tgtgtatgcc tttactgctg caaaagaggc 82860

ctgcgccgat ttaaataacc agctccgctc tgcagctcgc atagcttcag ttgaacagaa 82920

gattcgtgat atacaatcca aggttgagga acaaacaagt attcaacaga ttttaaatac 82980

aaacagacgc tatatagcac ccgattttat tcgcggtttg gataaaacag aagacgataa 83040

taccgataat atagacagac tggaagacgc ggtaggaccg aacatcgaac acgaaaatca 83100

tacttggttt ggagaagacg acgaagcgtt acttacacaa tggatgctga cgacacaccc 83160

cccaacctcc aaatatctcc aactgcagga cctttgcgtt cccaccacaa taccgacgga 83220

catgaaccaa atgcaaccgc agccgatcag caagaacgag aatccaccaa ccccacacac 83280

ggatgtgtaa atcatccatg ggccaatccg tcaactgcaa catgcatgga atcaccagaa 83340

cgatcacaac agacaagctt atttttatta aagcacggct taacgagaga tccaatacat 83400

caacgcgaaa gggtggacgt ttttccacaa tttaacaaac ccccatgggt ttttagaatt 83460

tccaaattat cccgtttaat tgtacccatc ttcacgctca atgaacagtt atgtttttct 83520

aaattacaga ttcgagatag acccaggttt gcgggacggg gaacgtatgg gcgtgttcat 83580

atatacccat cgtcaaaaat agctgtaaaa accatggaca gtcgtgtttt taatagagag 83640

ttaattaacg cgattttagc gagtgagggt tctatacgag caggggaaag gctaggtatt 83700

tctagcatag tttgcctttt aggtttttcg ttacaaacca aacagctact gtttccggca 83760

tacgacatgg atatggatga atacattgtt cgcctgtcca gacggttgac aatacctgat 83820

cacatagaca gaaaaattgc ccatgtattt ttagatttgg ctcaagcgtt gacgttttta 83880

aatcgaacgt gcggcctgac ccacctagat gtgaaatgtg gcaatatttt tcttaacgtc 83940

gacaactttg cctcgttgga aataaccaca gcagtaatcg gagactatag cctagtaaca 84000

ttaaatacgt attccctttg tactcgagcg atatttgaag ttggaaatcc atcccacccg 84060

gagcacgtac tacgcgtacc ccgggatgca tcgcagatgt catttcgttt ggtgttgagt 84120

catggaacaa accaaccccc tgaaatcttg cttgattata ttaatggaac gggccttact 84180

aaatatactg gaaccttgcc ccaaagagtt ggacttgcga ttgatcttta tgcattgggc 84240

caagcactct tagaagttat cctgctagga cgtcttcccg gacaactgcc catttcagta 84300

catcggaccc cgcattatca ctactacggt cataagttat caccagattt ggcgcttgat 84360

acgctggcat atcgatgtgt cctggcgcca tatatactcc catctgacat ccccggggac 84420

ttaaattata atccctttat acacgccgga gagctgaaca cccgtatttc ccggaattct 84480

ttacgccgga tattccagtg tcacgcagtg cgttacggcg taacgcactc aaagcttttc 84540

gaaggcatac gcattccggc ctcattatac ccagccactg ttgttacgtc gttgttgtgt 84600

cacgataatt cagaaatacg ctcggatcac cctttattat ggcacgatcg ggattggata 84660

ggatcgacat aagcccccag ccagccaaaa aaattgcccg tgtgggaggt ctacagcacc 84720

cttttgtaaa aacggatatt aacacgatta acgttgaaca ccattttata gacacgctac 84780

agaagacatc accgaacatg gactgtcgcg ggatgacagc gggtattttt attcgtttat 84840

cccacatgta taaaattcta acaactctgg agtctccaaa tgatgtaacc tacacaacac 84900

ccggttctac caacgcactg ttctttaaga cgtccacaca gcctcaggag ccgcatccgg 84960

aagagttagc atccaaatta acccaagacg acattaaacg tattctatta acaatagaat 85020

cggagactcg tggtcagggc gacaatgcca tttggacact actcagacga aatttaatca 85080

ccgcatcaac tcttaaatgg agtgtatctg gacccgtcat tccacctcag tggttttacc 85140

accataacac tacagacaca tacggtgatg cggcggcaat ggcgtttgga aaaaccaacg 85200

aaccggcggc acgagcgata gttgaagcat tgtttataga tccggctgat atccgtactc 85260

ctgatcattt aacgccagaa gctacaacta agttttttaa ttttgacatg ctcaatacca 85320

aatctccaag tctccttgtg ggtacaccaa gaatcggaac gtatgaatgt ggacttttaa 85380

tcgacgttcg aacgggactt ataggcgcgt cgttggacgt tcttgtatgt gacagggacc 85440

ctttaactgg caccctaaat ccccaccctg cagaaaccga catttcattt tttgaaatta 85500

aatgtcgtgc taaatacctc tttgatccag atgataaaaa taacccgctc ggtcggacgt 85560

acaccacgtt aataaataga cctacaatgg caaatctacg ggacttttta tatactataa 85620

aaaacccatg tgtaagcttc tttggaccct cagcaaaccc aagtacacgc gaggccttaa 85680

taacggatca cgttgaatgg aaacgtttag gatttaaagg tgggagggcc cttacagaac 85740

tcgacgccca tcatttgggc ctcaatcgga caatctcatc ccgagtgtgg gtatttaatg 85800

atccggacat acaaaagggg acaattacaa ccattgcatg ggccactgga gatacggctc 85860

ttcaaattcc tgtatttgcc aatccgcggc acgctaactt taaacaaatt gccgtacaaa 85920

cctatgtatt atccggttac tttccagcgc taaaactacg gcccttcctt gtcaccttta 85980

taggacgtgt gcgccgacca cacgaggtgg gagtcccatt gcgcgtcgat acacaagcgg 86040

ctgccattta cgaatataac tggccgacta tcccacccca ctgtgcggtt ccggttatag 86100

ccgttctaac gcctatcgaa gttgatgtgc ctagagtgac aaaaatactt aaagacacag 86160

gaaacaacgc gattacatca gcattgcggt cattgcgatg ggacaatctt catccagcgg 86220

tcgaggagga atctgtggat tgtgcaaacg gtacaacgag cttgttacgt gcaacggaga 86280

aaccgttgct ttgaactcag agttctttga agactttgac tttgatgaga atgtaacaga 86340

ggacgccgat aaatccacac aacgccgccc acgagtgatc gatgtaacac caaaacgaaa 86400

accttcggga aagagctccc attccaaatg cgcaaaatgt taaaccctga taaacgttct 86460

aataaaaaca tcaaatcatg gttggttact gtgaatgttt gttttattgc ttggggggtt 86520

acaagtacaa cccacgctac tcccacccac tgtttgatcg ctcgtataac agctcatcct 86580

cgcggtccgt ttcatatgtt gagtcatttt catagacgta gccgtagcct tgtgatgggt 86640

aatttgtgcg gcgagaattt ctatgtgcag gttttacttt tcgtatgtat ccccgtaccc 86700

gctcgggtac tcttcttacg gcaccgtaga accgactgcg tttctgtcga tgatacacat 86760

atgcacgcat caatctgaga agcaacatga caacggaaaa cacggccagg caagccaagg 86820

ttccccgagt tgtgggaatt aaccgtggag attgaaccga tatagggtca tataatcggt 86880

ccatatacga gtgcgcggcg gttcccaacg tagcacaggc cacgagcgtt cccagggacg 86940

gtcctattaa cacgtgtata taatgcgcca aaattaattc tgatactata agatatacaa 87000

ctgacaatgt actaaatgta gacatggcca cggacaccga tgaccacagt cccgtatgta 87060

gatgattcgc caccacaagt tccagcatta atgatacaaa taggatacat atcgccatca 87120

acgcagccat caaattcacg aacactgcgc gcgtaggccc cgcaaggcga tataaaaaga 87180

cgctctgctg tcgtaaattt gcgaccgctt ttatgttcgt ttcgtccaat tttccgcgtc 87240

cacaaaaata cgttgtaaat attacacttg tcgcaaaatg tccaagatat aatgtagcag 87300

ccacgccgat ttgcttgtaa gctaataata acacaacggc gtttaataac cacaatgaca 87360

aaagacccca aaaaagtgtt gtgggatcta caactaacca tgcaacaccg gagctttgcc 87420

ggacacgttg atttttcgtt tctcggtgta taatcgcggc cgtgatcagt gtatataccg 87480

ccatggccat tgccgttaaa gccgtgtagt aagtaaatgc cacaacgcta tgtggttcca 87540

aaaacaaaac cggggcgctg tatccacctc tatttccgga ccataccccc ccatctaggg 87600

tggcgttaaa taactcataa tcaactacgg cagcataaaa acaaggatcc ccggtatatt 87660

cagaagaggc ggcaattaac gtagccagga gcattaccgc acccaaagtg aacatcatca 87720

cctgaattat ccaaattcgc caattaagcg tatccatttg atgatctaac gcttccacct 87780

cgggtgtcgt ggtgtcgtac ggtgagactt tttcagaacg cggccccttc ttttgagttc 87840

ccatgtctcc caacaccggg gagagcaacg ccgccgtcta tgcgtccagt acacagctcg 87900

cgcgggcgtt atatggaggg gatctggttt cgtggattaa acacacccac ccgggaatta 87960

gcctggaact gcaattggat gttccagtaa aactaataaa acctggtatg tcacaaactc 88020

gcccggtaac cgtcgtacgt gcccctatgg gctctggtaa aacaacagcc ttgcttgagt 88080

ggcttcaaca cgcgttaaag gcagatatta gcgtactggt tgtctcatgt cgccgtagct 88140

ttacccagac gttgattcaa cggtttaacg atgcaggcct ctccggattc gtaacatatt 88200

tgacatccga gacatatatt atgggtttta aacgtttgat tgtgcaactt gaaagcctac 88260

accgcgtatc cagcgaagct atcgacagct acgacgtatt aatactggat gaggtaatgt 88320

cagtgattgg acaattatac tcccccacaa tgagacgtct ttccgcggtt gatagcctat 88380

tatatcgtct tttaaatcgc tgttctcaaa ttatcgcgat ggatgctaca gtaaactctc 88440

agtttattga tttaatctcc ggattgcgtg gagatgaaaa catacacaca attgtgtgta 88500

catacgcggg agttgggttc tccggaagaa cttgcacgat cctgcgtgat atgggcatcg 88560

acacgcttgt gcgagtcatt aaacgatctc ctgaacacga ggatgtacgt accatacacc 88620

aactacgtgg aacatttttt gacgaactag cactacgatt acaatgtggg cataacatct 88680

gtatattttc atcaacttta tcgttttcgg agctagttgc tcagttttgt gcaatattta 88740

cagactctat tcttatttta aactcaactc ggcccctatg taatgtaaac gaatggaaac 88800

attttcgcgt gttggtgtac actaccgtcg tgaccgttgg attgagtttt gacatggctc 88860

attttcatag catgtttgct tacataaagc caatgtcata tgggccggat atggtatcgg 88920

tctaccagtc attagggcgt gtacgtttat tgctacttaa tgaagttttg atgtacgtcg 88980

atggctcaag gaccagatgc ggacccctgt tctcgccaat gttactaaac tttaccatcg 89040

caaataaatt tcaatggttt cctacacaca cccaaataac taacaaactg tgctgtgcat 89100

ttaggcaacg atgtgcaaat gcatttacac gctcgaacac ccatctcttc tcaagattta 89160

aatacaaaca ccttttcgag agatgctctc tttggagttt agccgatagc attaatatct 89220

tacaaactct tttggcctct aaccaaattt tggttgtatt ggatggcatg ggtccaataa 89280

cggacgtttc cccagttcaa ttttgtgcat ttatacacga tctcagacat agcgctaacg 89340

ccgtagcttc ctgtatgcgt tctcttagac aggacaatga cagctgcttg accgattttg 89400

gcccttccgg atttatggcc gataacatta ccgcgtttat ggaaaagtat cttatggagt 89460

caattaatac cgaagaacaa attaaagtat ttaaagccct tgcatgtcca atagaacagc 89520

ctagactagt caatacggca atattggggg cgtgtatacg aatacctgaa gcgttggaag 89580

catttgacgt atttcaaaaa atatacacgc actacgcttc cggttggttt cccgtcctgg 89640

acaaaaccgg ggaatttagc atcgcgacta taactaccgc cccaaattta accacgcatt 89700

gggagctgtt tcgccgttgt gcctatattg caaaaacact caagtggaat ccgtccaccg 89760

aaggctgtgt aacacaagtt ttggatacgg acattaatac acttttcaat caacacgggg 89820

attcgctggc tcaactaata tttgaggtta tgcgctgtaa cgttaccgac gctaagatta 89880

tattaaaccg cccggtttgg cgaacaaccg gattcttaga tggatgccat aatcaatgct 89940

tccgtccaat ccctacaaaa cacgaatata acattgctct atttcgttta atttgggaac 90000

aattatttgg cgcccgcgta actaaaagta cccagacctt tccgggaagt actcgtgtga 90060

aaaacctaaa aaaaaaagat ctagaaactt tacttgattc aattaacgtg gatcgttctg 90120

catgtcgtac ctaccgccag ttgtataacc tgcttatgag ccatcgccat tcgttctccc 90180

aacagcgtta caaaattact gcccccgctt gggcacgcca cgtgtatttt caagcacatc 90240

aaatgcactt ggccccgcat gccgaagcca tgctacaatt agcgctatcg gaactgtccc 90300

cgggatcgtg gccgcggata aacggggcgg taaattttga aagtttataa cccattaata 90360

ccatatatgg acatccatag ggggggttac ataaatacta agcctctgta caacacaaag 90420

ggcctctaac aatgcactga accacaacca agctatggac gcaacgcaga ttaccttggt 90480

tagagaaagc ggacacgttt gtgccgcaag catatacaca tcctggacac agtccggaca 90540

attaacacag aacggtcttt ccgtgttata ctacttatta tgcaaaaact catgtgggaa 90600

atacgtccct aagtttgccg aaattaccgt acaacaagag gatttatgtc gctactccag 90660

gcatgggggg agtgtttctg cggcaacgtt tgcgtctatc tgcagggcgg cgtcctcggc 90720

tgcgttagac gcctggcccc ttgaaccact gggtaacgca gacacctggc gttgtctcca 90780

tggcactgcc ctggccactt tacggcgcgt attagggttt aaatcgtttt attcgccagt 90840

aacattcgag actgatacga atacaggtct tctgttaaaa acaatccccg atgaacacgc 90900

gttgaataat gacaacacgc catctaccgg agtattgagg gctaattttc ccgtggccat 90960

tgatgtttca gcagtcagcg catgtaacgc ccacacgcaa ggtacgtcgc tagcctacgc 91020

ccgcctgacc gcacttaaat ctaacggtga cacccagcaa caaacacctt tagacgtgga 91080

ggtaattaca ccaaaggcct acatacgtcg gaaatataag tctacgtttt ccccccctat 91140

agagcgggaa ggtcaaacct ccgatttgtt taaccttgaa gaacgccgct tggttcttag 91200

tggcaatcgc gcaattgtgg taagggtact cttaccgtgt tattttgact gtttaacaac 91260

ggattccacc gttacatctt ccctttcaat attagcaaca tatagactgt ggtacgcggc 91320

ggcgtttgga aaacccgggg ttgtccgtcc aatctttgcg tatttaggcc cggaactcaa 91380

tccgaagggt gaagacagag actacttttg tactgtcgga tttcccggat ggaccactct 91440

tcggacacaa actccagccg tcgaatctat tcgcacggct acggagatgt acatggaaac 91500

ggatgggttg tggccagtaa ccggtattca ggcctttcat tatctagccc cctggggaca 91560

gcatcccccc ttacctccgc gggtgcagga tcttattggg caaatccctc aagatactgg 91620

acatgcagat gcaactgtca attgggacgc gggccggata tctaccgtct tcaaacagcc 91680

tgtacaacta caagatcgtt ggatggcaaa gtttgatttc agcgcctttt ttcccacgat 91740

atactgcgct atgttcccca tgcattttag attaggcaaa atcgtcctgg ctagaatgcg 91800

tcgaggaatg gggtgcctaa aacccgcgtt ggtgtctttt tttggggggt tacggcacat 91860

actcccgagt atatacaaag ctattatttt tatagccaat gaaattagcc tttgcgtcga 91920

acaaacggcc ttggaacagg gctttgctat atgtacttat ataaaagatg gattttgggg 91980

aatcttcgcc gatttacata cgcgcaatgt atgttcagat caggcacgtt gttcggcctt 92040

aaatttagcg gccgcctgcg aaagagcagt cacgggctta ttacgaattc aactaggtct 92100

taactttaca cccgccatgg aaccggtact ccgggtcgag ggtgtgtaca ctcacgcatt 92160

tacctggtgt accacgggaa gctggctgtg gaatttacaa acaaacacgc ctccggattt 92220

agttggcgtg ccatggcgaa gtcaggcggc gcgagattta aaggagcgtc tttcaggact 92280

cctatgtacc gcaacaaaaa ttcgagaacg gatacaggaa aattgcatat gggaccgtgt 92340

cctatacgac atatgggccg gacaagttgt ggaggctgcc agaaaaacat acgtcgattt 92400

ttttgaacat gtttttgatc gccgttatac tccggtatac tggagtcttc aggagcaaaa 92460

ttcggaaaca aaagcaatac cggcatctta tctgacatac ggacacatgc aagataagga 92520

ttataaacca agacagataa ttatggttcg taatcccaac ccacatggac ctcctactgt 92580

tgtttactgg gaattgctac catcgtgtgc ctgtattccc cccatagact gcgctgctca 92640

tctcaagccc cttatacaca cgtttgtcac tattattaac catcttctag atgctcataa 92700

tgatttttca agtccatcat tgaaatttac tgacgatccc cttgcttcat ataacttctt 92760

gtttttatga caaaaaaaca cgccgcaaca acccatcctt aaaataaaag gtttatttac 92820

tttacaaccc gtggtgaatt tttatacgtt tcaaataact gaacattttt cggtgttacc 92880

atggtgcgat ttaaccacca aaaatatacg ctcttctgat attccgaatc tcgtaaaggt 92940

ccatttaaca atcccggggg cacttgcacc acaccatctg gacagggggg ggttccgtgg 93000

ggcaggtcaa aacgctgacc caccccacat gaatatatag cctttataat attgggggcc 93060

gttccaggct gagggttcag taacttaaca aacatataat gcggcaatac gcgggttttt 93120

gtaaaggggt tgttatcaac gacatacatt agagtgttta acaaccataa aactccctca 93180

tataaaaacc gacgcatttt ttccaaaggt cctatttgac actcaacgcg tctaagatat 93240

acagacaatt gtacaaacag cgatggagat gccccggagg gcccaatgcc ttccagatac 93300

attaaaataa cacataaggt aaaatctagg acattatccg ggcggaatag agtcatccga 93360

tagattaaca ggcgcggagg cacccccacc gtatacaccc tatcttcaac cgcagttaat 93420

acggaaaaaa taaatccgcg gaacgctggt tgagtaacac actccatgta gtaacgatca 93480

caggacacct cacttgaatc accattcaac actactaaaa cggtctcttg gtgttccggt 93540

tttacgcgca gtgatacaac agagtttgcc aaaaagcgtg gcttcaaacc ggttacctcc 93600

cgcgcctcgc atacgaatct tggtattgct tgtattctaa gatcttcgat cacgtcgctc 93660

acatccaacc cctcttcggc tcgtgttagt aagttgtcga tcgttacgct gcaacctaaa 93720

atgctgggta tatttattcc ggacatccca tcggccatcc ccgcgcctcc ggtttgctcg 93780

aattttatcc agtaaggtcg aatccgctgc atttaccttg tgtacccgta acctctcagg 93840

ggggtgtcct ttcataaaat gggataggtt tttatatcca acatgcatgt attggttatt 93900

tattttattg ggttccggga ttctttcgtc atcttctgta gggtcaggca aaccccagga 93960

aggacttggt gttctccgtg ggccccgttt tattacctct gcgcgaacct gcatttcata 94020

taatattcgg atttgggata aataggactc tgttctcgcc tttttaaaaa tagcctggca 94080

taactcttcc tctgacctat gtacctcgct ttgagttacc aagaatccca atcgggtggc 94140

ccgtaatatg aatgaaaaat acggcgcaac tagtaatgag attgacgcat ttgaatatga 94200

tacagaaatt tcctggcctt gattattgtt tacccggtga agcttaaaac agcgaacaag 94260

ttcctgtttc catagctcag acaaacgttt tatatcatct ccataagggg ggatataacg 94320

agattgaaaa ctattggcaa tatatgcatc atcccctatt atgccggtaa gatctataac 94380

ctcgtgattt aaatcggcaa tacgtgtttc ttctgccatt gtaatatgtg accctttaga 94440

tggctttatt tttaccctct cttcccgtaa ccgtttcagc tctccttctt tgaactggag 94500

cctttcggtc agatcgctgt tcacatcctt gagaccctca atggttttga ataaattatt 94560

cacataaccc tcgagcatgc cgttgatact gttgaccacc gatgttttaa acgcactttg 94620

aacgtttgtt gttccggaca ttgccccccc gttaaaggat tggttggcct tgccaaaccc 94680

cggttgtgat gtgtccaccg atccacttcc ttccagaatg tgattgcccg tttcttctag 94740

ataggaacgt acggtttcgg taatatctcc aacatgtctc atgtttttta agttaactat 94800

tagctttaca agtctagacg cggccgatcc agcccgtgtt gtatcgttct cgcccattat 94860

acgatcaacc gcacgtgtgc tgtgagatct atcatcttca ttccggcgac ctattaacac 94920

gcgcaaaggg gctgtattta aaacttggca gacgcgagca tgttcacgta atgcataaca 94980

ggccaacacc tccccagaaa gccgctgtaa gggtgagtca aatactacac cctccccaca 95040

tacaacgggc ggccacacga ccaaacactc tcccttcatg cccgttacat catcctttgc 95100

cataattaat cttcggttat aattataata aagacgcgtc ctatcataat ccataatagc 95160

aacattttgc atacactcaa ctaggcttgt gacaaccgcc gcccctctgg ccaacgttgc 95220

atcggcaact tttaacatct gggacagttc tgccgcttga cccatatacg tatttaatgg 95280

tgcaggggtt ccattctgtt ctgatcgtac ctttcttaca acgggcacaa tacctacaca 95340

ggctatccag tccacgtatt tggcaaaacc gacccttcca tttaaaccac tggtatagag 95400

acaaccggtt attccacgca gaaactcaag taacgatgac tgtaatgttt gacgccaggt 95460

ttcaaaaacc tgatgtgcaa gccgtacggc ttctgattct ccacataacc cataacgttc 95520

cgctagagcc ccggcatgca ggttacattg ttggatgtgg tggtcccaat ctgctgctag 95580

gtcctcatac cgagttgcat ccaacgcgtt catcaaaacg gttgcctgaa cttggcgaat 95640

tacagtttcc gtagaccgta cagcgctata tatgccttgt ccatcggtat atccaaagtc 95700

accggctagg atttttcgaa acaacatact ttgcgtggtt gggtgtatta acatccagcc 95760

atcttcctcc ggaaatgtac aaaaccctat atccggggcg tactcattcc agtatatatc 95820

gaacatgttc ttgtattggt catttgggtt acttccattc aagccctggt caatagaaac 95880

agaacttgct atcctttttt cttcactacc ggaactgtta ttaaaaagag acgttatttc 95940

ggccattgaa aaccacgatg aaaagatcaa tttctgtaga cagttcttca cccaaaaacg 96000

tttttaatcc agagacgccc aatggatttg atgacagtgt atatttaaac ttcacctcta 96060

tgcatagcat tcaacctatc ctctcacgga ttcgagaact tgccgcaatt acgattccaa 96120

aagaacgtgt tccgcggttg tgttggttta aacagttact cgaactgcaa gcgcctcctg 96180

aaatgcagag gaatgagctc cccttctccg tttatttaat tagcggaaat gccggctccg 96240

gaaaaagcac gtgtatccaa acgcttaacg aagctatcga ttgcattatt accggatcca 96300

ccagggttgc tgcccaaaat gttcatgcta agttatcaac ggcttatgcg agtcgtccga 96360

taaacacaat ctttcatgaa tttggttttc gcggaaatca cattcaggct cagctgggcc 96420

gttacgcata taactggact acgacccccc cttctattga ggacctgcaa aaaagagata 96480

ttgtatacta ctgggaagtt ttaattgata taacaaaacg agtgtttcaa atgggggacg 96540

acggtcgcgg aggaacatcg acatttaaaa ccctgtgggc aattgaacgt ttgcttaata 96600

aacctacagg ctcaatgtcc ggaaccgcgt ttatcgcatg cggttccctt ccggctttta 96660

cccggagcaa cgttattgtt attgatgaag caggattgct agggcgtcat attctcacgg 96720

ccgttgttta ctgttggtgg cttttgaatg ctatatatca aagccctcag tacataaacg 96780

gtcgaaaacc ggtcatagta tgcgtcggtt cgcccaccca aactgactcg ttagaatctc 96840

attttcaaca tgacatgcag cgttcacacg taactcctag tgaaaatata ctcacgtata 96900

taatctgcaa tcaaactctg cgtcaatata ctaacatctc acataactgg gcaatcttta 96960

ttaataacaa acgatgtcaa gaggacgatt ttggaaatct tttaaaaacg cttgagtacg 97020

ggctacctat taccgaagca catgcgcgtc tggtcgatac atttgttgta cctgcatcct 97080

atattaacaa tcctgctaat ctccccggat ggacgcgtct gtattcgtcg cataaggagg 97140

tgagcgcgta tatgagtaag ttacacgcgc atttaaaact atcgaaaaat gaccattttt 97200

ctgtgtttgc cttaccgact tatacattca tccggctaac ggcatttgat gaataccgca 97260

aattaacggg acaacccgga ctttctgttg aacattggat acgggcaaac tccggtcgtt 97320

tgcacaatta ttcccaaagc cgagatcatg acatgggaac agttaaatac gaaacacatt 97380

caaatcgcga cttaattgta gcccgtacag acatcactta cgtgctaaat agtctcgtag 97440

ttgtaaccac aagactacgt aagttagtta ttggattcag tggtacattt caatcgtttg 97500

caaaggtttt acgtgacgac tcctttgtga aggctcgagg agagacatcc attgaatatg 97560

cttaccggtt tctgtcaaac ctaatctttg gaggcttgat taacttttac aattttttgt 97620

taaataaaaa cctacatccc gataaggtat cgttagcata caaacggtta gctgccttaa 97680

ccctggagtt attgtctgga acaaacaaag cccccttaca cgaagcagcg gttaatgggg 97740

cgggtgccgg gattgaccgt gatggtgcag ctacttctgc cgataaagcc ttctgcttta 97800

ccaaagcccc cgagtccaaa gtaacggcct ccatacccga agacccggat gatgtaattt 97860

ttacggcact taacgacgag gttattgact tggtatactg ccagtacgaa ttttcctatc 97920

ccaaatcatc caatgaggtc catgctcagt ttctgttaat gaaagctatt tacgatggtc 97980

gatatgccat attagcagag cttttcgaaa gcagctttac aaccgccccc tttagcgcgt 98040

atgtcgataa tgttaatttc aacggaagcg agcttttgat cggcaatgtg cggggggggc 98100

tgttatcttt ggcattacaa acagatacgt ataccctttt ggggtatact tttgcacccg 98160

tgccagtctt tgtagaggaa ctgacccgaa aaaagctgta ccgcgaaact accgaaatgt 98220

tatatgctct acacgtacct cttatggtct tacaggatca acatgggttt gtgtccatcg 98280

taaacgctaa cgtatgtgaa tttaccgagt ctatagagga tgcagaattg gcaatggcca 98340

ccacggtgga ctatggcctt agttctaaac tagccatgac aattgcacgc tcacagggcc 98400

tgagtttaga gaaggtagct atctgtttta cggcggataa actgcgccta aatagtgtgt 98460

atgttgccat gtcgcgtacg gtctcctcta ggttcttaaa aatgaatcta aaccctctac 98520

gggaacgata tgaaaaatcc gcagaaatta gcgatcacat tcttgccgct ctacgtgatc 98580

ccaacgtaca cgttgtgtat taaagcattg tataaaaaca cgcatgcggg cttgctgttc 98640

tcatttctag gttttgtctt aaatacaccc gccatgagca tctctggacc cccaacgacg 98700

tttattttat ataggttaca tggggtcagg cgggttcttc actggacttt accggatcat 98760

gaacaaaccc tctacgcatt tacgggtggg tcaagatcaa tggcggtgaa gacggacgct 98820

cgatgtgata caatgagcgg tggtatgatc gtccttcaac acacccatac agtgaccctg 98880

ctaaccatag actgttctac tgacttttca tcatacgcat ttacgcaccg ggatttccac 98940

ttacaggaca aaccccacgc aacatttgcg atgccgttta tgtcctgggt cggttctgac 99000

ccaacatctc agctgtacag taatgtgggg ggggtactat ccgtaataac ggaagatgac 99060

ctatccatgt gtatctcaat tgttatatac ggtttacggg taaacagacc tgacgatcag 99120

accacaccaa caccaacccc gcaccagtat acatcgcaaa ggcggcagcc tgaaaccaac 99180

tgtccttcac cacaaccggc ctttttcaca tcagacgacg acgttctttc gttaatatta 99240

cgggacgccg caaacgcgta aagacagatt caagactaac atttatccca actgattaca 99300

tttcatacgc gaataaacga cacaaaaaat ttatatttaa cggcttttaa tttgaagaca 99360

cctatcctct taacgttgag gagccttgca ggttgggtgc cgcgcttcac cggtattata 99420

cataaccgat ttaccgtgtt tacggcagtc tgaccattta ccagtgtatg tctgtaatac 99480

gacgttgttg tgtcccgaca aaattaactc gcgtacaaat ttctgatgtt cccccggcgt 99540

ggcaacgctg gcatttccaa acacattacg ttctcgtacg tccatgaccg ctattttcag 99600

tattaattgg ttggtcggtc aaagtatttt ccttatgtaa aaggacacga tctaaagccg 99660

taaactcgta cacaaacact ggtaccaacg gacgcgattt tccgtccgtt gagcgggtgt 99720

aatatcggcg aggtcttctt gcacgaatac tctcgtacag taggtttctg acacggggtg 99780

catgggtttt ttgacacaac acaaacattt gcaggctctt atgactggat ggattgaatt 99840

tatttttaga tagggtcacg tgtttttgtc gtgacacgcc tcgaccagaa aaggctgcgg 99900

ttttcgtaca cgcgaccgtt atttcacagg cgttcataat caagctgcgg cggatggtgt 99960

cggttaattg tctccgccca agttcgtcaa tagatgatac catgaacaac gtatcaaatg 100020

gtacatagtc gtctttggtt ttctcaatac agcccgcgtg cccaatcgga aaattttcat 100080

ttgcatcaac gctattttct gtaaaatcgg tctgaacact gtgttggctg gctacctgtt 100140

taaaatttgg gatcgaacac ggtccacgat gcaatcccca accccattga agcaatgccg 100200

tcggtacgga aggaggcaac tccgaaaaca ttatggtacg cgagagggtc gattggagtg 100260

ttatataaca ctccaatcga tctcgggttc gcctttacgc gtaaaatact cattggcttg 100320

aacgaaatgt cgacaattcc gaaatggaac acgggacaat ggcgacggat gcgcgtgtgt 100380

tagcaccaga tgacatcttg aattcggttg ggttgtcttc tgtgcatgcg caccccacag 100440

cataaaaact aaccctgtac ggttctcgca taacctctgt agcacgcgtt gcaccagccg 100500

cccccagcct aagtatacat gcgaccccgg agtcccgcga cgaaccgtaa gcgtggtatt 100560

cagcaataac accccctgcc ttgcccaact ctccaggcat ccgtgagtgg gcggagtcat 100620

atttgggtat gattccatga gggccgcaaa aatattttta agactagacg gtggtgttat 100680

gccacgtttt acactaaacg ctagcccatg tgcatgtccc gcggtagggt atggatcttg 100740

accaataatt acaacgcgaa tgctctgggg tccgcaaaat cgcgtccatg caaaaatatc 100800

gcctgtagat ggaagtattt cttcccctga atttaaaaga cgattgtatt ctaaaaaaat 100860

acctttcgcg tacggctctt taagttcgtc cgacaacagg tcataccact caggggaaat 100920

gttaaacttg ctgaaaactt caaccgaatc cagttgcgaa gagacggggg tgaacgtttc 100980

cgtgtcgtaa tgatgtgaca tgttatttaa cttgaaggtt ggggggtcta gcttaacccc 101040

caaaggcggc ccgcggggtc gcttgcgggt ttttttggta accggatggg ccaaaacata 101100

aatgtccttt gaatccgata gtttcatttc attggcatac gcgttggaac aaacggtcgg 101160

ctccccagac acatccattt tccgggatat ttgtggaaga tggagtagag tctacccata 101220

caccggaaag ggcatccaac aaagcatcgc gtatgtcccc gcttttatgt tcttcaccaa 101280

cagattgtgt cagccccttt aaggtgacgt atggatttgt ccagtacgcc atttgtttgt 101340

ctttaaacca aagtataact tccggtactg gacattttgt cttaaccacg attcccgata 101400

gcgcctcgct gaggtttgat accgggggtg ccgcatagtc ccacgcctca tataccgatg 101460

acacgcacgg ttccgttata atcaaactca catccgatag cggtttggct ccaaaaaaca 101520

acggagtgtc gtcttggaga tgaagacaat acgcgattgt gatagttttt aaaaaaacta 101580

tcatctgcag taaccattta tgtgatgcca tgacgcttgt gttttccctt cactacgacg 101640

ttgtcgtatc ctttgaaaaa cttgaccact ctaatggaag catggacaag tatgagtttt 101700

atatatacag ttggccttta gttaaactct tggtgtcata tctcattttc ctaaaaaggg 101760

cgatcttaat atgtcaaacg tcacggcgtg ccgacaaagc gaatttccat gcaagatttg 101820

gatgtagtat ttatacaccc aatcacacgt cacgtattaa gctttacagt cccccgttat 101880

ctgatataat cacttttctt aacacgtcat cgggaaaaca gatgtttata ttatacctct 101940

cgcggtcatt tatggcaaat acttagaccg ttttcaagcg gactgaaaac gctcaaattg 102000

ccttttggag gcctgcccaa cggccattat cccttggatc taagattgat ttgcggtaac 102060

gtttgccaat caagctttaa aaacgtaccc caaacttaaa acgctcaaat tgccttttgg 102120

aggcctgccc aacggccatt atcccttgga tctaagattg atttgcggta acgtttgcca 102180

aacccacgca tttcagttta aatatttcta agcattctta gtgcgtactt ggcagcgtgc 102240

ttaaaatatc aaccaatatc cattatgcta aacgtttcct tctatccgtt tcaatccatt 102300

aaaagtccat taccaaaaat gatgcatcat acctaattca cctaaaaacc tgactcattg 102360

cagcagcgtt tcctccttgc agactatcca gttggcattt taaacgggtc cggctgcctg 102420

aaccgaaaac accgttgcct ttactgtaag tacaaaacta aaatttatat ttgcgtgcgt 102480

attttgtaac atatatgcct tttatccccc cgcaagtttg ctttaccctc gccttcacca 102540

cccccgccac cttccggcca ttgtaataac tttaattgct ataagacata cccaaaccgg 102600

atgatttttg ccgctggaaa aacagcttct aattttcccg tctcaactcg gccttggttg 102660

catctccaag tataccttta gtttgctccc gtagaggtgt ataaatacaa acggtgacaa 102720

gtattgagcg taatctcaaa tttttgtaat ttagggcgga gcgcttacga cagcacatgc 102780

gtactgttag actgttatgt ttattgtatt tgcagagcag gatgccccgg ttactccgag 102840

accggattgc gggcattccg aatcgtgtac ggacttacca gggggcagta tttacacctt 102900

gggttccaga tataccaacc cttacgacca atagcaacac tcaggtattt ttaaaatgca 102960

cgtttaatga tcataattta catacagttg gtaataaagc agaccgtgga tgtttaaggc 103020

atttccttcc ccctcccaac aaactaggac ttcttcatct tgtttggaat acctttaccc 103080

gctttaccgg cagagctttt tttggtaagg tgtttcagtg aacctgatgt tgatccggag 103140

gtggaggggg tattggactc ccctgtggga gaggcaactt tgcgggtttt acttccctta 103200

catgccgaat cagactcaga tgtcaggtct attgttaagc atcgtttaac gtctctgccg 103260

gtatgaaata aacggcgctt agcacccctt gcgcttcccg gtttaatccc cggtaacaca 103320

gaaaaaagcc tgactttttg gggtgtattt accaatcggg tatccctttc atcgccacga 103380

gaggtctccc cggttgaggt ggtttctggt cttacaattg gacctgtaat tagttggatg 103440

gctgtatctt tccaggtcca ggtttgcatg gttaggcggg ttggatcggt acatcgatcc 103500

aacaagaata acatgtttgt tacaaacggt cctgttgaat catgcaaaag acaacgcagg 103560

gatgttttta atcccgcctc atcacgcccg taaataccta tatagtttaa tatcaacatt 103620

tttgtaggct ctacaatttc gggttgatac agttccgcaa gttgatcatc aagccatccg 103680

agtaaaggtt gcatgtaaca cgggaatctc gcgtttccct ctgttcctct atccgtggct 103740

cgaaaaggca gtctgtccat ggttcgtggg tcttgattaa ttcccacaga tactggacga 103800

tcacggtagt cctgcccccc ggtccggggt tgctgtgcag attcaatcga gccatacacc 103860

accggggtcg ccgatcgaac agcaggttgg tctttaaaaa ataccttccg taaaaatgat 103920

gcggtagagc atgttttggt tacaccaggg ctcgagtctc gggtcggtgg ttgtatagaa 103980

tcctgttgag agtcacttgg tgactctgct gtgggctctc tagccgacga ttgaaggggc 104040

ccagggtttg gtgattgaat gggctcccga ctcgatcttg atgttggctg ttggatggac 104100

tcccgactcg gtcctgggct tggtggcaga agatctatga catctcccgg taggatgtcg 104160

atggaatctt caaatgacgg ctcagaaaaa ccatcgtcgt cggatgggtg cacttcatat 104220

tccttgtaac ttgtatcact tacgatctta tgcaggatgg attgcactgg acaccggcag 104280

agaggacact ggacgctggt ggaggtccat gcccgaatac aaacaaagca gaagtcgtgc 104340

aaacacggca tggtttttcc gagatcggaa acggtgctca tgcatatggt gcaggtatta 104400

tccgaagcgt cggaggtgcc gctaccgccc gctaatatgg tatccatggt aacaactggc 104460

tgtattctaa tgtccgggca tccaaacacg tagcagaact gccatgcgtt ctaaattgtg 104520

agttgtggcg agtacatttt tataattggt accaacgaag acacacccct atatccctcc 104580

acccatttct tttaagtccc acccactaaa acgtgggtat aaaatgtgta ttggggtagg 104640

cggacagtcc caacaaacag ggaagttgat tggtataacc ttgggccggg tatacagcta 104700

agtgacattt tagattctgt ctttatttag ataaagagcg atacgaagac atttctccac 104760

ccccctgtaa tacccgtaaa taaaggtaag tccacaaaca aaagcactgt atataggaag 104820

tcgggtgtat tgggacagtt actccattag aggcgtacag acaatactgg gatagggtaa 104880

tgcaagtccc ccccgatggt cgccccgcaa acgcgcgggg aggtggggtc gctttttttt 104940

ttctctctcg agggggccgc gagagggctg ggcccccccc cccggggtcc gccgggcgcc 105000

cagaaaccgg ggggggttat tttcgggggg ggtccgacca gcccgcccgt cgcccgcccg 105060

cacagacaga cagacacttt tttcataaaa accgttccgc ttttattaac aaacaacaaa 105120

cagtccgcgc gccggtggcg ctcacgagaa aaggagggga ctccgtcacc cccgactctg 105180

cggggggctc ctccccccgc gccctcccca cacatcgtcc tcgtcctcgg aggacgagga 105240

cgaggacaac agctccacct tgaccgccgg gcgcgagccc acccggcggt ctcgcagcac 105300

acccggggcc accgacacga cgctcacccc aaaggatgac cccggtgcgt ccccgtcgtc 105360

cccgcccccc tcctcgctgt cccacgcgtc ttcacacccc acctcccaat cgtccggctc 105420

caaagcgtgt tctctgtcgt ctgcggtgcg ccgctgtcgc cccgcctggg tttctgccgg 105480

ccgttccgag cccccgtggt gtccgaacgc gaaccgtgtt ccgtcgctcc cctccaacac 105540

cgtctccgcg gccccaaaac cgggcggcca cattactctg ggaatcgggg ggagggcatt 105600

ccgagcctcg tccgccgacg catacagcgc caccgaccga ccggccacgg gtggaagcac 105660

gagtggttcc gcggcagggt cgggttccag cagggcgtgg cggcaaaaca ccctcgccca 105720

ggtgggtacg tcgccggcct ccggcccggc ggcccccggt ctccgtccct cgggaaggaa 105780

gacgggtcga agcgcggcac ccaggcccca tcggtttgct gcgcggtggc tatgtgccgc 105840

ctcgtccaca aagtcggctg ccccgagccc cagaccccga gactgtcgcg cgaggtcctt 105900

gcaaccgtca aaacccggca gcacgtactg ccggtattca cggggcgaca gggggacgcg 105960

ggtcttgggg cccgcgcggg tacacacggt gtatgcgacg ttcccaccgc ggcacaaaca 106020

caggggttgt tcgcccgggt acaggttggc aaacgcagtc tcgatacgag caaaactcgc 106080

tggcccaaag gtgcgcgacg atgcaaacac ggcccgggcg agtccttctg tgaccgccga 106140

gtctggccat cggacgacgg cctgggcgtc cggtcgcgcc ggggcccgga cgtacacgtg 106200

atactgagac aaagcgggtc catccccggg ccacctctcg agggccaccg cgtccaacac 106260

cagcaaccgg cgccgggcag aggccaaccg cgagcctaga tactcgacgg ccccggcaaa 106320

ggccaggtct cgggtcgaca gtaataaaac gccccgggcg ttcaaagcgg acacgtccgg 106380

cgggccggtc cagttcccgg cccaggcatg agtgctcggc aggcacaacc ggttactcag 106440

ggctgccagg accacagaca gtccccctcg ggatggactc catgacggtc ccggatctgt 106500

cgcgagggtg ctctcgaggg ggccgttgat gtcctctccg ggcaacggat cgtagatgat 106560

cagaagcctc acatcctccg ggtctgggat ctgccgcatc caggcgcacc tccgtcgcag 106620

cgcctccact ccgctgggtg gaccaaaccg tcggtctcct ccgcccggac gccgagcggc 106680

gatttccgcc aaggcgccgg gatcaaagct tagcgcaggg cgccaggccg tgggaaacaa 106740

tgggtcgtcg accagacggg cgatggtttc gggggtacag tacgccttgc gagcctggtc 106800

cgacggaccc ggggtatgca gggccccccg gggaatacgc cgaaatcccc cgtttggggc 106860

cggtccgtca agtggcatcg ttattacggc ggggggatcc accacagggc ccgaggtgat 106920

ggtcacgggc tcggataccc gcctcttggc cttggaaacc acatgatcgt ctgcaacccg 106980

ggcgtccgcg acgggtgtct ccctaatctt gtcgaggagg cttctgctct cgactggctg 107040

ggacttgcgc ttgcgcggag ttcgtaaacg atcatccggt ggacacacag aaagagagcg 107100

cgcggcggcc gacggctgag ggtcgggagt ctgtgtggcc ggggttgttg gagaagggtg 107160

accgcgggag atccgcgccg ccggactgga gcccgttgcc tcggggtatg ccatgccggc 107220

aaaggctctg cggagactct gtaggataaa gtgtttttgg gcccggtcgt accgacggct 107280

catagccacg gccgcggccg cgtgggggag agcccagagg gcctcccccg tggccatggc 107340

ttcgcctaca tgcggaacgg gagacgctac gctccccgta acggcggtac ccgcccgtcc 107400

cggtggcaac agcttttggt agaactggtt cagggccgag ttgacaccgg tcagcttggg 107460

gttctggagc catgctatag ggtctctgtc tggacagtag atcaggttaa tcagcgcgcg 107520

gtactgtcta gccggatctc ccaactccgg cacgtaaagc ggcgcgggtt cagttgaggc 107580

ctcgtaacga gcccgcgccg ctctcacagc ctcatcctcc cagtgaccct ctctggtctc 107640

cccggacggt ccaaaccgca ccctgttgga tgggaggggc gccgatccgg gccaagggct 107700

tccgtcgggc atcatgagcg gccccgacac cgggggaatt atcggggttc tggatcgcgg 107760

cagggaaaat gatttctgtc tctggcgccc cggttccccc gcaagacgtt tggtcttacg 107820

aatcctcgga tcgggaccgc tgatggatcg atatcccggt tggatatttt gtttcgtcga 107880

cccaccatca tttgagtccg aatcatccga atttgacggg gaaggggcgt gttcgcgtcc 107940

ggacctgctg cctgtagttt cacttcccac cgaaacgcgc cggggttcat cgtcttcatc 108000

ctccgatgac gatccccacg acgaggaaga ggatgaagac gaaacaaact cacgactctt 108060

tggctttttc tccaccgggc tgtcatcctc aatcgggtct ggtgcgtggg atcttcccgg 108120

cagggccaaa aacgctctag gtttgccccc cgacgaacgt ccagggacgc gaggtgttat 108180

accccgggca tcatgtttcc ttgggcgggt atcatcggtc tcaaacggca ggtccgcctt 108240

tgccccctta gcgggaacgc tgtccgaaag gacgtggtac aattgctcaa ccgggccggg 108300

tacaggtcca ccgggtttcc gcgccgggag tgggacctta accttcaaag tctttttctt 108360

cgggctcttt ccctgagcgg gccgttgagt tttctggaga actactccgt cccccgatgc 108420

atgcgcatga cccgcttgct catcgcccgg ctttttaccc gagatggact gagtttgtct 108480

gtctcgatgg accaccgacg gcaaacctgg tgaatttcct ctcgtcgttt gtcggggtat 108540

agaccgctgg tcttcccgtt gatcgttccc ggcggcgtct ccaacaggag acgcggggga 108600

tacaggggag aaggcctgcg ggaacggagg ggtcgtacct ctgcccgttt ccccatcgtt 108660

catcggtggt tttggagacc tagcaagctt cgttccgaga gagactgtct cgagggagcg 108720

atcggctcct gttggttctc gcgcgccggc ctccgagaat cgggtgtgga agacctcggc 108780

cagcgggatt acaggcgagc ccgttagatc ctgaccgtcc tcgcatacgt agtcgtcttg 108840

tgttagctct tcgccaacat cttccgttct gggttctggt tgaagtcccg atacggaggg 108900

aattgaaacg atctcatgtt cccgtcccac catgaccccg ttctctccaa atagtagatc 108960

gtcaggctga ctcgaggtga ccacccgggc cctgtgttcg gcggccgcgg cggccgcgtc 109020

caacaggtcc attaactcca aagtatcagg cgaccccgcg cgttggggtg tagagcgctg 109080

catcggcggc gtatccatcg cgctggggtg aatttagacg tacccgagtt ttccaaacgc 109140

tctcgcagcc ttcaaaggat tgcggttgcg gttggtgagg gagttccaac agtacttaaa 109200

acgtgttgtg cccccccctc gaccgcatat ttcctccccg tgtcgtcacc gtgtaaatat 109260

tcttaatgat aagacgatgt agtgattgga cgagactcga ggcgggaagt tcatggacca 109320

tagtatgcgt ttaaggagag accgctggtt ggcgatgtac gcccggtgtc tatttccgca 109380

taccttacaa catcataaca aggataccca gacatgtgaa tttcatttac atatgtttaa 109440

ataacaacca atcatcgtgt gtctacagac gatatataat atacataaac acaattgggg 109500

ttgtctcaca cgcaaaacat cttatataac acgggttgtt tccacccatc cggcatctag 109560

ttaatcaaat gcacgtcgac ggtgtgtttg ggtccctctc cgtcgtcatt acgttcgctc 109620

aatcaacaag cgtatacacc accacccctc ccaacgatta tcatgtcagg cggcacgaag 109680

cccgcgataa cccataaaat acacacgggg ttgtggtgtt cacgtaaccc cccgccgatg 109740

gggagggggc gcggtacccc gccgatgggg agggggcgcg gtaccccgcc gatggggagg 109800

gggcgcggta ccccgccgat gtttataacc ataattctct aaaccgttgt agaaaatcac 109860

aaaaaaattt attcaaaaac aagtcgaaga acttcatatc tgaggcatgt aaacccattc 109920

gcacttcctg gggtggaatg gggtggggtg ggggggtgaa aaaggggggg gttaaattgg 109980

gcgtccgcat gtctgtggtg tacgccaatc ggatacactc ttttaatctg cattcgcact 110040

tcccgttttt tcactgtatg ggttttcatg ttttggcatg tgtccaacca ccgttcgcac 110100

tttctttcta tatatatata tatatatata tagagagaga gagagagaga gagggagaga 110160

gtttcttgtt cgcgcgtgtt cccgcgatgt cgcggtttta tggggtgtgg gcgggctttt 110220

cacagaatat atatattcca aatggagcgg caggcttttt aaaatcgatt tgacgtgata 110280

aaaaaaacac acgggccccc ctttttttgg tgttataaag gcaacccaat cgaaggtctc 110340

ccgccccgga atcccccatt gccattttac ccaagtagcc ttattcatag atgtaaacgt 110400

ttgggtgtgt gttttgttgt gcagggttcg tccgattcat aacgcgacag cgtcgagtcg 110460

gttttaaggg aaaaggttac tacggcccca aggacatgtt ttgcacctca ccggctacgc 110520

ggggcgactc gtccgagtca aaacccgggg catcggttga tgttaacgga aagatggaat 110580

atggatctgc accaggaccc ctgaacggcc gggatacgtc gcggggcccc ggcgcgtttt 110640

gtactccggg ttgggagatc cacccggcca ggctcgttga ggacatcaac cgtgtttttt 110700

tatgtattgc acagtcgtcg ggacgcgtca cgcgagattc acgaagattg cggcgcatat 110760

gcctcgactt ttatctaatg ggtcgcacca gacagcgtcc cacgttagcg tgctgggagg 110820

aattgttaca gcttcaaccc acccagacgc agtgcttacg cgctacttta atggaagtgt 110880

cccatcgacc ccctcggggg gaagacgggt tcattgaggc gccgaatgtt cctttgcata 110940

ggagcgcact ggaatgtgac gtatctgatg atggtggtga agacgatagc gacgatgatg 111000

ggtctacgcc atcggatgta attgaatttc gggattccga cgcggaatca tcggacgggg 111060

aagactttat agtggaagaa gaatcagagg agagcaccga ttcttgtgaa ccagacgggg 111120

tacccggcga ttgttatcga gacggggatg ggtgcaacac cccgtcccca aagagacccc 111180

agcgtgccat cgagcgatac gcgggtgcag aaaccgcgga atatacggcc gcgaaagcgc 111240

tcaccgcgtt gggcgagggg ggtgtagatt ggaagcgacg tcgacacgaa gccccgcgcc 111300

ggcatgatat accgcccccc catggcgtgt agtctttata aataaataca atggtttggc 111360

tcgtgtcttt ttttgatgtc tgtctgtggg ggagtggggt gttgtggata ttagagggta 111420

gagggtgctg gtttgaacgt ctccattaac ccacggggtc cccacacggg ccgtgtggta 111480

tgaatctctg cggatcccgc ggtgagcacc cgggcggtga atatgccgga ctttactgca 111540

cacgacacga tacccccgcg caccgggctc tcatgaacga cgccgaacgg tacttcgccg 111600

ccgcgctatg cgccatatct accgaggcct acgaggcttt tatacacagc ccctccgaga 111660

gaccgtgcgc gagtttgtgg gggagggcaa aggacgcctt cggacggatg tgcggggagc 111720

tcgcagcgga tagacaacgt ccaccctcgg ttccgccgat ccgcagagcg gtgttatcgt 111780

tattacgcga gcaatgcatg ccggatccac aatcgcatct ggagctcagc gagcggctga 111840

tattgatggc atattggtgc tgtttgggac acgccggact tccgactatt ggattgtcgc 111900

ccgataataa atgcatccgc gccgaattat atgaccgccc cgggggaatt tgtcacaggc 111960

tttttgacgc gtacctgggc tgcgggtccc ttggagtccc aagaacccac gagagatcct 112020

gacaccccat ccctttatat agaaaaaaaa aaaaataaat ttaaaacata caccggataa 112080

aagcgtactg ttttttattt aaatttacac gctcgacgtt gccccggttc ggtgatcacc 112140

gggtcttatc tatatacacc gtgtaactcg aacccccgtg actccctcca atcgcgttac 112200

caaactcttc ttccgtatcc gtagattccg agtcctcgaa atcgtccact tatccaacaa 112260

attgtgacgt tatatatccc aaggcaaagg ccgctcccgt catagcaaat acaaagacaa 112320

ttattagcgt aatataacag aattttttac gatgatatat tttatgttga tattttccaa 112380

ttcgacgcaa aaattcatct gccgtttcat tttcgctatc actataataa cacttttcag 112440

ccgaacggct cggttgtatg gctgttatcg ttgtattatt tggttgcgct cgcggggtta 112500

ccaccgcttc catcagtaag gccacggcct caccctccat ggtgttttgt ccggccatag 112560

aaatccagat tgtaaggcca gcaggctagt ttaaaagtgt ttaataccac accttttgat 112620

atttatatac atgcaagatt ctagattatt catcaatagg tcgtttaaag cgcgttttca 112680

taaacgttgt cagctatacc gacattctca caaagaggta aagttacctt acgttattat 112740

taaataaaac atgtagacat tattaataat cctaggaaca atcaaatcca tatttgtaag 112800

ttatgtttaa cccctcccct ttttgtcatt atctccgccc tcttataatc ggatcacttt 112860

ataagtgtgt cggtgagtat attttgtaca gttgttggac aacaggtttt tggttcatta 112920

acactatcaa cataagtcgg ggtatacaag tataatgaac gacgttgatg caacagacac 112980

ctttgttgga caaggaaagt tccgtggcgc catctcaaca tcaccgtcac atattatgca 113040

aacatgtggg tttatacaac agatgtttcc agttgaaatg tcgcccggca tagaatctga 113100

ggatgatccc aattatgacg ttaacatgga tatacagtct tttaatatat ttgatggtgt 113160

acacgaaact gaagccgaag cctctgtggc attgtgcgca gaagcacgcg ttggaattaa 113220

taaagcggga tttgtaatat taaaaacgtt tacaccaggg gcggaaggtt ttgcgtttgc 113280

gtgtatggac agtaaaacat gtgaacatgt ggtcattaaa gcgggtcaac gtcaaggaac 113340

ggccaccgag gcaaccgtgt taagagcgtt aacccaccca tccgttgtac agcttaaagg 113400

aacgtttacg tataacaaaa tgacatgtct tatattacca cgttaccgaa cagatttata 113460

ctgctatcta gctgcaaagc gcaacctccc catatgtgac attttagcaa ttcagcgatc 113520

tgtattacgc gcgttacagt atcttcataa taacagtatt attcaccgtg atataaaatc 113580

tgaaaatata tttattaacc acccaggtga tgtttgtgtg ggagactttg gagcagcgtg 113640

tttccccgtg gatattaatg ccaacaggta ttatggctgg gctggaacaa tcgccacaaa 113700

ctctcctgag ttattggcta gagatccata tggacctgcc gtggacatat ggagtgccgg 113760

gattgtatta tttgaaatgg ctacaggaca gaactcgtta tttgaacgag acggtttaga 113820

tggcaattgt gacagtgagc gtcaaattaa acttattata cgacgatctg gaactcatcc 113880

caatgaattt cccattaacc ctacatcaaa tcttcgtcga caatacattg gtttggcaaa 113940

acggtcttct cgaaaacccg gatccaggcc attgtggaca aatctatatg agttgccaat 114000

tgatttggag tatttgatat gtaagatgtt atcgtttgac gcacgtcatc gaccatcggc 114060

agaggtgttg cttaaccact ctgttttcca aactcttccc gatccatatc caaatccaat 114120

ggaagttgga gattaaaatt cattaagcct gttaataaaa tattgtataa attgtgttta 114180

taacgtataa cccgttaagg caaatagggt acaaacgcgc aatgttttga aatactaata 114240

taaataacat aaccaataga aacttaatac agagtcacgc cccattacaa caaggataaa 114300

acacgggatc attttcttaa cattgtagta gcgctgaaaa gcgtcccctc ccccggctca 114360

cagagctgct cttcggtgta gttgggtata ctggtgcgcc tcatttaatc gcgatgtttt 114420

taatccaatg tttgatatcg gccgttatat tttacataca agtgaccaac gctttgatct 114480

tcaagggcga ccacgtgagc ttgcaagtta acagcagtct cacgtctatc cttattccca 114540

tgcaaaatga taattataca gagataaaag gacagcttgt ctttattgga gagcaactac 114600

ctaccgggac aaactatagc ggaacactgg aactgttata cgcggatacg gtggcgtttt 114660

gtttccggtc agtacaagta ataagatacg acggatgtcc ccggattaga acgagcgctt 114720

ttatttcgtg taggtacaaa cattcgtggc attatggtaa ctcaacggat cggatatcaa 114780

cagagccgga tgctggtgta atgttgaaaa ttaccaaacc gggaataaat gatgctggtg 114840

tgtatgtact tcttgttcgg ttagaccata gcagatccac cgatggtttc attcttggtg 114900

taaatgtata tacagcgggc tcgcatcaca acattcacgg ggttatctac acttctccat 114960

ctctacagaa tggatattct acaagagccc tttttcaaca agctcgtttg tgtgatttac 115020

ccgcgacacc caaagggtcc ggtacctccc tgtttcaaca tatgcttgat cttcgtgccg 115080

gtaaatcgtt agaggataac ccttggttac atgaggacgt tgttacgaca gaaactaagt 115140

ccgttgttaa ggaggggata gaaaatcacg tatatccaac ggatatgtcc acgttacccg 115200

aaaagtccct taatgatcct ccagaaaatc tacttataat tattcctata gtagcgtctg 115260

tcatgatcct caccgccatg gttattgtta ttgtaataag cgttaagcga cgtagaatta 115320

aaaaacatcc aatttatcgc ccaaatacaa aaacaagaag gggcatacaa aatgcgacac 115380

cagaatccga tgtgatgttg gaggccgcca ttgcacaact agcaacgatt cgcgaagaat 115440

cccccccaca ttccgttgta aacccgtttg ttaaatagaa ctaattatcc cggattttat 115500

attaaataaa ctatatgcgt tttatttagc gttttgatta cgcgttgtga tatgagggga 115560

aggattaaga atctcctaac tataagttaa cacgcccaca tttgggcggg gatgttttat 115620

gaagccttaa aggccgagct ggtatacacg agagcagtcc atggttttag acctcgggcg 115680

aattgcgtgg ttttaagtga ctatattccg agggtcgcct gtaatatggg gacagttaat 115740

aaacctgtgg tgggggtatt gatggggttc ggaattatca cgggaacgtt gcgtataacg 115800

aatccggtca gagcatccgt cttgcgatac gatgattttc acatcgatga agacaaactg 115860

gatacaaact ccgtatatga gccttactac cattcagatc atgcggagtc ttcatgggta 115920

aatcggggag agtcttcgcg aaaagcgtac gatcataact caccttatat atggccacgt 115980

aatgattatg atggattttt agagaacgca cacgaacacc atggggtgta taatcagggc 116040

cgtggtatcg atagcgggga acggttaatg caacccacac aaatgtctgc acaggaggat 116100

cttggggacg atacgggcat ccacgttatc cctacgttaa acggcgatga cagacataaa 116160

attgtaaatg tggaccaacg tcaatacggt gacgtgttta aaggagatct taatccaaaa 116220

ccccaaggcc aaagactcat tgaggtgtca gtggaagaaa atcacccgtt tactttacgc 116280

gcaccgattc agcggattta tggagtccgg tacaccgaga cttggagctt tttgccgtca 116340

ttaacctgta cgggagacgc agcgcccgcc atccagcata tatgtttaaa acatacaaca 116400

tgctttcaag acgtggtggt ggatgtggat tgcgcggaaa atactaaaga ggatcagttg 116460

gccgaaatca gttaccgttt tcaaggtaag aaggaagcgg accaaccgtg gattgttgta 116520

aacacgagca cactgtttga tgaactcgaa ttagaccccc ccgagattga accgggtgtc 116580

ttgaaagtac ttcggacaga aaaacaatac ttgggtgtgt acatttggaa catgcgcggc 116640

tccgatggta cgtctaccta cgccacgttt ttggtcacct ggaaagggga tgaaaaaaca 116700

agaaacccta cgcccgcagt aactcctcaa ccaagagggg ctgagtttca tatgtggaat 116760

taccactcgc atgtattttc agttggtgat acgtttagct tggcaatgca tcttcagtat 116820

aagatacatg aagcgccatt tgatttgctg ttagagtggt tgtatgtccc catcgatcct 116880

acatgtcaac caatgcggtt atattctacg tgtttgtatc atcccaacgc accccaatgc 116940

ctctctcata tgaattccgg ttgtacattt acctcgccac atttagccca gcgtgttgca 117000

agcacagtgt atcaaaattg tgaacatgca gataactaca ccgcatattg tctgggaata 117060

tctcatatgg agcctagctt tggtctaatc ttacacgacg ggggcaccac gttaaagttt 117120

gtagatacac ccgagagttt gtcgggatta tacgtttttg tggtgtattt taacgggcat 117180

gttgaagccg tagcatacac tgttgtatcc acagtagatc attttgtaaa cgcaattgaa 117240

gagcgtggat ttccgccaac ggccggtcag ccaccggcga ctactaaacc caaggaaatt 117300

acccccgtaa accccggaac gtcaccactt ctacgatatg ccgcatggac cggagggctt 117360

gcagcagtag tacttttatg tctcgtaata tttttaatct gtacggctaa acgaatgagg 117420

gttaaagcct atagggtaga caagtccccg tataaccaaa gcatgtatta cgctggcctt 117480

ccagtggacg atttcgagga ctcggaatct acggatacgg aagaagagtt tggtaacgcg 117540

attggaggga gtcacggggg ttcgagttac acggtgtata tagataagac ccggtgatca 117600

ccgaaccggg gcaacgtcga gcgtgtaaat ttaaataaaa aacagtacgc ttttatccgg 117660

tgtatgtttt aaatttattt tttttttttt ctatataaag ggatggggtg tcaggatctc 117720

tcgtgggttc ttgggactcc aagggacccg cagcccaggt acgcgtcaaa aagcctgtga 117780

caaattcccc cggggcggtc atataattcg gcgcggatgc atttattatc gggcgacaat 117840

ccaatagtcg gaagtccggc gtgtcccaaa cagcaccaat atgccatcaa tatcagccgc 117900

tcgctgagct ccagatgcga ttgtggatcc ggcatgcatt gctcgcgtaa taacgataac 117960

accgctctgc ggatcggcgg aaccgagggt ggacgttgtc tatccgctgc gagctccccg 118020

cacatccgtc cgaaggcgtc ctttgccctc ccccacaaac tcgcgcacgg tctctcggag 118080

gggctgtgta taaaagcctc gtaggcctcg gtagatatgg cgcatagcgc ggcggcgaag 118140

taccgttcgg cgtcgttcat gagagcccgg tgcgcggggg tatcgtgtcg tgtgcagtaa 118200

agtccggcat attcaccgcc cgggtgctca ccgcgggatc cgcagagatt cataccacac 118260

ggcccgtgtg gggaccccgt gggttaatgg agacgttcaa accagcaccc tctaccctct 118320

aatatccaca acaccccact cccccacaga cagacatcaa aaaaagacac gagccaaacc 118380

attgtattta tttataaaga ctacacgcca tgggggggcg gtatatcatg ccggcgcggg 118440

gcttcgtgtc gacgtcgctt ccaatctaca cccccctcgc ccaacgcggt gagcgctttc 118500

gcggccgtat attccgcggt ttctgcaccc gcgtatcgct cgatggcacg ctggggtctc 118560

tttggggacg gggtgttgca cccatccccg tctcgataac aatcgccggg taccccgtct 118620

ggttcacaag aatcggtgct ctcctctgat tcttcttcca ctataaagtc ttccccgtcc 118680

gatgattccg cgtcggaatc ccgaaattca attacatccg atggcgtaga cccatcatcg 118740

tcgctatcgt cttcaccacc atcatcagat acgtcacatt ccagtgcgct cctatgcaaa 118800

ggaacattcg gcgcctcaat gaacccgtct tccccccgag gggtcgatgg ggacacttcc 118860

attaaagtag cgcgtaagca ctgcgtctgg gtgggttgaa gctgtaacaa ttcctcccag 118920

cacgctaacg tgggacgctg tctggtgcga cccattagat aaaagtcgag gcatatgcgc 118980

cgcaatcttc gtgaatctcg cgtgacgcgt cccgacgact gtgcaataca taaaaaaaca 119040

cggttgatgt cctcaacgag cctggccggg tggatctccc aacccggagt acaaaacgcg 119100

ccgggggccc gcgacgtatc ccggccgttc aggggtcctg gtgcagatcc atattccatc 119160

tttccgttaa catcaaccga tgccccgggt tttgactcgg acgagtcgcc ccgcgtagcc 119220

ggtgaggtgc aaaacatgtc cttggggccg tagtaacctt ttcccttaaa accgactcga 119280

cgctgtcgcg ttatgaatcg gacgaaccct gcacaacaaa acacacaccc aaacgtttac 119340

atctatgaat aaggctactt gggtaaaatg gcaatggggg attccggggc gggagacctt 119400

cgattgggtt gcctttataa caccaaaaaa aggggggccc gtgtgttttt tttatcacgt 119460

caaatcgatt ttaaaaagcc tgccgctcca tttggaatat atatattctg tgaaaagccc 119520

gcccacaccc cataaaaccg cgacatcgcg ggaacacgcg cgaacaagaa actctctccc 119580

tctctctctc tctctctctc tatatatata tatatatata tatagaaaga aagtgcgaac 119640

ggtggttgga cacatgccaa aacatgaaaa cccatacagt gaaaaaacgg gaagtgcgaa 119700

tgcagattaa aagagtgtat ccgattggcg tacaccacag acatgcggac gcccaattta 119760

acccccccct ttttcacccc cccaccccac cccattccac cccaggaagt gcgaatgggt 119820

ttacatgcct cagatatgaa gttcttcgac ttgtttttga ataaattttt ttgtgatttt 119880

ctacaacggt ttagagaatt atggttataa acatcggcgg ggtaccgcgc cccctcccca 119940

tcggcggggt accgcgcccc ctccccatcg gcggggtacc gcgccccctc cccatcggcg 120000

gggggttacg tgaacaccac aaccccgtgt gtattttatg ggttatcgcg ggcttcgtgc 120060

cgcctgacat gataatcgtt gggaggggtg gtggtgtata cgcttgttga ttgagcgaac 120120

gtaatgacga cggagaggga cccaaacaca ccgtcgacgt gcatttgatt aactagatgc 120180

cggatgggtg gaaacaaccc gtgttatata agatgttttg cgtgtgagac aaccccaatt 120240

gtgtttatgt atattatata tcgtctgtag acacacgatg attggttgtt atttaaacat 120300

atgtaaatga aattcacatg tctggtatcc cttgttatga tgttgtaagg tatgcggaaa 120360

tagacaccgg gcgtacatcg ccaaccagcg gtctctcctt aaacgcatac tatggtccat 120420

gaacttcccg cctcgagtct cgtccaatca ctacatcgtc ttatcattaa gaatatttac 120480

acggtgacga cacggggagg aaatatgcgg tcgagggggg ggcacaacac gttttaagta 120540

ctgttggaac tccctcacca accgcaaccg caatcctttg aaggctgcga gagcgtttgg 120600

aaaactcggg tacgtctaaa ttcaccccag cgcgatggat acgccgccga tgcagcgctc 120660

tacaccccaa cgcgcggggt cgcctgatac tttggagtta atggacctgt tggacgcggc 120720

cgccgcggcc gccgaacaca gggcccgggt ggtcacctcg agtcagcctg acgatctact 120780

atttggagag aacggggtca tggtgggacg ggaacatgag atcgtttcaa ttccctccgt 120840

atcgggactt caaccagaac ccagaacgga agatgttggc gaagagctaa cacaagacga 120900

ctacgtatgc gaggacggtc aggatctaac gggctcgcct gtaatcccgc tggccgaggt 120960

cttccacacc cgattctcgg aggccggcgc gcgagaacca acaggagccg atcgctccct 121020

cgagacagtc tctctcggaa cgaagcttgc taggtctcca aaaccaccga tgaacgatgg 121080

ggaaacgggc agaggtacga cccctccgtt cccgcaggcc ttctcccctg tatcccccgc 121140

gtctcctgtt ggagacgccg ccgggaacga tcaacgggaa gaccagcggt ctataccccg 121200

acaaacgacg agaggaaatt caccaggttt gccgtcggtg gtccatcgag acagacaaac 121260

tcagtccatc tcgggtaaaa agccgggcga tgagcaagcg ggtcatgcgc atgcatcggg 121320

ggacggagta gttctccaga aaactcaacg gcccgctcag ggaaagagcc cgaagaaaaa 121380

gactttgaag gttaaggtcc cactcccggc gcggaaaccc ggtggacctg tacccggccc 121440

ggttgagcaa ttgtaccacg tcctttcgga cagcgttccc gctaaggggg caaaggcgga 121500

cctgccgttt gagaccgatg atacccgccc aaggaaacat gatgcccggg gtataacacc 121560

tcgcgtccct ggacgttcgt cggggggcaa acctagagcg tttttggccc tgccgggaag 121620

atcccacgca ccagacccga ttgaggatga cagcccggtg gagaaaaagc caaagagtcg 121680

tgagtttgtt tcgtcttcat cctcttcctc gtcgtgggga tcgtcatcgg aggatgaaga 121740

cgatgaaccc cggcgcgttt cggtgggaag tgaaactaca ggcagcaggt ccggacgcga 121800

acacgcccct tccccgtcaa attcggatga ttcggactca aatgatggtg ggtcgacgaa 121860

acaaaatatc caaccgggat atcgatccat cagcggtccc gatccgagga ttcgtaagac 121920

caaacgtctt gcgggggaac cggggcgcca gagacagaaa tcattttccc tgccgcgatc 121980

cagaaccccg ataattcccc cggtgtcggg gccgctcatg atgcccgacg gaagcccttg 122040

gcccggatcg gcgcccctcc catccaacag ggtgcggttt ggaccgtccg gggagaccag 122100

agagggtcac tgggaggatg aggctgtgag agcggcgcgg gctcgttacg aggcctcaac 122160

tgaacccgcg ccgctttacg tgccggagtt gggagatccg gctagacagt accgcgcgct 122220

gattaacctg atctactgtc cagacagaga ccctatagca tggctccaga accccaagct 122280

gaccggtgtc aactcggccc tgaaccagtt ctaccaaaag ctgttgccac cgggacgggc 122340

gggtaccgcc gttacgggga gcgtagcgtc tcccgttccg catgtaggcg aagccatggc 122400

cacgggggag gccctctggg ctctccccca cgcggccgcg gccgtggcta tgagccgtcg 122460

gtacgaccgg gcccaaaaac actttatcct acagagtctc cgcagagcct ttgccggcat 122520

ggcatacccc gaggcaacgg gctccagtcc ggcggcgcgg atctcccgcg gtcacccttc 122580

tccaacaacc ccggccacac agactcccga ccctcagccg tcggccgccg cgcgctctct 122640

ttctgtgtgt ccaccggatg atcgtttacg aactccgcgc aagcgcaagt cccagccagt 122700

cgagagcaga agcctcctcg acaagattag ggagacaccc gtcgcggacg cccgggttgc 122760

agacgatcat gtggtttcca aggccaagag gcgggtatcc gagcccgtga ccatcacctc 122820

gggccctgtg gtggatcccc ccgccgtaat aacgatgcca cttgacggac cggccccaaa 122880

cgggggattt cggcgtattc cccggggggc cctgcatacc ccggtcccgt cggaccaggc 122940

tcgcaaggcg tactgtaccc ccgaaaccat cgcccgtctg gtcgacgacc cattgtttcc 123000

cacggcctgg cgccctgcgc taagctttga tcccggcgcc ttggcggaaa tcgccgctcg 123060

gcgtccgggc ggaggagacc gacggtttgg tccacccagc ggagtggagg cgctgcgacg 123120

gaggtgcgcc tggatgcggc agatcccaga cccggaggat gtgaggcttc tgatcatcta 123180

cgatccgttg cccggagagg acatcaacgg ccccctcgag agcaccctcg cgacagatcc 123240

gggaccgtca tggagtccat cccgaggggg actgtctgtg gtcctggcag ccctgagtaa 123300

ccggttgtgc ctgccgagca ctcatgcctg ggccgggaac tggaccggcc cgccggacgt 123360

gtccgctttg aacgcccggg gcgttttatt actgtcgacc cgagacctgg cctttgccgg 123420

ggccgtcgag tatctaggct cgcggttggc ctctgcccgg cgccggttgc tggtgttgga 123480

cgcggtggcc ctcgagaggt ggcccgggga tggacccgct ttgtctcagt atcacgtgta 123540

cgtccgggcc ccggcgcgac cggacgccca ggccgtcgtc cgatggccag actcggcggt 123600

cacagaagga ctcgcccggg ccgtgtttgc atcgtcgcgc acctttgggc cagcgagttt 123660

tgctcgtatc gagactgcgt ttgccaacct gtacccgggc gaacaacccc tgtgtttgtg 123720

ccgcggtggg aacgtcgcat acaccgtgtg tacccgcgcg ggccccaaga cccgcgtccc 123780

cctgtcgccc cgtgaatacc ggcagtacgt gctgccgggt tttgacggtt gcaaggacct 123840

cgcgcgacag tctcggggtc tggggctcgg ggcagccgac tttgtggacg aggcggcaca 123900

tagccaccgc gcagcaaacc gatggggcct gggtgccgcg cttcgacccg tcttccttcc 123960

cgagggacgg agaccggggg ccgccgggcc ggaggccggc gacgtaccca cctgggcgag 124020

ggtgttttgc cgccacgccc tgctggaacc cgaccctgcc gcggaaccac tcgtgcttcc 124080

acccgtggcc ggtcggtcgg tggcgctgta tgcgtcggcg gacgaggctc ggaatgccct 124140

ccccccgatt cccagagtaa tgtggccgcc cggttttggg gccgcggaga cggtgttgga 124200

ggggagcgac ggaacacggt tcgcgttcgg acaccacggg ggctcggaac ggccggcaga 124260

aacccaggcg gggcgacagc ggcgcaccgc agacgacaga gaacacgctt tggagccgga 124320

cgattgggag gtggggtgtg aagacgcgtg ggacagcgag gaggggggcg gggacgacgg 124380

ggacgcaccg gggtcatcct ttggggtgag cgtcgtgtcg gtggccccgg gtgtgctgcg 124440

agaccgccgg gtgggctcgc gcccggcggt caaggtggag ctgttgtcct cgtcctcgtc 124500

ctccgaggac gaggacgatg tgtggggagg gcgcgggggg aggagccccc cgcagagtcg 124560

ggggtgacgg agtcccctcc ttttctcgtg agcgccaccg gcgcgcggac tgtttgttgt 124620

ttgttaataa aagcggaacg gtttttatga aaaaagtgtc tgtctgtctg tgcgggcggg 124680

cgacgggcgg gctggtcgga ccccccccga aaataacccc ccccggtttc tgggcgcccg 124740

gcggaccccg gggggggg 124758

<210> 2
<211> 666
<212> DNA
<213> Varicella-zoster virus

<400> 2
atggccaccg tgcactactc ccgccgacct gggaccccgc cggtcaccct cacgtcgtcc 60

cccggcatgg atgacgttgc gacccccatt ccctacctac ccacatacgc cgaggccgtg 120

gcagacgcgc ccccccctta cagaagccgc gagagtctgg tgttctcccc gcctcttttt 180

cctcacgtgg agaatggcac cacccaacag tcttacgatt gcctagactg cgcttatgat 240

ggaatccaca gacttcagct ggcttttcta agaattcgca aatgctgtgt accggctttt 300

ttaattcttt ttggtattct cacccttact gctgtcgtgg tcgccattgt tgccgttttt 360

cccgaggaac ctcccaactc aactacacga aactactgtc cggaagggga aggtatttat 420

tctcgcttgc agcttgtcgc gcgtgtatgc acaacaaaag ctatatatgt caccaaagcc 480

aacgtcgcca tctggagtac tacacccagt acattgcata acctgtccat ctgcattttc 540

agttgcgcgg acgcctttct ccgggatcgt ggccttggga catcaaccag cggaataaga 600

accgccggtg gtcttgcccg aacgacgagt ggcgacgcgt tgttctgcat aagctctgta 660

tgctga 666

<210> 3
<211> 1365
<212> DNA
<213> Varicella-zoster virus

<400> 3
atggggctct ttggactgac acgctttatc catgaacata aactggttaa acccagcatc 60

atttcaacgc cacccggagt tttaaccccc gtggcggtag acgtatggaa cgtcatgtac 120

acattgttgg aacgtttata ccctgtgggt aaacgcgaga atttacacgg accatctgta 180

acgatacatt gtcttggagt cttattgcgg ctattaacac aacggtcata ctatccgata 240

tttgtattgg aacgttgtac agacggccca ttatcacgtg gagccaaggc aattatgtca 300

cgggccatga accacgatga aaggggaacc tcggacttaa cccgtgttct actatcatcc 360

aacacatgtt ctatcaagta taacaaaaca tcggaaacat atgacagtgt gtttcgaaac 420

tcttccgcga gttgtattcc tagcgaagaa aacaaatccc aggatatgtt tttggacggt 480

tgtccacgac aaactgacaa gatgatctgc ctgcgcgacc aaaacgtatg cagtcttacc 540

tctacaatgc catcccgagg acatcctaac catcgattat atcacaaatt gtgtgcaagt 600

cttattagat ggatggggta tgcatacgtc gaggcggttg acattgaggc ggacgaggca 660

tgtgcaaact tatttcatac gcgtacagtg gctttggttt atacgacaga tactgattta 720

ctcttcatgg gctgtgatat tttgttagat gcaattccta tgtttgctcc agtagtacga 780

tgtcgcgatt tgcttcaata tttaggaatt acataccctg aatttttggt tgcctttgtt 840

cgctgtcaga ccgatttgca tacaagtgac aacctaaaat ctgttcagca agttattcag 900

gataccggcc tgaaaattcc acatcaaatg gacacttcaa cgcgctcccc cacttacgac 960

tcgtggagac atggcgaggt tttcaaaagt cttaccgtag ccacgtcggg taaaacagaa 1020

aacggagtgt ccgtttccaa atatgcatct aaccgatcgg aggtgacagt agacgccagt 1080

tgggctttaa accttctgcc accctcatcc tccccattgg ataatttgga acgcgcattt 1140

gttgaacata taatcgccgt ggtaactcca ttgacccgcg gtcgcctaaa gttaatgaaa 1200

cgtgtaaata ttatgcaaaa tacggcagac ccatatatgg ttattaacac cttatatcat 1260

aacttaaagg gggaaaaaat ggctcgccaa tacgcacgta tttttaaaca gtttattcct 1320

actccactcc cactaaacac tgtattaaca aaatattgga attaa 1365

<210> 4
<211> 3600
<212> DNA
<213> Varicella-zoster virus

<400> 4
atggaaaata ctcagaagac tgtgacagtg cccacggggc ccctgggtta cgtttatgcg 60

tgccgggttg aagatttgga tctggaggaa atttcatttt tggccgctcg tagcacggac 120

tctgatttgg ctttattacc tttgatgcgt aatttgaccg tggaaaaaac ttttacatcc 180

agcctggcgg tggtttctgg agcacgcact acgggtcttg ccggagctgg tattacctta 240

aaactcacta ccagtcattt ctatccatct gtctttgtct ttcacggagg caaacacgtt 300

ttacccagct ctgcggcccc aaatctcaca cgcgcgtgta acgcggctcg agaacggttt 360

gggttttcac gctgccaagg gcctcctgtt gacggtgctg ttgagacgac cggcgctgag 420

atatgcaccc gccttggatt agagccagaa aatacaatat tatacttggt ggtcacggca 480

ttgtttaagg aagccgtatt tatgtgcaac gtgtttctgc attatggagg actcgatatt 540

gttcatatta accatgggga tgttatacgt ataccgttat ttccggtaca acttttcatg 600

cccgatgtta accgtctggt acccgaccca ttcaacactc atcacaggtc tatcggagag 660

ggttttgtat acccaacacc cttttataac accgggttgt gccatttaat acatgactgt 720

gttattgctc ccatggccgt tgccttgcgc gtcagaaatg taactgccgt cgcccgagga 780

gcggcccacc ttgcttttga tgaaaatcac gagggggcag tactcccccc tgacattacg 840

tacacgtatt ttcagtcctc ttcaagtgga accactaccg cccgtggagc gcgtcgaaac 900

gatgtcaact ccacgtctaa gcctagccca tcgggggggt ttgaaagacg gttggcgtct 960

attatggccg ctgacacagc cttgcacgca gaagttatat tcaacactgg aatttacgaa 1020

gaaactccaa cagatatcaa agaatggcca atgtttatag gcatggaggg cactttgcca 1080

aggctaaacg ctctggggtc atataccgct cgtgtggccg gggtcattgg tgcgatggtt 1140

ttcagcccaa attctgcgtt gtatctaact gaggtggagg atagcgggat gaccgaagcc 1200

aaggatgggg gaccgggtcc atcatttaat cgattttacc agtttgccgg acctcattta 1260

gctgcgaatc cccaaacaga tcgagatggc cacgttctat ccagtcagtc tacgggttca 1320

tcaaacacag agtttagcgt ggattatttg gcactcattt gtggatttgg agcacccctg 1380

ttggcgcgac tgctttttta tctagaacgc tgtgacgctg gtgcgtttac agggggtcac 1440

ggggatgcgt taaaatatgt tacggggacc tttgactctg aaattccatg tagtttatgt 1500

gaaaaacaca cgcggccggt atgcgctcac acaacagtac accgacttag acaacgcatg 1560

ccgcgatttg gacaagccac ccgtcaacct attggggtgt ttggaacaat gaacagccaa 1620

tatagcgact gcgatcctct aggaaactat gctccatatt taatccttcg aaaacccggg 1680

gatcaaacgg aagcagcaaa ggcaaccatg caggacactt atagggctac actagaacgc 1740

ttgtttatcg atctagaaca agagcgacta ctggatcgcg gtgccccatg ttcttccgag 1800

ggactatcgt ctgtcattgt ggatcatcca acgtttcgtc gcatattaga cacactgcgt 1860

gcgcgtatag aacagacaac aacacaattt atgaaagtgt tggttgagac ccgcgattat 1920

aagatccgtg aaggattatc cgaagccacc cattcaatgg cgttaacgtt tgatccatac 1980

tcaggagcat tttgtcccat taccaatttt ttagttaaac gaacacacct agccgtggta 2040

caagacttag cattaagcca gtgtcattgt gtattttacg gacagcaagt tgaggggcgg 2100

aactttcgta accaattcca acctgttttg cggcggcgtt ttgttgacct gtttaatggg 2160

gggtttatat caacacgctc tataaccgta acattatctg aaggtcctgt atccgcccca 2220

aatccgacat tgggacaaga cgcgcccgcg gggcgtacct ttgatgggga tttagcgcgc 2280

gtaagcgtgg aagttattcg ggatatacga gttaaaaata gggtcgtttt ttcaggtaac 2340

tgtacaaatc tctctgaggc agcccgggca aggcttgtag gccttgcaag tgcgtaccaa 2400

cgccaagaaa aaagagtgga tatgttacac ggggccctag ggtttttgct taaacagttt 2460

cacggcctgt tatttcctcg gggtatgcca ccaaacagta aatcccccaa cccgcagtgg 2520

ttttggaccc tgttacaacg caaccagatg ccggcagata aacttacaca cgaagagatt 2580

accactattg cagctgttaa acggtttacc gaggaatatg cagcactaaa ctttattaat 2640

ctacccccaa cctgcatagg agaattagcc cagttttata tggcaaatct tattcttaaa 2700

tactgcgatc attcacagta ccttataaat accttaactt ctataattac gggtgccagg 2760

cgcccgcgtg acccatcatc cgttttgcat tggattcgta aagatgtcac gtccgccgcg 2820

gacatagaaa cccaagcaaa ggcgcttctt gaaaaaacgg aaaacttacc ggaattatgg 2880

actacggctt ttacttcaac tcatttagtc cgcgcggcca tgaatcaacg tcccatggtc 2940

gttttaggaa taagcattag taaatatcac ggagcggcag gaaacaaccg cgtctttcag 3000

gcagggaatt ggagcggttt aaacgggggt aaaaatgtat gcccgctatt tacatttgat 3060

cgcactcgcc gttttataat aacatgtcct agaggaggtt ttatctgccc cgtaacaggt 3120

ccctcgtcgg gaaatcgaga aaccacccta tccgaccaag ttcgcggtat aattgtcagt 3180

ggcggggcca tggttcaatt agccatatac gccacggttg tgcgtgcagt gggcgctcga 3240

gcacaacata tggcatttga cgactggtta agtcttacag acgatgagtt tttagccaga 3300

gacttggagg agttacacga ccagattatc caaaccctgg aaacgccctg gaccgtagaa 3360

ggcgctctag aagcagtaaa gattctagat gaaaaaacga cagcgggaga tggggaaacc 3420

cccacaaacc tagcatttaa ttttgattct tgtgaaccaa gccatgacac cacatctaac 3480

gtattaaaca tttcagggtc aactgtccct ggtcttaaac gaccccccga agatgacgaa 3540

ctctttgatc ttagtggtat tcccataaaa catgggaaca ttacaatgga aatgatttaa 3600

3600

<210> 5
<211> 732
<212> DNA
<213> Varicella-zoster virus

<400> 5
atgaaaaatc cgcagaaatt agcgatcaca ttcttgccgc tctacgtgat cccaacgtac 60

acgttgtgta ttaaagcatt gtataaaaac acgcatgcgg gcttgctgtt ctcatttcta 120

ggttttgtct taaatacacc cgccatgagc atctctggac ccccaacgac gtttatttta 180

tataggttac atggggtcag gcgggttctt cactggactt taccggatca tgaacaaacc 240

ctctacgcat ttacgggtgg gtcaagatca atggcggtga agacggacgc tcgatgtgat 300

acaatgagcg gtggtatgat cgtccttcaa cacacccata cagtgaccct gctaaccata 360

gactgttcta ctgacttttc atcatacgca tttacgcacc gggatttcca cttacaggac 420

aaaccccacg caacatttgc gatgccgttt atgtcctggg tcggttctga cccaacatct 480

cagctgtaca gtaatgtggg gggggtacta tccgtaataa cggaagatga cctatccatg 540

tgtatctcaa ttgttatata cggtttacgg gtaaacagac ctgacgatca gaccacacca 600

acaccaaccc cgcaccagta tacatcgcaa aggcggcagc ctgaaaccaa ctgtccttca 660

ccacaaccgg cctttttcac atcagacgac gacgttcttt cgttaatatt acgggacgcc 720

gcaaacgcgt aa 732

<210> 6
<211> 483
<212> DNA
<213> Varicella-zoster virus

<400> 6
tcattggcat acgcgttgga acaaacggtc ggctccccag acacatccat tttccgggat 60

atttgtggaa gatggagtag agtctaccca tacaccggaa agggcatcca acaaagcatc 120

gcgtatgtcc ccgcttttat gttcttcacc aacagattgt gtcagcccct ttaaggtgac 180

gtatggattt gtccagtacg ccatttgttt gtctttaaac caaagtataa cttccggtac 240

tggacatttt gtcttaacca cgattcccga tagcgcctcg ctgaggtttg ataccggggg 300

tgccgcatag tcccacgcct catataccga tgacacgcac ggttccgtta taatcaaact 360

cacatccgat agcggtttgg ctccaaaaaa caacggagtg tcgtcttgga gatgaagaca 420

atacgcgatt gtgatagttt ttaaaaaaac tatcatctgc agtaaccatt tatgtgatgc 480

cat 483